# FASCICULE DE BREVET EUROPEEN

(11) **EP 2 797 898 B1**
(45) Date de publication et mention de la délivrance du brevet: **14.09.2016**
(21) Numéro de dépôt: 12808851.5
(22) Date de dépôt: 28.12.2012
(51) Int. Cl.: C07D 241/04, C07D 409/06, C07D 487/04, C07D 487/08, A61K 31/4965, A61P 35/00

(54) **DERIVES PIPERAZINYLES POUR LE TRAITEMENT DE CANCERS**
PIPERAZINYL-DERIVATE FÜR DIE BEHANDLUNG VON KREBS
PIPERAZINYL DERIVATIVES FOR THE TREATMENT OF CANCERS

(30) Priorité: 30.12.2011 FR 1162586
(43) Date de publication de la demande: 05.11.2014
(73) Titulaire: Pitty, Marc-Henry, 92100 Boulogne - Billancourt (FR)
(72) Inventeur: CARNIATO, Denis, 91460 Marcoussis (FR); BRIAND, Jean-François, 91400 Saclay (FR); GUTMANN, Mathieu, 91640 Vaugrigneuse (FR); BUSNEL, Olivier, 59251 Allennes les Marais (FR); BOUGERET, Cécile, 58000 Nevers (FR); DEPREZ, Benoit, 59000 Lille (FR); JAILLARDON, Karine, 91240 Saint Michel s/Orge (FR)
(74) Mandataire: Regimbeau
(86) Numéro de dépôt international: PCT/EP2012/077059
(87) Numéro de publication internationale: WO 2013/098393

(56) Documents cités:
- WO-A1-2009/150248
- WO-A1-2012/009678

## Description

La présente invention concerne des composés pipérazinyles utiles notamment pour le traitement du cancer, ainsi que les compositions les contenant et leur procédé de préparation.

Avec l'allongement de la durée de vie, le cancer, une des premières causes de mortalité au monde, touche de plus en plus de personnes et reste difficile à soigner.

Le développement de résistance aux agents anticancéreux est un problème sérieux qui freine considérablement le traitement de nombreux types de cancers. La baisse de tolérance à un agent est souvent accompagnée de résistance croisée à une variété d'autres agents. Cette résistance multiple aux agents anticancéreux (Multidrug Resistance, MDR) est causée par de nombreux mécanismes dont seul un petit nombre est bien caractérisé. Ces mécanismes incluent une augmentation de l'efflux des médicaments, une augmentation des capacités de détoxication de la cellule, une altération des cibles moléculaires affectées par ces agents-anticancéreux, une modification du système de réparation de l'ADN ainsi qu'une modification des voies apoptotiques (Baguley, Mol. Biotechnol., 2010, 46, 308-316 ; Gatti et al., Methods Mol. Med. 2005, 111, 127-148 ; Longley et al., J. Pathol. 2005, 205, 275-292 ; Kohno et al., Eur. J. Cancer 2005, 41, 2577-2586).

Le développement de traitements anticancéreux pouvant échapper à ces mécanismes de résistance est un enjeu majeur et jusqu'à maintenant les essais initiés n'ont donné que peu de résultats.

Des agents anticancéreux plus particulièrement destinés au traitement d'un cancer résistant à une chimiothérapie sont décrits dans WO 2009/150248. Ils répondent à la formule générale (I) suivante : 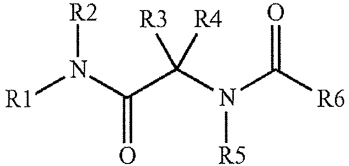 dans laquelle R1 et R2 peuvent former, avec l'atome d'azote qui les porte, un hétérocycle tel qu'un groupe pipérazinyle éventuellement substitué, les seuls composés exemplifiés étant éventuellement substitués sur l'atome d'azote de la pipérazine.

Les inventeurs de la présente demande de brevet ont découvert de manière surprenante que l'introduction d'un substituant X en alpha du second atome d'azote de la pipérazine (voir formule (I) ci-dessous) permettait d'améliorer les propriétés physico-chimiques des composés, en particulier leurs solubilités, leurs propriétés pharmacocinétiques ainsi que leurs activités biologiques.

La présente demande de brevet a donc plus particulièrement pour objet un composé pipérazinyle substitué de formule générale (I) suivante : 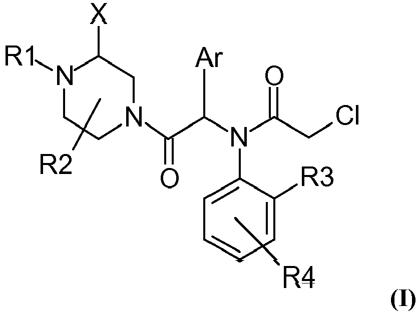 ainsi que ses sels pharmaceutiquement acceptables, ses stéréoisomères ou mélanges de stéréoisomères en toutes proportions, en particulier un mélange d'énantiomères, et notamment un mélange racémique, pour laquelle :
- X représente un groupement (C₁-C₆)alkyle, phényle, benzyle, C(O)OR5, ou C(O)NHR5,
- R1 représente un atome d'hydrogène ou un groupement C(O)H, C(O)R6, ou C(O)OR6,
- R2 représente un atome d'hydrogène ou un groupement (C₁-C₆)alkyle, ou R2 forme avec R1 ou X une chaîne hydrocarbonée saturée de manière à former un cycle à 5 ou 6 chaînons, notamment à 5 chaînons,
- R3 représente un atome d'hydrogène ou d'halogène ou un groupement (C₁-C₆)alkyle, ou (C₁-C₆)alkoxy,
- R4 représente un atome d'hydrogène ou d'halogène, CN, NO₂, ou un groupement (C₁-C₆)alkyle, (C₁-C₆)alkoxy, aryloxy, benzyloxy, ou hétéroaryloxy, ledit groupement étant éventuellement substitué par un ou plusieurs atomes d'halogène,
- Ar représente un groupement thiophényle ou un groupement phényle éventuellement substitué par un ou plusieurs atomes d'halogène, et
- R5 et R6 représentent, indépendamment l'un de l'autre, un groupement (C₁-C₆)alkyle, aryl-(C₁-C₆)alkyle ou aryle, ledit groupement étant éventuellement substitué par un ou plusieurs atomes d'halogène.

Par « halogène », on entend au sens de la présente invention un atome de fluor, de brome, de chlore ou d'iode. Avantageusement, il s'agit d'un atome de fluor, de brome ou de chlore.

Par groupement « alkyle », on entend, au sens de la présente invention, tout groupe hydrocarboné saturé, linéaire ou ramifié, comportant avantageusement 1 à 6, de préférence 1 à 4 atomes de carbone. Il peut s'agir en particulier des groupes méthyle, éthyle, n-propyle, iso-propyle, n-butyle, iso-butyle, sec-butyle, tert-butyle, n-pentyle, néopentyle ou n-hexyle. Avantageusement il s'agit d'un groupe méthyle, éthyle, isopropyle, tert-butyle ou isobutyle.

Le cas échéant, le groupement alkyle peut être substitué par un ou plusieurs atomes d'halogène, en particulier brome, chlore et fluor et avantageusement fluor. Il s'agira en particulier dans ce cas du groupement -CF₃.

Par groupement « alkoxy », on entend, au sens de la présente invention, un groupement alkyle tel que défini ci-dessus lié au reste de la molécule par l'intermédiaire d'un atome d'oxygène. Des exemples de groupement alkoxy sont le groupe méthoxy, éthoxy, isopropoxy ou encore tert-butoxy. Avantageusement, il s'agit du méthoxy ou du tert-butoxy, et encore plus avantageusement, il s'agit du méthoxy.

Le cas échéant, le groupement alkoxy peut être substitué par un ou plusieurs atomes de fluor. Dans ce cas, il s'agira avantageusement du groupement -OCHF₂ ou -OCF₃, en particulier -OCF₃.

Par groupement « aryle », on entend, au sens de la présente invention, un groupement aromatique, comportant de préférence de 5 à 10 atomes de carbone et comprenant un ou plusieurs cycles accolés. Avantageusement, il s'agit du phényle.

Par groupement « hétéroaryle », on entend, au sens de la présente invention, tout groupe aryle tel que défini ci-dessus dans lequel un ou plusieurs atomes de carbone ont été remplacés par un ou plusieurs hétéroatomes, avantageusement 1 à 4, et encore plus avantageusement 1 à 2, tels que par exemple des atomes de soufre, azote ou oxygène. Avantageusement, il s'agit d'un groupe furyle, thiophényle, pyridinyle, pyrimidyle, quinolinyle, 1,2,3-thiadiazolyle, benzoimidazolyle, indazolyle ou 1,2,3-benzotriazolyle.

Par groupement « aryloxy », on entend, au sens de la présente invention, un groupement aryle tel que défini ci-dessus lié au reste de la molécule par l'intermédiaire d'un atome d'oxygène. Il s'agit avantageusement d'un groupement phényloxy.

Par groupement « hétéroaryloxy », on entend, au sens de la présente invention, un groupement hétéroaryle tel que défini ci-dessus lié au reste de la molécule par l'intermédiaire d'un atome d'oxygène. Il s'agit avantageusement d'un groupement pyridinyloxy.

Par groupement « aryl-(C₁-C₆)alkyle », on entend au sens de la présente invention, un groupement aryle tel que défini ci-dessus lié au reste de la molécule par l'intermédiaire d'un groupement alkyle tel que défini ci-dessus comprenant 1 à 6 atomes de carbone. Avantageusement, il s'agit d'un groupe benzyle ou 1-phénéthyle, et encore plus avantageusement d'un benzyle.

Dans la présente invention, on entend par « pharmaceutiquement acceptable » ce qui est utile dans la préparation d'une composition pharmaceutique qui est généralement sûre, non toxique et ni biologiquement ni autrement non souhaitable et qui est acceptable pour une utilisation vétérinaire de même qu'en pharmacopée humaine.

Par « sels pharmaceutiquement acceptables » d'un composé, on entend désigner dans la présente invention des sels qui sont pharmaceutiquement acceptables, comme défini ici, et qui possèdent l'activité pharmacologique souhaitée du composé parent. De tels sels comprennent :
(1) les hydrates et les solvates,
(2) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, avantageusement, ce sera l'acide chlorhydrique ; et
(3) les sels formés lorsqu'un proton acide présent dans le composé parent soit est remplacé par un ion métallique, par exemple un ion de métal alcalin (Na⁺, K⁺ ou Li⁺ par exemple), un ion de métal alcalino-terreux (comme Ca²⁺ ou Mg²⁺) ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Dans la présente invention, on entend désigner par « stéréoisomères », au sens de la présente invention, des diastéréoisomères ou des énantiomères. Il s'agit donc d'isomères optiques. Les stéréoisomères qui ne sont pas des images dans un miroir l'un de l'autre sont ainsi désignés par « diastéréoisomères », et les stéréoisomères qui sont des images dans un miroir non superposables sont désignés par « énantiomères ».

Un atome de carbone lié à quatre substituants non identiques est appelé un centre chiral.

Un mélange équimolaire de deux énantiomères est appelé mélange racémique.

Les composés selon la présente invention pourront en particulier répondre à la formule (I-bis) suivante : 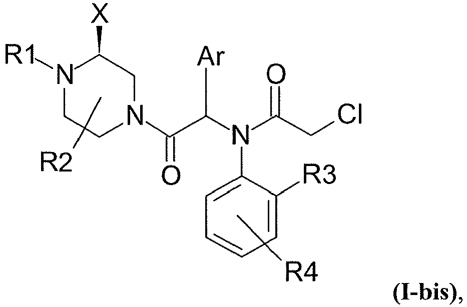 l'atome d'azote portant le groupement X étant alors de configuration (S).

Avantageusement, X représentera un groupe (C₁-C₆)alkyle, en particulier (C₁-C₄)alkyle, phényle ou benzyle.

Avantageusement, R1 représentera un atome d'hydrogène ou un groupement C(O)R6, ou C(O)OR6, en particulier un atome d'hydrogène.

Avantageusement, R2 représentera un atome d'hydrogène ou un groupement (C₁-C₆)alkyle, tel que méthyle.

Avantageusement, R3 représentera un atome d'hydrogène ou un groupement (C₁-C₆)alkyle, tel que méthyle.

Avantageusement, R4 représentera un atome d'hydrogène ou d'halogène, ou un groupement (C₁-C₆)alkyle, (C₁-C₆)alkoxy, ou aryloxy, ledit groupement étant éventuellement substitué par un ou plusieurs atomes d'halogène, en particulier de fluor.

Avantageusement, Ar représentera un groupement thiophényle ou un groupement phényle substitué par un ou plusieurs atomes de fluor tel que le 4-fluoro-phényle.

Selon un mode de réalisation particulier de l'invention, X représente un groupement (C₁-C₆)alkyle, phényle, benzyle, C(O)OR5, C(O)NHR5; R1 représente un atome d'hydrogène ; R2 représente un atome d'hydrogène ou un groupement (C₁-C₆)alkyle, avantageusement (C₁-C₄)alkyle ou forme avec R1 ou X une chaîne hydrocarbonée saturée de manière à former un cycle à 5 chaînons ; R3 représente un atome d'hydrogène ou d'halogène ou un groupement (C₁-C₆)alkyle, en particulier (C₁-C₃)alkyle, ou (C₁-C₆)alkoxy, tel que méthoxy ; R4 représente un atome d'halogène, CN, NO₂ ou un groupement (C₁-C₆)alkyle, (C₁-C₆)alkoxy, aryloxy, benzyloxy, ou hétéroaryloxy, ledit groupement étant éventuellement substitué par un ou plusieurs atomes d'halogène ; Ar représente un groupement thiophényle ou un groupement phényle éventuellement substitué par un halogène ; et R5 et R6 représentent, indépendamment l'un de l'autre, un groupement (C₁-C₆)alkyle, aryl-(C₁-C₆)alkyle, ou aryle, ledit groupement étant éventuellement substitué par un ou plusieurs atomes d'halogène.

Encore plus avantageusement, X représente un groupement (C₁-C₆)alkyle, phényle, benzyle, C(O)OR5, C(O)NHR5 ; R1 représente un atome d'hydrogène ; R2 représente un atome d'hydrogène ou un groupement (C₁-C₆)alkyle, avantageusement (C₁-C₄)alkyle; R3 représente un atome d'hydrogène ou d'halogène ou un groupement (C₁-C₆)alkyle, en particulier (C₁-C₃)alkyle, ou (C₁-C₆)alkoxy, tel que méthoxy ; R4 représente un atome d'halogène, ou un groupement (C₁-C₆)alkyle, (C₁-C₆)alkoxy, aryloxy, benzyloxy, ou hétéroaryloxy, ledit groupement étant éventuellement substitué par un ou plusieurs atomes d'halogène ; Ar représente un groupement thiophényle ou un groupement phényle éventuellement substitué par un halogène ; et R5 et R6 représentent, indépendamment l'un de l'autre, un groupement (C₁-C₆)alkyle, aryl-(C₁-C₆)alkyle, ou aryle, ledit groupement étant éventuellement substitué par un ou plusieurs atomes d'halogène.

De manière encore plus avantageuse, X représente un groupement (C₁-C₆)alkyle, phényle, ou benzyle ; R1 et R2 représentent un atome d'hydrogène ; R3 représente un atome d'hydrogène ou d'halogène ou un groupement (C₁-C₆)alkyle, en particulier (C₁-C₃)alkyle ; R4 représente un atome d'halogène, ou un groupement (C₁-C₆)alkyle, (C₁-C₆)alkoxy, aryloxy, ou benzyloxy, ledit groupement étant éventuellement substitué par un ou plusieurs atomes d'halogène ; Ar représente un groupement thiophényle ou un groupement phényle éventuellement substitué par un halogène ; et R5 et R6 représentent, indépendamment l'un de l'autre, un groupement (C₁-C₆)alkyle, aryl-(C₁-C₆)alkyle, ou aryle, ledit groupement étant éventuellement substitué par un ou plusieurs atomes d'halogène.

De préférence, X représente un groupement (C₁-C₆)alkyle, phényle, ou benzyle ; R1 et R2 représentent un atome d'hydrogène ; R3 représente un atome d'hydrogène ou un groupement (C₁-C₆)alkyle, en particulier (C₁-C₃)alkyle ; R4 représente un atome d'halogène, ou un groupement (C₁-C₆)alkyle, (C₁-C₆)alkoxy, aryloxy, ou benzyloxy, ledit groupement étant éventuellement substitué par un ou plusieurs atomes d'halogène ; Ar représente un groupement thiophényle ou un groupement phényle éventuellement substitué par un atome de fluor tel que le 4-fluoro-phényle ; et R5 et R6 représentent, indépendamment l'un de l'autre, un groupement (C₁-C₆)alkyle, aryl-(C₁-C₆)alkyle, ou aryle, ledit groupement étant éventuellement substitué par un ou plusieurs atomes de fluor.

Il s'agira en particulier de l'un des exemples I-1a à I-63 décrits dans la partie expérimentale ci-après, ou d'un de leurs sels pharmaceutiquement acceptables, de leurs stéréoisomères ou mélanges de stéréoisomères en toutes proportions, en particulier un mélange d'énantiomères, et notamment un mélange racémique.

La présente invention concerne également un composé de formule (I) tel que défini ci-dessus pour son utilisation en tant que médicament, en particulier destiné au traitement ou à la prévention d'un cancer, et en particulier au traitement d'un cancer résistant à une chimiothérapie.

La présente invention concerne également l'utilisation d'un composé de formule (I) tel que défini ci-dessus pour la fabrication d'un médicament, en particulier destiné au traitement ou à la prévention d'un cancer, et en particulier au traitement d'un cancer résistant à une chimiothérapie.

La présente invention concerne également une méthode de traitement ou de prévention du cancer, en particulier d'un cancer résistant à une chimiothérapie, comprenant l'administration d'une quantité suffisante d'un composé de formule (I) tel que défini ci-dessus à un patient en ayant besoin.

La présente invention a également pour objet une composition pharmaceutique comprenant au moins un composé de formule (I) tel que défini ci-dessus, en association avec un ou plusieurs excipients pharmaceutiquement acceptables.

Dans un mode de réalisation particulier, cette composition pourra comprendre au moins un autre principe actif.

En particulier, ce(s) principe(s) actif(s) pourra(pourront) être des agents anticancéreux utilisés classiquement dans le traitement du cancer. Ces agents anticancéreux pourront être choisis en particulier parmi le cisplatine et ses dérivés tels que le carboplatine et l'oxalyplatine; des taxanes tels que le taxol, le taxotère, le paclitaxel et le docétaxel ; des alcaloïdes de vinca tels que la vinblastine, la vincristine et la vinorelbine ; des analogues de purine tels que la mercaptopurine, la thioguanine, la pentostatine et la 2-chlorodésoxyadénosine ; des inhibiteurs de topoisomerase I tels que des composés de la camptothécine comme l'irinotecan et le topotecan ; des inhibiteurs de topoisomerase II tels que l'épipodophyllotoxine, la podophyllotoxine et leurs dérivés comme l'étoposide et le téniposide ; des dérivés nucléosides antitumoraux tels que le 5-fluorouracile, la leucovorine, la gemcitabine ou la capécitabine ; des agents alkylants tels que des moutardes à l'azote comme la cyclophosphamide, la mechloréthamine, la chlorambucile et le melphalan, des nitroso-urées comme la carmustine, la lomustine et la streptozocine, des alkylsulfonates comme le busulfan, des éthylènimines et des méthylmélamines comme la thiotépa et l'hexaméthylmélamine, et des tétrazines comme la dacarbazine ; des dérivés d'antracyclines anti-tumoraux tels que la daunorubicine, l'adriamycine, le doxil, l'idarubicine et la mitoxantrone ; des molécules ciblant le récepteur IGF-I telles que la picropodophylline ; des dérivés de tetracarcin tels que le tetrocarcin A ; des corticostéroïdes tels que la prednisone ; des anticorps tels que le trastuzumab (anticorps anti-HER2), le rituximab (anticorps anti-CD20), le gemtuzamab, le cetuximab, le pertuzumab et le bevacizumab; des antagonistes ou des modulateurs sélectifs des récepteurs des oestrogènes tels que le tamoxifen, le fulvestrant, le toremifène, le droloxifène, le faslodex et le raloxifène ; des inhibiteurs d'aromatase tels que l'exémestane, l'anastrozole, le letrozole et le vorozole ;des agents de différenciation tels que les rétinoïdes comme l'acide rétinoïque et la vitamine D et des agents bloquant le métabolisme de l'acide rétinoïque tels que l'accutane ; des inhibiteurs d'ADN méthyle-transférase tels que l'azacytidine et le decitabine ; des antifolates tels que le permetrexed disodique ; des antibiotiques tels que l'antinomycine D, la bléomycine, la mitomycine C, l'actinomycine D, la carminomycine, la daunomycine et la plicamycine ; des antimétabolites tels que la chlofarabine, l'aminoptérine, la cytosine arabinoside, la floxuridine et le méthotrexate ; des agents induisant l'apoptose et des agents antiangiogéniques des inhibiteurs de Bcl-2 tels que YC 137, BH 312, ABT 737, le gossypol, HA 14-1, TW 37 et l'acide décanoïque ; des agents se liant à la tubuline tels que la combrestatine, des dérivés de colchicine et le nocodazole ; des inhibiteurs de kinase tels que le flavoperidol, le mésylate d'imatinib, l'erlotinib et le gefitinib ; des inhibiteurs de farnésyl transférase tels que le tipifarnib ; des inhibiteurs des histone-désacétylases tels que le butyrate de sodium, l'acide suberoylanilide hydroxamique, le depsipeptide, NVP- LAQ824, R306465, JNJ-26481585 et la trichostatine A; des inhibiteurs du système ubiquitine-protéasome tels que MLN .41, le bortezomib et l'yondelis ; et des inhibiteurs de télomérase tels que la telomestatine.

Les composés selon l'invention peuvent être administrés par voie orale, sublinguale, parentérale, sous-cutanée, intramusculaire, intraveineuse, transdermique, locale ou rectale.

Dans les compositions pharmaceutiques de la présente invention pour l'administration orale, sublinguale, parentérale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration parentérale, sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs de goût ou des édulcorants.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

Les composés de l'invention peuvent être utilisés à des doses comprises entre 0,01 mg et 1000 mg par jour, donnés en une seule dose une fois par jour ou administrés en plusieurs doses tout au long de la journée, par exemple deux fois par jour en doses égales. La dose administrée par jour est avantageusement comprise entre 5 mg et 500 mg, encore plus avantageusement entre 10 mg et 200 mg. Il peut être nécessaire d'utiliser des doses sortant de ces gammes ce dont l'homme du métier pourra se rendre compte lui-même.

La présente invention a également pour objet une composition pharmaceutique comprenant :
(i) au moins un composé de formule (I) tel que défini ci-dessus, et
(ii) au moins un autre principe actif,
en tant que produits de combinaison pour une utilisation simultanée, séparée ou étalée dans le temps.

En effet, il est courant de traiter le cancer par bi- ou trithérapie. Il pourrait être utile notamment d'associer les molécules de l'invention avec un ou plusieurs composés anticancéreux permettant ainsi de traiter le cancer, d'une part, et de prévenir l'apparition de cellules cancéreuses résistantes, d'autre part.

En particulier, ce(s) principe(s) actif(s) pourra(pourront) être des agents anticancéreux utilisés classiquement dans le traitement du cancer. Ces agents anticancéreux pourront être choisis en particulier parmi le cisplatine et ses dérivés tels que le carboplatine et l'oxalyplatine; des taxanes tels que le taxol, le taxotère, le paclitaxel et le docétaxel ; des alcaloïdes de vinca tels que la vinblastine, la vincristine et la vinorelbine ; des analogues de purine tels que la mercaptopurine, la thioguanine, la pentostatine et la 2-chlorodésoxyadénosine ; des inhibiteurs de topoisomerase I tels que des composés de la camptothécine comme l'irinotecan et le topotecan ; des inhibiteurs de topoisomerase II tels que l'épipodophyllotoxine, la podophyllotoxine et leurs dérivés comme l'étoposide et le téniposide ; des dérivés nucléosides antitumoraux tels que le 5-fluorouracile, la leucovorine, la gemcitabine ou la capécitabine ; des agents alkylants tels que des moutardes à l'azote comme la cyclophosphamide, la mechloréthamine, la chlorambucile et le melphalan, des nitroso-urées comme la carmustine, la lomustine et la streptozocine, des alkylsulfonates comme le busulfan, des éthylènimines et des méthylmélamines comme la thiotépa et l'hexaméthylmélamine, et des tétrazines comme la dacarbazine ; des dérivés d'antracyclines anti-tumoraux tels que la daunorubicine, l'adriamycine, le doxil, l'idarubicine et la mitoxantrone ; des molécules ciblant le récepteur IGF-I telles que la picropodophylline ; des dérivés de tetracarcin tels que le tetrocarcin A ; des corticostéroïdes tels que la prednisone ; des anticorps tels que le trastuzumab (anticorps anti-HER2), le rituximab (anticorps anti-CD20), le gemtuzamab, le cetuximab, le pertuzumab et le bevacizumab; des antagonistes ou des modulateurs sélectifs des récepteurs des oestrogènes tels que le tamoxifen, le fulvestrant, le toremifène, le droloxifène, le faslodex et le raloxifène ; des inhibiteurs d'aromatase tels que l'exémestane, l'anastrozole, le letrozole et le vorozole ;des agents de différenciation tels que les rétinoïdes comme l'acide rétinoïque et la vitamine D et des agents bloquant le métabolisme de l'acide rétinoïque tels que l'accutane ; des inhibiteurs d'ADN méthyle-transférase tels que l'azacytidine et le decitabine ; des antifolates tels que le permetrexed disodique ; des antibiotiques tels que l'antinomycine D, la bléomycine, la mitomycine C, l'actinomycine D, la carminomycine, la daunomycine et la plicamycine ; des antimétabolites tels que la chlofarabine, l'aminoptérine, la cytosine arabinoside, la floxuridine et le méthotrexate ; des agents induisant l'apoptose et des agents antiangiogéniques des inhibiteurs de Bcl-2 tels que YC 137, BH 312, ABT 737, le gossypol, HA 14-1, TW 37 et l'acide décanoïque ; des agents se liant à la tubuline tels que la combrestatine, des dérivés de colchicine et le nocodazole ; des inhibiteurs de kinase tels que le flavoperidol, le mésylate d'imatinib, l'erlotinib et le gefitinib ; des inhibiteurs de farnésyl transférase tels que le tipifarnib ; des inhibiteurs des histone-désacétylases tels que le butyrate de sodium, l'acide suberoylanilide hydroxamique, le depsipeptide, NVP- LAQ824, R306465, JNJ-26481585 et la trichostatine A; des inhibiteurs du système ubiquitine-protéasome tels que MLN .41, le bortezomib et l'yondelis ; et des inhibiteurs de télomérase tels que la télomestatine.

La présente invention a également pour objet une composition pharmaceutique telle que définie ci-dessus, pour son utilisation à titre de médicament, destiné notamment au traitement ou à la prévention du cancer, en particulier au traitement d'un cancer résistant à une chimiothérapie.

La présente invention concerne également un procédé de préparation d'un composé de formule (I) tel que défini ci-dessus comprenant les étapes successives suivantes :
a) réaction d'une amine de formule (II) suivante : 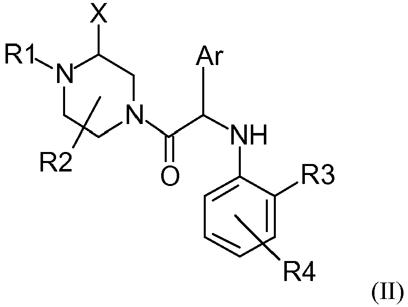 avec X, R1, R2, R3, R4 et Ar tels que définis précédemment, R1 ne représentant pas un atome d'hydrogène,
   avec du chlorure de chloroacétyle en présence d'une base pour donner un composé de formule (1) avec R1 ≠ H, et
b) éventuellement déprotection de l'atome d'azote portant le groupement R1 ≠ H pour donner un composé de formule (I) avec R1 = H.

### Etape a) :

La base utilisée pour cette étape sera de préférence une base faible telle que NaHCO₃.

L'amine de formule (II) pourra être obtenue par réaction d'une pipérazine de formule (III) suivante : 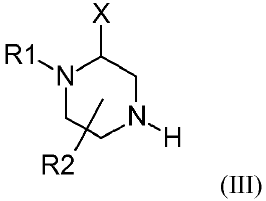 avec X, R1 et R2 tels que définis précédemment, R1 ne représentant pas un atome d'hydrogène,
avec un acide de formule (IV) suivante : 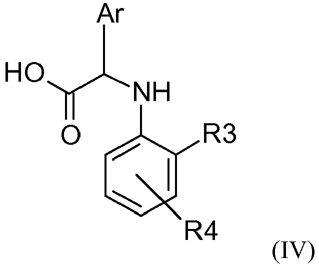 avec R3, R4 et Ar tels que définis précédemment.

Cette réaction peut être réalisée dans des conditions de couplage peptidique bien connues de l'homme du métier.

Le couplage est ainsi réalisé de préférence en présence d'un agent de couplage, tel que le diisopropylcarbodiimide (DIC), le dicyclohexylcarbodiimide (DCC), le chlorhydrate de 1-(3-diméthylaminopropyl)-3-éthylcarbodiimide (EDC), le carbonyldiimidazole (CDI), le 2-(1H-benzotriazole-1-yl)-1,1,3,3-tetraméthyluronium hexafluorophosphate (HBTU), le 2-(1H-benzotriazole-1-yl)-1,1,3,3-tetraméthyluronium tetrafluoroborate (TBTU) ou encore le O-(7-azobenzotriazol-1-yl)-1,1,3,3-tétraméthyluronium hexafluorophosphate (HATU), éventuellement associé à un auxiliaire de couplage tel que le N-hydroxy succinimide (NHS), le N-hydroxy benzotriazole (HOBt), le 3,4-dihydro-3-hydroxy-4-oxo-1,2,3-benzotriazole (HOOBt), le 1-hydroxy-7-azabenzotriazole (HAt) ou le N-hydroxysylfosuccinimide (sulfo NHS). De préférence, il s'agira du HBTU.

Une base telle que la diisopropyl-éthylamine (DIPEA) peut également être présente.

La pipérazine de formule (III) est soit commerciale soit préparée selon des méthodes bien connues de l'homme du métier.

L'acide de formule (IV) peut être préparé quant à lui selon les étapes successives suivantes :
i) réaction d'un cétoester de formule (V) suivante : 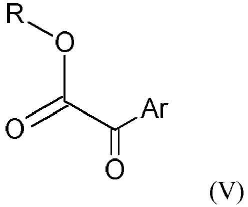 avec Ar tel que défini précédemment et R représentant un groupe (C₁-C₆)alkyle, tel qu'éthyle,
   avec une aniline de formule (VI) suivante : 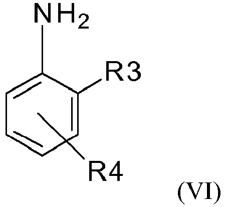 avec R3 et R4 tels que définis précédemment,
   pour donner une imine de formule (VII) suivante : 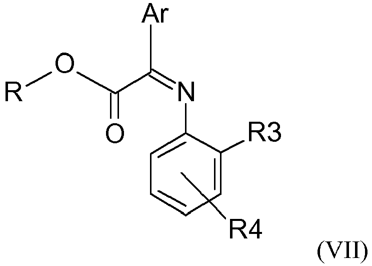 avec R, R3, R4 et Ar tels que définis précédemment,
ii) réduction de l'imine de formule (VII) obtenue à l'étape précédente pour donner une amine de formule (VIII) suivante : 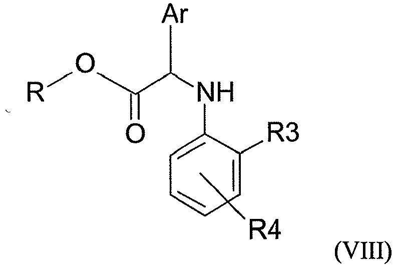 avec R, R3, R4 et Ar tels que définis précédemment, et
iii) saponification de la fonction ester du composé de formule (VIII) obtenu à l'étape précédent pour donner l'acide de formule (IV).

L'étape i) peut être réalisée en présence d'un acide comme l'acide para-toluène sulfonique (APTS). La réaction peut être réalisée dans un solvant polaire tel que le toluène. De préférence, le milieu réactionnel sera chauffé au reflux en utilisant un DeanStark de manière à éliminer l'eau formée au cours de la réaction au fur et à mesure.

Le cétoester (V) utilisé pour cette réaction est soit commercial, soit préparé par une réaction de Friedel-Crafts à partir de chlorure d'oxalate d'éthyle et de l'aromatique correspondant, en présence d'un acide de Lewis comme le chlorure d'aluminium AlCl₃.

L'aniline (VI) utilisée pour cette réaction est soit commerciale, soit préparée par des méthodes bien connues de l'homme du métier.

L'étape ii) de réduction peut être réalisée en présence d'un agent réducteur bien connu de l'homme du métier tel que le cyanoborohydrure de sodium.

L'étape iii) de saponification peut être réalisée dans des conditions bien connues de l'homme du métier, notamment en présence d'une base telle que NaOH, KOH ou LiOH.

### Etape b) :

Cette étape sera réalisée de préférence avec un composé de formule (I) pour lequel R1 = CO₂R6, tel que CO₂tBu, par traitement avec un acide tel que HCl.

Le composé ainsi obtenu pourra être séparé du milieu réactionnel par des méthodes bien connues de l'homme du métier, comme par exemple par extraction, évaporation du solvant ou encore par précipitation et filtration.

Le composé pourra être par ailleurs purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

Le procédé selon la présente invention pour préparer des composés selon la présente invention avec R1 ≠ H est présenté sur le schéma réactionnel suivant :

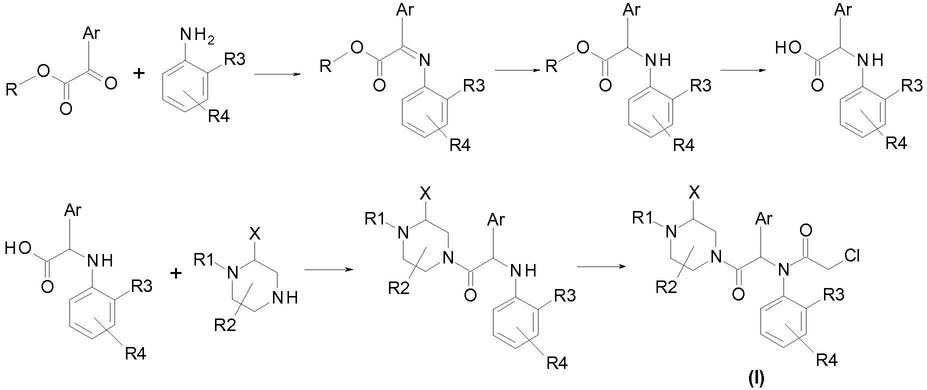

Les exemples suivants illustrent l'invention sans toutefois la limiter.

### FIGURE :

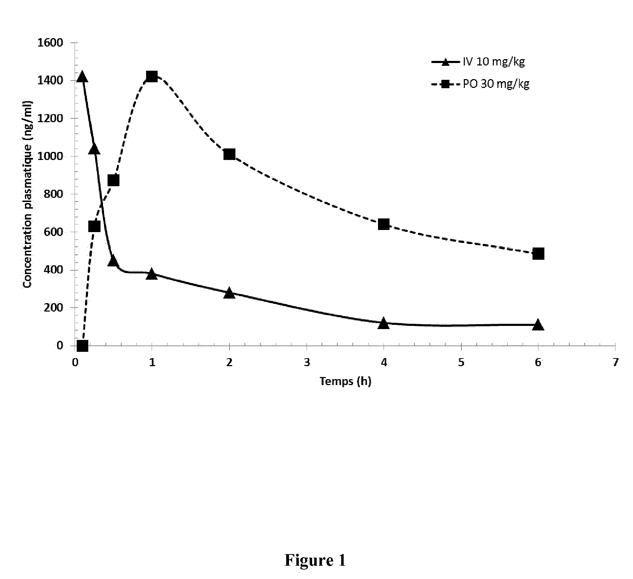

La figure 1 présente les courbes temps-concentration plasmatique pour une souris ayant reçu le composé I-43 dia2 administré par voie intra-veineuse (IV) à la dose de 10 mg/kg ou par voie orale (PO) à la dose de 30 mg/kg.

### EXEMPLES :

### I - Synthèse de composés selon l'invention

Dans la partie qui suit, deux nomenclatures distinctes ont été adoptées lorsque les deux diastéréoisomères d'un composé selon l'invention ont été séparés :
- a / b désignant chacun la structure particulière d'un diastéréoisomère unique,
- dia1 / dia2 désignant respectivement le diastéréoisomère le moins polaire et le plus polaire dans le système chromatographique utilisé.

En effet, la stéréochimie particulière de chacun des diastéréoisomères n'a pas été déterminée. Par conséquent, il est impossible d'attribuer à chacun des diastéréoisomères dia1 et dia2 isolés la structure particulière a et b. C'est pourquoi une double nomenclature a été utilisée.

Les abréviations suivantes ont été utilisées dans cette partie :
- CCM: Chromatographie sur Couche Mince
- DCM: Dichlorométhane
- DIEA: Diisopropyléthylamine
- HBTU: 2-(1H-Benzotriazole-1-yl)-1,1,3,3-tetraméthyluronium hexafluorophosphate
- LCMS: Chromatographie liquide couplée à un spectromètre de masse
- RMN: Résonance Magnétique Nucléaire
- TA: Température ambiante

### Exemples I-1a et I-1b : Diastéréoisomères de l'ester tert-butylique de l'acide 4-[2-[(2-chloro-acétyl)-(4-phénoxy-phényl)-amino]-2-(4-fluoro-phényl)-acétyl]-(S)-2-isopropyl-pipérazine-1-carboxylique.

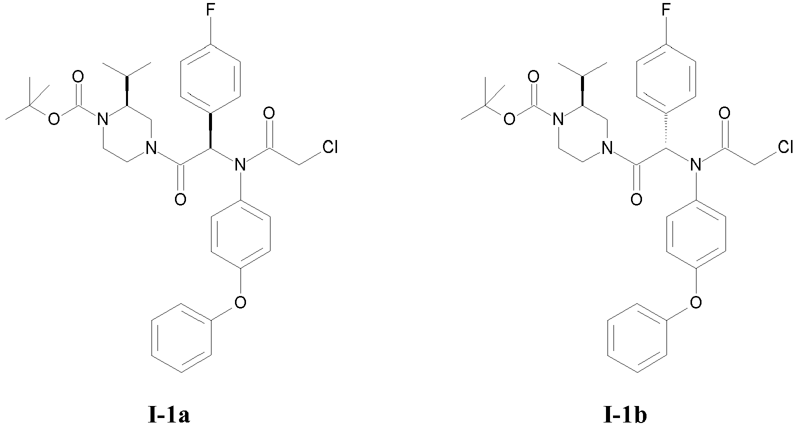

### Stade 1 : Ester éthylique de l'acide (4-fluoro-phényl)-oxo-acétique (1) :

A une solution de chlorure d'aluminium (21,13 g ; 160 mmol) dans le DCM (200 mL) à 0°C sous argon, est ajouté goutte à goutte pendant 10 min le chlorure d'oxalate d'éthyle (17,9 mL ; 160 mmol). Le milieu est laissé sous agitation pendant 10 minutes. Le fluorobenzène (14,7 mL ; 160 mmol) dilué dans 30 mL de DCM, est ajouté goutte à goutte à 0°C. Le milieu est laissé sous agitation à température ambiante pendant 12 heures. Le milieu est lavé à l'eau et la phase organique est séchée sur MgSO₄. Après évaporation, l'huile récupérée est purifiée par flash chromatographie sur gel de silice avec l'éluant cyclohexane-acétate d'éthyle 90-10.
Récupération d'une huile jaune (17,08 g ; 54 %).
RMN ¹H (300 MHz, CDCl₃) : δ 8,04-8,14 (m ; 1,8 H); 7,15-7,24 (m ; 1,9 H) ; 4,46 (q ; *J* = 7.2 Hz ; 2,0 H) ; 1,44 (t ; *J* = 7.2 Hz ; 3,0 H).

### Stade 2 : Ester éthylique de l'acide (4-fluoro-phényl)-[(Z)-4-phénoxy-phénylimino]-acétique (2) :

A une solution de **1** (3,92 g ; 20 mmol) dans le toluène (25 mL), sont ajoutés successivement l'acide para-toluène sulphonique (200 mg ; 1 mmol) et la 4-phénoxyphényle-aniline (3,70 g ; 20 mmol) en présence de tamis moléculaire. Le milieu est porté au reflux dans un DeanStark pendant 20 heures. Le milieu est lavé à l'eau et la phase organique est séchée sur MgSO₄. Après évaporation, l'huile récupérée est purifiée par flash chromatographie sur gel de silice avec l'éluant cyclohexane-acétate d'éthyle 90-10.
Récupération d'une huile jaune (6,27 g ; 86 %).
LCMS [M+H] = 364 (C₂₂H₁₈FNO₃)

### Stade 3 : Ester éthylique de l'acide (4-fluoro-phényl)-(4-phénoxy-phnylamino)-acétique (3):

A une solution de **2** (6,27 g ; 17,26 mmol) dans le méthanol (75 mL) et l'acide acétique (7,5 mL), est ajouté le cyanoborohydrure de sodium (1,63 g ; 26 mmol). Le milieu est laissé sous agitation pendant 1 heure à TA. Le méthanol est évaporé partiellement, la solution est neutralisée avec Na₂CO₃ avec ajout d'eau si nécessaire. Le milieu est extrait au DCM et la phase organique est séchée sur MgSO₄. Après évaporation, l'huile récupérée est purifiée par flash chromatographie sur gel de silice avec l'éluant cyclohexane-acétate d'éthyle 95-5.
Récupération d'une huile jaune (5,91 g; 93 %).
LCMS [M+H] = 366 (C₂₂H₂₀FNO₃)
RMN ¹H (300 MHz, CDCl₃) : δ 7,45-7,5 (m ; 1,9 H) ; 7,23-7,31 (m ; 1,9 H) ; 6,97-7,11 (m ; 2,9 H) ; 6,81-6,94 (m ; 3,9 H) ; 6,54 (d ; *J* = 9,0 Hz ; 2,0 H) ; 5,01 (br ; 1,0 H) ; 4,90 (br ; 0,9 H) ; 4,10-4,32 (m ; 2,0 H) ; 1,23 (t ; *J* = 7,0 Hz ; 3,0 H).

### Stade 4 : Acide (4-fluoro-phényl)-(4-phénoxy-phénylamino)-acetique (4) :

A une solution de **3** (8,04 g ; 22 mmol) dans 130 mL d'acétonitrile est ajouté 66 mL d'une solution 1 M en LiOH (3 éq). Le milieu réactionnel est laissé sous agitation pendant 2 à 3 heures, la fin de la réaction est contrôlée par CCM (cyclohexane-acétate d'éthyle 60-40). L'acétonitrile est partiellement évaporé, le milieu est acidifié avec une solution 1 M en HCl additionné de 200 mL d'eau. Le milieu est filtré et le solide récupéré est lavé trois fois à l'eau puis est séché au dessicateur sous vide en présence de P₂O₅.
Récupération poudre blanche (7,17 g ; 97 %).
LCMS [M+H] = 338 (C₂₀H₁₆FNO₃)
RMN ¹H (300 MHz, DMSO) : δ 7,55 (dd ; *J* = 8,5 Hz ; *J* = 5,6 Hz ; 2,1 H) ; 7,28 (t ; *J* = 7,9 Hz ; 2,1 H) ; 7,20 (t ; *J* = 8,5 Hz ; 2,1 H) ; 6,99 (t ; *J* = 7,0 Hz ; 1,1 H) ; 6,74-6,90 (m ; 4,0 H) ; 6,62-6,70 (m ; 2,0 H) ; 5,10 (s ; 1,0 H).

### Stade 5 : ester ter-butylique de l'acide 4-[2-(4-fluoro-phényl)-2-(4-phénoxy-phénylamino)-acétyl]-(S)-2-isopropyl-pipérazine-1-carboxylique (5) :

A une solution de **4** (7,17 g ; 21,2 mmol) dans le DCM (150 mL) en présence d'un équivalent de DIEA (3,7 mL) est ajoutée une solution du chlorhydrate de la Boc-alpha-(S)-isopropyle-pipérazine (5,63 g ; 21,26 mmol) en présence d'1 éq de DIEA (3,7 mL) dans 50 mL de DCM, suivi du HBTU (8,06 g ; 21,2 mmol). Le milieu est laissé sous agitation pendant 12 heures. Le milieu est lavé à l'eau et la phase organique est séchée sur MgSO₄. Après évaporation, l'huile récupérée est purifiée par flash chromatographie sur gel de silice avec l'éluant cyclohexane-acétate d'éthyle 80-20.
Récupération d'une mousse blanche (11,90 g ; 100 %).
LCMS [M+H] = 548 (C₃₂H₃₈FN₃O₄)

### Stade 6 : Ester tert-butylique de l'acide 4-[2-[(2-chloro-acétyl)-(4-phénoxy-phényl)-amino]-2-(4-fluoro-phényl)-acétyl]-(S)-2-isopropyl-pipérazine-1-carboxylique

A une solution de **5** (11,86 g ; 22,66 mmol) dans 250 mL de DCM en présence de NaHCO₃ (7,30 g ; 87,0 mmol), est ajouté le chlorure de chloroacétyle (3,45 mL ; 43,3 mmol). Le milieu est laissé sous agitation pendant 12 heures. Le milieu est lavé à l'eau et la phase organique est séchée sur MgSO₄. Après évaporation, l'huile récupérée est purifiée par flash chromatographie sur gel de silice avec un gradient cyclohexane-acétate d'éthyle de 95-5' à 50-50 pour obtenir séparément les deux diastéréoisomères sous forme de mousse incolore :

### Diastéréoisomère le moins polaire (I-1 dial)

(3,80 g ; 28 %)
LCMS [M+H] = 625 (C₃₄H₃₉ClFN₃O₅)
RMN ¹H (300 MHz, CDCl₃) : δ 7,92-8,01 (m ; 1,0 H) ; 7,30-7,40 (m ; 2,0 H) ; 7,10-7,18 (m ; 1,1 H) ; 7,01-7,09 (m ; 1,1 H) ; 6,84-7,00 (m ; 6,1 H) ; 6,55-6,65 (m ; 1,1 H) ; 6,32-6,48 (m ; 2,1 H) ; 4,72 (d ; *J* = 13,5 Hz ; 0,5 H) 4,63 (d ; *J* = 13,5 Hz ; 0,4 H) ; 3,52-3,96 (m ; 4,0 H) ; 3,10-3,27 (m ; 0,5 H) ; 2,85-3,07 (m ; 0,4 H) ; 2,23-2,85 (m ; 0,5 H + 0,7 H + 0,4 H) ; 1,87-2,14 (m ; 0,6 H) ; 1,42 (s ; 8,7 H) ; 1,17 (d ; *J* = 6,6 Hz ; 1,0 H) ; 1,03 (d ; *J* = 6,6 Hz ; 1,3 H) ; 0,88 (d ; *J* = 6,6 Hz ; 1,1 H) ;0,69 (d ; *J* = 6,6 Hz ; 1,3 H).

### Diastéréoisomère le plus polaire (I-1 dia2)

(3,29 g ; 24 %)
LCMS [M+H] = 625 (C₃₄H₃₉ClFN₃O₅)
RMN ¹H (300 MHz, CD₂Cl₂) : δ 7,85-8,0 (m ; 1,0 H) ; 7,36 (t ; *J* = 7,6 Hz ; 2,1 H) ; 6,99-7,21 (m ; 3,2 H) ; 6,81-6,98 (m ; 5,2 H) ; 6,63 (br ; 1,1 H) ; 6,35-6,55 (m ; 2,1 H) ; 4,65 (d ; *J* = 13,1 Hz ; 0,6 H) 4,42 (d ; *J* = 13,1 Hz ; 0,3 H) ; 3,50-4,16 (m ; 4,9 H) ; 3,00-3,43 (m ; 0,9 H) ; 2,57-2,90 (m ; 1,9 H) ; 1,98-2,18 (m ; 0,7 H) ; 1,36-1,49 (m ; 10,0 H) ; 1,73 (d ; *J =* 6,5 Hz ; 2,1 H) ; 0,90 (d ; *J* = 6,5 Hz ; 2,1 H) ; 0,63 (d ; *J* = 6,5 Hz ; 1,0 H) ;0,20 (d ; *J* = 6,5 Hz ; 0,9 H).

### Exemples I-2a et I-2b : Diastéréoisomères de l'ester tert-butylique de l'acide 4-[-2-[(2-chloro-acétyl)-(4-phénoxy-phényl)-amino]-2-(4-fluoro-phényl)-acétyl]-(R)-2-isopropyl-pipérazine-1-carboxylique.

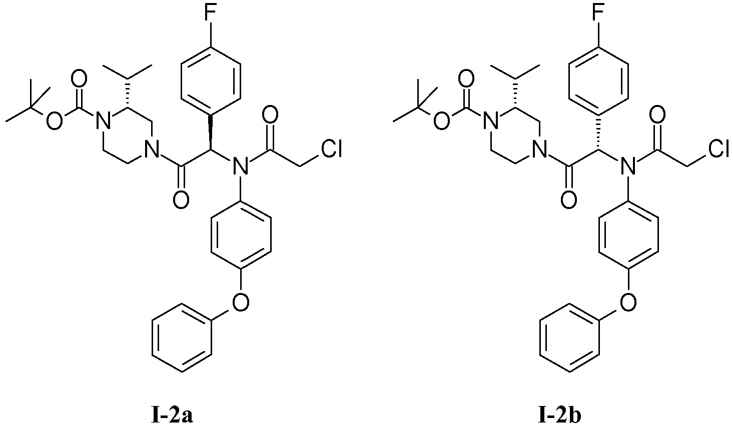

### Stade 1 : Ester tert-butylique de l'acide 4-[2-(4-fluoro-phényl)-2-(4-phénoxy-phénylamino)-acétyl]-(R)-2-isopropyl-pipérazine-1-carboxylique (6) :

A une solution d'acide (4-fluoro-phényl)-(4-phénoxy-phénylamino)-acétique **4** (253 mg ; 0,75 mmol) dans le DCM (10 mL) en présence d'un équivalent de DIEA (131 µL) est ajoutée une solution de la Boc-alpha-(R)-isopropyle-pipérazine (171 mg ; 0,75 mmol) en présence d'1 éq de DIEA (131 µL) dans 5 mL de DCM, suivi du HBTU (285 mg ; 0,75 mmol). Le milieu est laissé sous agitation pendant 12 heures. Le milieu est lavé à l'eau et la phase organique est séchée sur MgSO₄. Après évaporation, l'huile récupérée est purifiée par flash chromatographie sur gel de silice avec l'éluant cyclohexane-acétate d'éthyle 80-20.
Récupération d'une mousse blanche (369 mg ; 90 %).
LCMS [M+H] = 548 (C₃₂H₃₈FN₃O₄)

### Stade 2 : Ester ter-butylique de l'acide 4-[-2-[(2-chloro-acétyl)-(4-phénoxy-phényl)-amino]-2-(4-fluoro-phényl)-acétyl]-(R)-2-isopropyl-pipérazine-1-carboxylique :

Les deux diastéréoisomères ont été préparés à partir de **6** en suivant le même mode opératoire que pour la préparation de l'exemple 1 (stade 6).
Récupération séparément des deux diastéréoisomères sous forme de mousse incolore :

### Diastéréoisomère le moins polaire (I-2 dia1) (195 mg ; 42 %)

LCMS [M+H] = 625 (C₃₄H₃₉ClFN₃O₅)
RMN ¹H (300 MHz, CD₂Cl₂) : δ 7,85-8,00 (m ; 1,0 H) ; 7,36 (t ; *J* = 7,6 Hz ; 2,0 H) ; 6,99-7,21 (m ; 3,1 H) ; 6,81-6,98 (m ; 4,9 H) ; 6,63 (br ; 1,0 H) ; 6,35-6,55 (m ; 2,1 H) ; 4,65 (d ; *J* = 13,0 Hz ; 0,7 H) 4,42 (d ; *J* = 13,0 Hz ; 0,2 H) ; 3,50-4,16 (m ; 4,9 H) ; 3,00-3,43 (m ; 0,8 H) ; 2,57-3,90 (m ; 2,0 H) ; 1,98-2,18 (m ; 0,8 H) ; 1,36-1,49 (m ; 10,5 H) ; 1,73 (d ; *J* = 6,5 Hz ; 2,0 H) ; 0,90 (d ; *J* = 6,5 Hz ; 2,0 H) ; 0,63 (d ; *J* = 6,5 Hz ; 0,8 H) ;0,20 (d ; *J* = 6,5 Hz ; 0,8 H).

### Diastéréoisomère le plus polaire (I-2 dia2) (122 mg ; 26 %)

LCMS [M+H] = 625 (C₃₄H₃₉ClFN₃O₅)
RMN ¹H (300 MHz, CDCl₃) : δ 7,92-8,0 (m ; 1,0 H) ; 7,30-7,40 (m ; 2,0 H) ; 7,10-7,18 (m ; 1,0 H) ; 7,01-7,09 (m ; 1,1 H) ; 6,84-7,00 (m ; 6,0 H) ; 6,55-6,65 (m ; 1,1 H) ; 6,32-6,48 (m ; 2,1 H) ; 4,72 (d ; *J* = 13,5 Hz ; 0,4 H) 4,63 (d ; *J* = 13,5 Hz ; 0,3 H) ; 3,52-3,96 (m ; 4,7 H) ; 3,10-3,27 (m ; 0,7 H) ; 2,85-3,07 (m ; 0,5 H) ; 2,23-2,85 (m ; 0,5 H + 0,6 H + 0,8 H) ; 1,87-2,14 (m ; 0,9 H) ; 1,42 (s ; 8,6 H) ; 1,17 (d ; *J* = 6,6 Hz ; 1,4 H) ; 1,03 (d; *J* = 6,6Hz; 2,1 H); 0,88 (d ; *J* = 6,6 Hz ; 2,1 H) ;0,69 (d ; *J* = 6,6 Hz ; 1,6 H).

### Exemples I-3a et I-3b : Chlorhydrate des diastéréoisomères du 2-chloro-N-[1-(4-fluoro-phenyl)-2-((S)-3-isopropyl-piperazin-1-yl)-2-oxo-ethyl]-N-(4-phenoxy-phenyl)-acetamide.

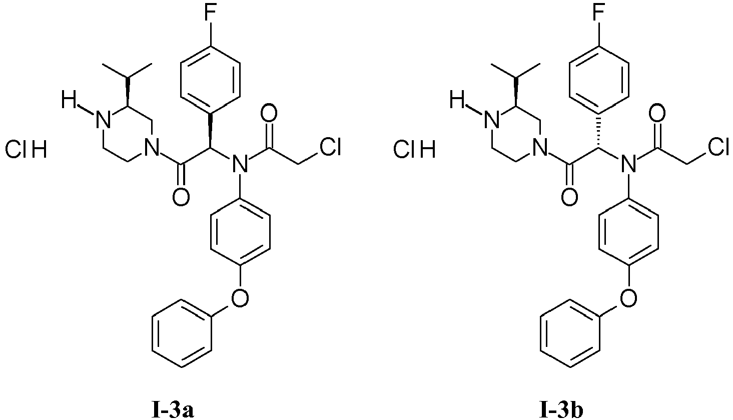

A une solution du diastéréoisomère I-1 dia2 (3,24 g ; 5,2 mmol) dans 50 mL de DCM est ajouté le HCl gaz par bullage. Le milieu réactionnel est laissé sous agitation pendant 12 heures à TA. Le DCM est évaporé et l'huile résiduelle est précipitée dans l'éther.
On obtient l'exemple **1-3 dia2** sous forme d'une poudre blanche après filtration : (2,53 g ; 87 %).
LCMS [M+H] = 524 (C₂₉H₃₂Cl₂FN₃O₃)
RMN ¹H (300 MHz, DMSO) : δ 8,60-9,35 (m ; 1,6 H) ; 7,77 (br ; 0,8 H) ; 7,30-7,40 (m ; 2,0 H) ; 7,00-7,23 (m ; 5,1 H) ; 6,80-7,00 (m ; 3,1 H) ; 6,54-6,76 (m ; 3,0 H) ; 4,56 (d ; *J* = 13,3 Hz ; 1,0 H) ; 3,88-4,16 (m ; 3,0 H) ; 3,00-3,30 (m ; 3,1 H) ; 2,65-2,96 (m ; 1,7 H) ; 1,52-2,00 (m ; 1,6 H) ; 1,00 (t ; *J* = 7,4 Hz ; 2,4 H) ; 0,59 (dd ; *J* = 15,6 Hz ; *J* = 6,7 Hz ; 3,5 H).
en appliquant le même procédé à partir de l'exemple **I-1 dia1,** on obtient l'exemple **1-3 dia1** sous forme d'une poudre blanche après filtration : (63 mg).
LCMS [M+H] = 524 (C₂₉H₃₂Cl₂FN₃O₃)
RMN ¹H (300 MHz, DMSO) : δ 8,65-9,6 (br ; 1,2 H) ; 7,77 (br ; 0,8 H) ; 7,30-7,40 (m ; 2,0 H) ; 6,36-7,25 (m ; 11,7 H) ; 4,40-4,60 (m ; 0,8 H) ; 4,00-4,12 (m ; 2,0 H) ; 3,76-3,98 (m ; 0,9 H) ; 3,37-3,63 (m ; 0,9 H) ; 2,65-3,30 (m ; 4,0 H) ; 1,77-2,06 (m ; 1,6 H) ; 0,89-1,06 (m; 6,1 H).

### Exemples I-4a et 1-4b : Chlorhydrate des diastéréoisomères du 2-Chloro-N-[1-(4-fluoro-phenyl)-2-((R)-3-isopropyl-piperazin-1-yl)-2-oxo-ethyl]-N-(4-phenoxy-phenyl)-acetamide.

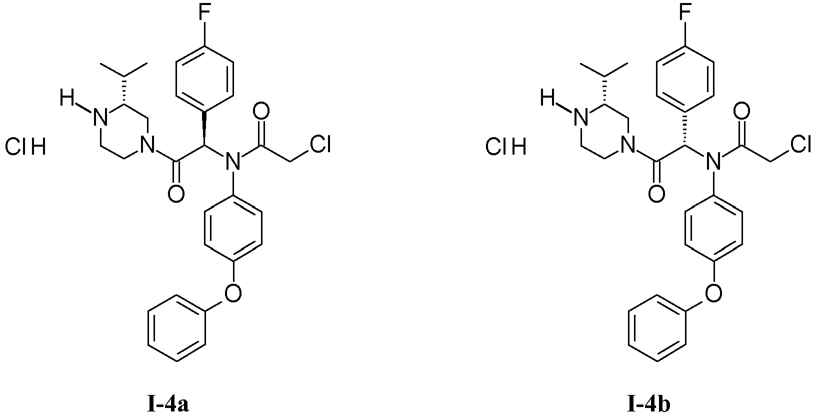

Le même protocole que celui des exemples I-3a et I-3b a été utilisé à partir de chacun des diastéréoisomères I-2a et I-2b.

### A partir du premier diastéréoisomère de l'exemple I-2 (I-4 dia1) :

Récupération d'une poudre blanche après filtration : (95 mg) LCMS [M+H] = 524 (C₂₉H₃₂Cl₂FN₃O₃)
RMN ¹H (300 MHz, DMSO) : δ 8,65-9,6 (br ; 1,3 H) ; 7,77 (br ; 0,4 H) ; 7,30-7,40 (m ; 2,0 H) ; 6,36-7,25 (m ; 11,8 H) ; 4,40-4,60 (m ; 0,9 H) ; 4,00-4,12 (m ; 2,0 H) ; 3,76-3,98 (m ; 1,0 H) ; 3,37-3,63 (m ; 1,0 H) ; 2,65-3,30 (m ; 3,8 H) ; 1,77-2,06 (m ; 1,8 H) ; 0,89-1,06 (m; 6,1 H).

### A partir du second diastéréoisomère de l'exemple I-2 (1-4 dia2) :

Récupération d'une poudre blanche après filtration : (95 mg)
LCMS [M+H] = 524 (C₂₉H₃₂Cl₂FN₃O₃)
RMN ¹H (300 MHz, DMSO) : δ 8,60-9,35 (m ; 1,7 H) ; 7,77 (br ; 0,9 H) ; 7,30-7,40 (m ; 2,0 H) ; 7,00-7,23 (m ; 5,0 H) ; 6,80-7,00 (m ; 3,0 H) ; 6,54-6,76 (m ; 2,9 H) ; 4,56 (d ; *J* = 13,3 Hz ; 1,0 H) ; 3,88-4,16 (m ; 3,0 H) ; 3,00-3,30 (m ; 3,1 H) ; 2,65-2,96 (m ; 1,7 H) ; 1,52-2,06 (m ; 1,9 H) ; 1,00 (t ; *J* = 7,2 Hz ; 2,4 H) ; 0,59 (dd ; *J* = 15,4 Hz ; *J* = 6,7 Hz ; 3,3 H).

### Exemples I-5a et I-5b : Diastéréoisomères de l'ester tert-butylique de l'acide 4-[-2-[(2-chloro-acétyl)-(2-méthyl-4-phénoxy-phényl)-amino]-2-(4-fluoro-phényl)-acétyl]-(S)-2-isopropyl-pipérazine-1-carboxylique.

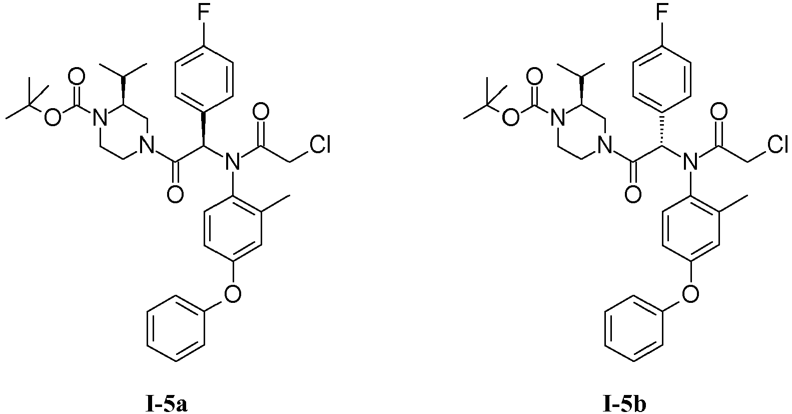

### Stade 1 : Ester tert-butylique de l'acide 4-[2-(4-fluoro-phényl)-2-(2-méthyl-4-phénoxy-phénylamino)-acétyl]-(S)-2-isopropyl-pipérazine-1-carboxylique (8) :

A une solution d'acide (4-fluoro-phényl)-(2-méthyl-4-phénoxy-phénylamino)-acétique (9,29 g ; 26,4 mmol) dans le DCM (150 mL) en présence d'un équivalent de DIEA (4,6 mL) est ajouté une solution du chlorhydrate de la Boc-alpha-(S)-isopropyle-pipérazine (7,00 g ; 26,4 mmol) en présence d'1 éq de DIEA (4,6 mL) dans 50 mL de DCM, suivi du HBTU (10,00 g ; 26,4 mmol). Le milieu est laissé sous agitation pendant 12 heures. Le milieu est lavé à l'eau et la phase organique est séchée sur MgSO₄. Après évaporation, l'huile récupérée est purifiée par flash chromatographie sur gel de silice avec l'éluant cyclohexane-acétate d'éthyle 80-20.
Récupération d'une mousse blanche (14,13 g ; 95 %).
LCMS [M+H] = 562 (C₃₃H₄₀FN₃O₄)

### Stade 2 : Ester tert-butylique de l'acide 4-[-2-[(2-chloro-acétyl)-(2-méthyl-4-phénoxy-phényl)-amino]-2-(4-fluoro-phényl)-acétyl]-(S)-2-isopropyl-pipérazine-1-carboxylique

A une solution de **8** (14,13 g ; 25,1 mmol) dans 250 mL de DCM en présence de NaHCO₃ (8,40 g ; 100,0 mmol), est ajouté le chlorure de chloroacétyle (4,00 mL ; 50,0 mmol). Le milieu est laissé sous agitation pendant 12 heures. Le milieu est lavé à l'eau et la phase organique est séchée sur MgSO₄. Après évaporation, l'huile récupérée est purifiée par flash chromatographie sur gel de silice avec l'éluant cyclohexane-acétate d'éthyle 90-10 jusqu'à 50-50 progressivement.
Récupération des deux diastéréoisomères sous forme de mousse incolore :

### Diastéréoisomère le moins polaire (I-5 dia1) (3,83 g ; 24 %)

LCMS [M+H] = 639 (C₃₅H₄₁ClFN₃O₅)
RMN ¹H (300 MHz, CD₂Cl₂) : δ 7,94-8,57 (m ; 1,0 H) ; 7,35 (t ; *J* = 7,9 Hz ; 2,0 H) ; 7,07-7,27 (m ; 3,0 H) ; 6,74-6,95 (m ; 5,0 H) ; 6,58 (br d ; *J* = 2,6 Hz ; 1,1 H) ; 6,51 (br s ; 0,2 H) ; 6,41 (s ; 0,8 H) ; 6,31 (br s ; 0,3 H) ; 4,62 (d ; *J* = 13,5 Hz ; 0,7 H) 4,39 (d ; *J* = 13,5 Hz ; 0,3 H) ; 3,53-4,05 (m ; 4,8 H) ; 3,04-3,46 (m ; 0,8 H) ; 2,41-2,96 (m ; 2,1 H) ; 2,04-2,23 (m ; 0,8 H) ; 1,82-1,95 (m ; 2,2 H) ; 1,43 (br s ; 10,1 H) ; 1,07 (d ; *J* = 6,5 Hz ; 2,1 H) ; 0,90 (d ; *J* = 6,5 Hz ; 2,3 H) ; 0,63 (d ; *J* = 6,5 Hz ; 1,0 H) ;0,29 (d ; *J* = 6,5 Hz ; 0,8 H).

### Diastéréoisomère le plats polaire (I-5 dia2) (4,40 g ; 27 %)

LCMS [M+H] = 639 (C₃₅H₄₁ClFN₃O₅)
RMN ¹H (300 MHz, CDCl₃) : δ 8,00-8,10 (m ; 1,0 H) ; 7,30-7,40 (m ; 2,1 H) ; 6,98-7,18 (m ; 3,2 H) ; 6,73-6,90 (m ; 5,3 H) ; 6,52-6,58 (m ; 1,0 H) ; 6,34-6,39 (m ; 1,0 H) ; 4,71 (d ; *J =* 13,5 Hz ; 0,7 H) ; 4,49 (d ; *J =* 13,5 Hz ; 0,4 H) ; 3,50-4,00 (m ; 4,7 H) ; 3,10-3,30 (m ; 0,7 H) ; 2,86-3,07 (m ; 0,4 H) ; 2,54-2,85 (m ; 1,5 H) ; 2,30-2,47 (m ; 0,4 H) ; 1,80-1,87 (m ; 2,8 H) ; 1,54-1,60 (m ; 2,5 H) ; 1,42 (br s ; 8,8 H) ; 1,19 (d ; *J=* 6,6 Hz; 1,1 H); 1,00 (d ; *J* = 6,6 Hz ; 1,5 H) ; 0,88 (d ; *J* = 6,6 Hz ; 1,2 H) ;0,64 (d ; *J* = 6,6 Hz ; 1,5 H).

### Exemples I-6a et I-6b : Diastéréoisomères de l'ester tert-butylique de l'acide 4-[-2-[(2-chloro-acétyl)-(4-phénoxy-phényl)-amino]-2-(4-fluoro-phényl)-acétyl]-(R)-2-isopropyl-pipérazine-1-carboxylique

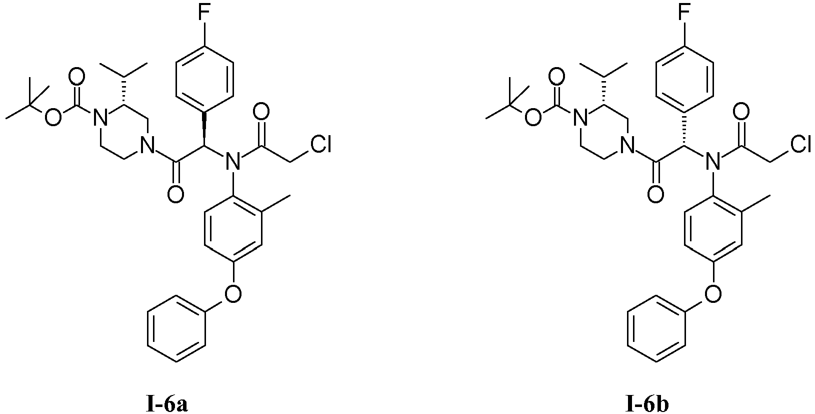

Ces deux diastéréoisomères ont été préparés de la même manière qu'à l'exemple précédent sous forme de mousse incolore :

### Diastéréoisomère le moins polaire (I-6 dia1) (97 mg ; 30 %)

LCMS [M+H] = 639 (C₃₅H₄₁ClFN₃O₅)
RMN ¹H (300 MHz, CD₂Cl₂) : δ 7,94-8,57 (m ; 0,9 H) ; 7,35 (t ; *J* = 7,9 Hz ; 1,9 H) ; 7,05-7,25 (m ; 3,1 H) ; 6,72-6,93 (m ; 5,0 H) ; 6,58 (br d ; *J* = 2,6 Hz ; 1,1 H) ; 6,41 (s ; 0,8 H) ; 6,31 (br s ; 0,3 H) ; 4,63 (d ; *J* = 13,5 Hz ; 0,8 H) 4,40 (d ; *J* = 13,5 Hz ; 0,3 H) ; 3,51-4,06 (m ; 4,8 H) ; 3,03-3,45 (m ; 1,0 H) ; 2,41-2,96 (m ; 1,6 H) ; 2,02-2,21 (m ; 0,8 H) ; 1,82-1,95 (m ; 2,1 H) ; 1,43 (br s ; 10,1 H) ; 1,07 (d ; *J* = 6,5 Hz ; 2,1 H) ; 0,90 (d ; *J* = 6,5 Hz ; 2,3 H) ; 0,63 (d ; *J* = 6,5 Hz ; 1,0 H) ;0,29 (d ; *J* = 6,5 Hz ; 0,8 H).

### Diastéréoisomère le plus polaire (I-6 dia2) (90 mg ; 28 %)

LCMS [M+H] = 639 (C₃₅H₄₁ClFN₃O₅)
RMN ¹H (300 MHz, CDCl₃) : δ 8,00-8,10 (m ; 1,0 H) ; 7,30-7,40 (m ; 2,1 H) ; 6,98-7,18 (m ; 3,1 H) ; 6,73-6,90 (m ; 5,1 H) ; 6,52-6,58 (m ; 1,0 H) ; 6,34-6,39 (m ; 1,0 H) ; 4,70 (d ; *J* = 13,5 Hz ; 0,7 H) ; 4,49 (d ; *J* = 13,5 Hz ; 0,4 H) ; 3,50-4,00 (m ; 4,8 H) ; 3,10-3,30 (m ; 0,7 H) ; 2,86-3,07 (m ; 0,4 H) ; 2,54-2,85 (m ; 1,5 H) ; 2,30-2,47 (m ; 0,4 H) ; 1,80-1,87 (m ; 2,8 H) ; 1,54-1,60 (m ; 2,6 H) ; 1,42 (br s ; 8,6 H) ; 1,20 (d ; *J* = 6,6 Hz; 1,1 H); 1,01 (d; *J* = 6,6Hz; 1,5 H); 0,89 (d ; *J* = 6,6 Hz; 1,2 H) ;0,64 (d ; *J* = 6,6 Hz ; 1,5 H).

### Exemples I-7a et I-7b : Chlorhydrate des diastéréoisomères du 2-chloro-N-[-1-(4-fluoro-phényl)-2-((S)-3-isopropyl-pipérazin-1-yl)-2-oxo-éthyl]-N-(2-méthyl-4-phénoxy-phényl)-acétamide

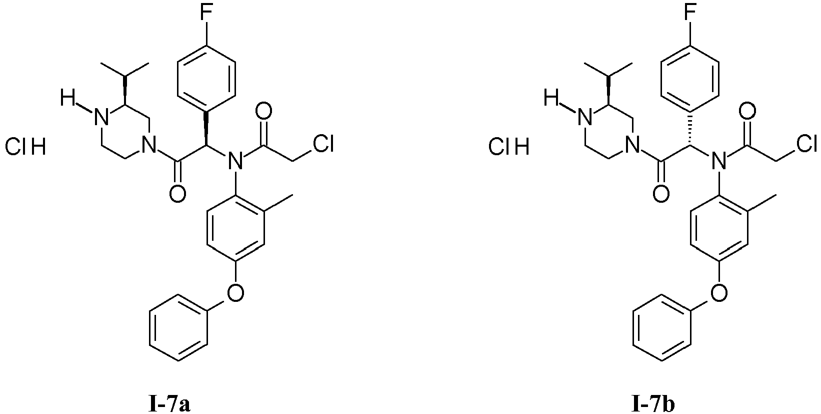

A une solution de l'un des diastéréoisomères I-5a et I-5b dans 50 mL de DCM est ajouté le HCl gaz par bullage. Le milieu réactionnel est laissé sous agitation pendant 12 heures à TA. Le DCM est évaporé et l'huile résiduelle est précipitée dans l'éther éthylique.

### A partir du premier diastéréoisomère de l'exemple I-5 (1-7 dia1) :

Récupération d'une poudre blanche après filtration : (26 mg)
LCMS [M+H] = 538 (C₃₀H₃₄Cl₂FN₃O₃)
RMN ¹H (300 MHz, DMSO) : δ 8,79-9,33 (m ; 1,3 H) ; 7,83 (t ; *J* = 9,0 Hz ; 1,0 H) ; 7,24-7,40 (m ; 4,0 H) ; 6,97-7,15 (m ; 3,1 H) ; 6,73-6,89 (m ; 3,2 H) ; 6,64 (d ; *J* = 2,7 Hz ; 0,9 H) ; 6,51-6,59 (m ; 1,0 H) ; 4,40-4,55 (br m ; 1,1 H) ; 3,86-4,09 (m ; 3,6 H) ; 3,45-3,60 (m ; 0,7 H) ; 2,78-3,05 (m ; 2,8 H) ; 1,79-2,00 (m ; 4,5 H) ; 1,61-1,77 (m ; 0,7 H) ; 0,97 (d ; *J* = 6,7 Hz ; 6,0 H).

### A partir du second diastéréoisomère de l'exemple I-5 (I-7 dia2) :

Récupération d'une poudre blanche après filtration : (2,62 g)
LCMS [M+H] = 538 (C₃₀H₃₄Cl₂FN₃O₃)
RMN ¹H (300 MHz, DMSO) : δ 8,78-9,51 (m ; 1,9 H) ; 7,82 (t ; *J* = 8,9 Hz ; 0,9 H) ; 7,20-7,41 (m ; 4,0 H) ; 6,97-7,16 (m ; 3,1 H) ; 6,71-6,90 (m ; 3,1 H) ; 6,61-6,70 (m ; 1,9 H) ; 4,46-4,60 (br m ; 1,0 H) ; 3,85-4,15 (m ; 3,1 H) ; 3,00-3,30 (m ; 3,0 H) ; 2,57-2,96 (m ; 1,8 H) ; 1,43-1,98 (m ; 4,3 H) ; 1,00 (dd ; *J* = 8,8 Hz ; *J* = 7,0 Hz ; 2,7 H) ; 0,71 (d ; *J* = 6,8 Hz ; 1,6 H) ; 0,65 (d ; *J* = 6,8 Hz ; 1,5 H).

### Exemple I-8 : Chlorhydrate du 2-chloro-N-[-1-(4-fluoro-phényl)-2-((R)-3-isopropyl-pipérazin-1-yl)-2-oxo-éthyl]-N-(2-méthyl-4-phénoxy-phényl)-acetamide

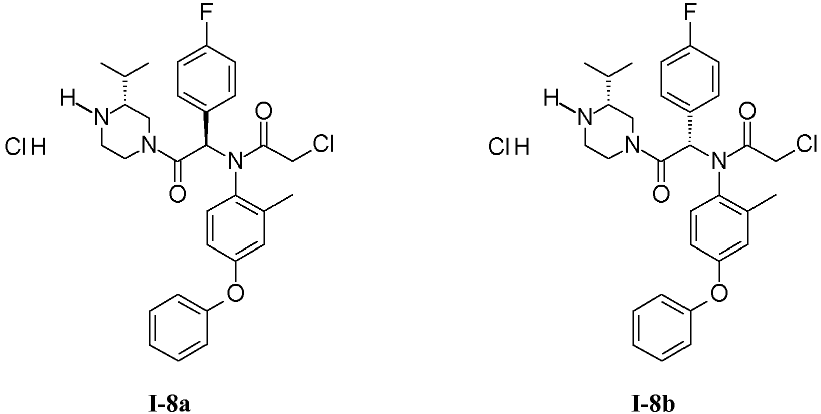

Le même protocole que celui de l'exemple précédent a été utilisé à partir de chacun des diastéréoisomères de l'exemple I-6.

### A partir du premier diastéréoisomère de l'exemple I-6 (I-8 dia1) :

Récupération d'une poudre blanche après filtration : (34 mg ; 56 %)
LCMS [M+H] = 538 (C₃₀H₃₄Cl₂FN₃O₃)
RMN ¹H (300 MHz, DMSO) : δ 8,79-9,33 (m ; 1,3 H) ; 7,83 (t ; *J =* 9,0 Hz ; 0,9 H) ; 7,24-7,40 (m ; 4,0 H) ; 6,97-7,15 (m ; 3,1 H) ; 6,73-6,89 (m ; 3,1 H) ; 6,64 (d ; *J =* 2,7 Hz ; 1,0 H) ; 6,51-6,59 (m ; 1,0 H) ; 4,41-4,56 (br m ; 1,1 H) ; 3,86-4,09 (m ; 3,4 H) ; 3,45-3,60 (m ; 0,7 H) ; 2,78-3,05 (m ; 2,8 H) ; 1,79-2,00 (m ; 4,4 H) ; 1,61-1,77 (m ; 0,8 H) ; 0,97 (d ; *J* = 6,7 Hz ; 6,0 H).

### A partir du second diastéréoisomère de l'exemple I-6 (I-8 dia2) :

Récupération d'une poudre blanche après filtration : (30 mg ; 54 %)
LCMS [M+H] = 538 (C₃₀H₃₄Cl₂FN₃O₃)
RMN ¹H (300 MHz, DMSO) : δ 8,78-9,51 (m ; 1,5 H) ; 7,82 (t ; *J* = 8,9 Hz ; 1,0 H) ; 7,20-7,41 (m ; 4,0 H) ; 6,97-7,16 (m ; 3,1 H) ; 6,71-6,90 (m ; 3,2 H) ; 6,61-6,70 (m ; 1,9 H) ; 4,46-4,60 (br m ; 1,0 H) ; 3,85-4,15 (m ; 3,1 H) ; 3,00-3,30 (m ; 2,9 H) ; 2,57-2,96 (m ; 1,8 H) ; 1,43-1,98 (m ; 4,3 H) ; 1,00 (dd ; *J* = 8,8 Hz ; *J* = 7,0 Hz ; 2,7 H) ; 0,71 (d ; *J* = 6,8 Hz ; 1,6 H) ; 0,65 (d ; *J* = 6,8 Hz ; 1,5 H).

En utilisant les mêmes modes opératoires et les mêmes modes de séparation par chromatographie sur silice que ci-dessus, les exemples suivants ont été préparés à partir d'anilines et de pipérazines diversement substituées. Ils ont été isolés soit sous forme de mélange de deux ou quatre diastéréoisomères (un numéro d'exemple pour la même structure chimique), soit sous forme de diastéréoisomères séparés. Dans ce dernier cas, la nomenclature a / b a été utilisée pour désigner chacun des diastéréoisomères.

| | |
|---|---|
| 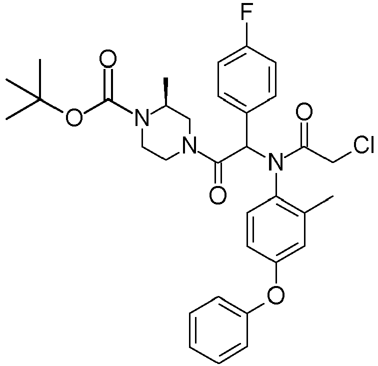 | 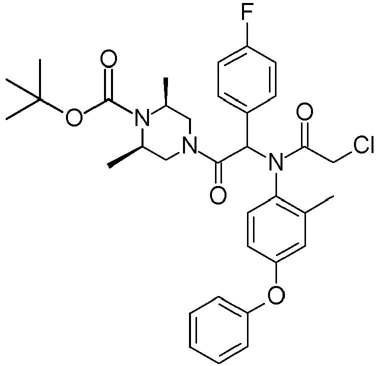 |
| I-9 | I-10 |
| 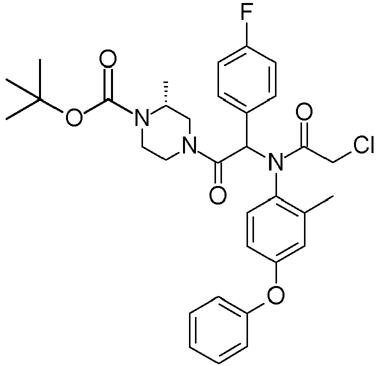 | 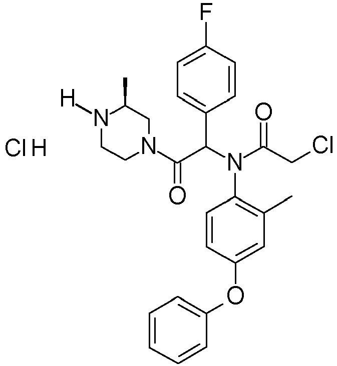 |
| I-11 | I-12 |
| 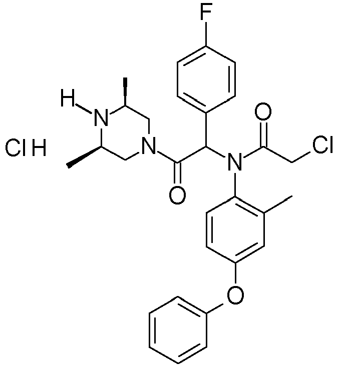 | 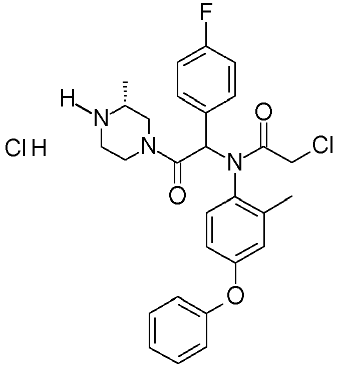 |
| I-13 | I-14 |
| 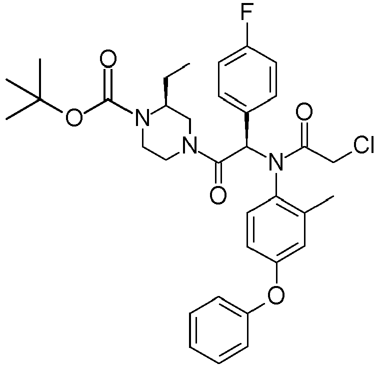 | 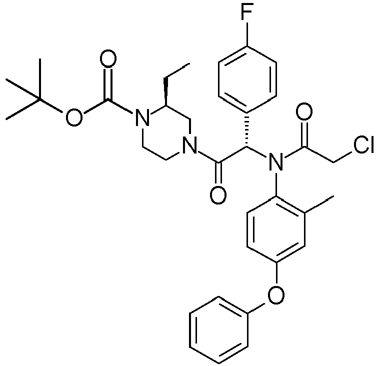 |
| I-15a | I-15b |
| 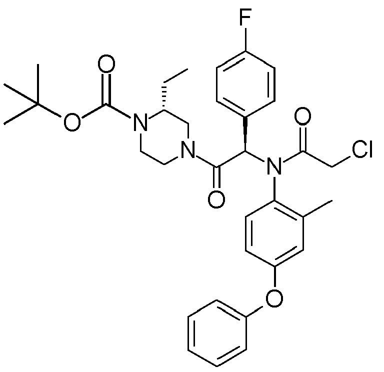 | 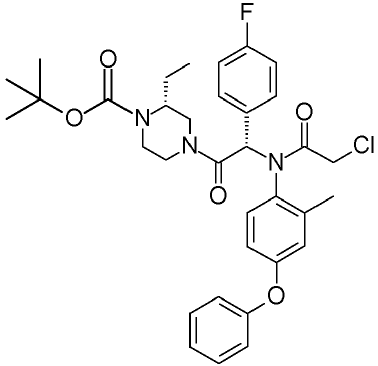 |
| I-16a | I-16b |
| 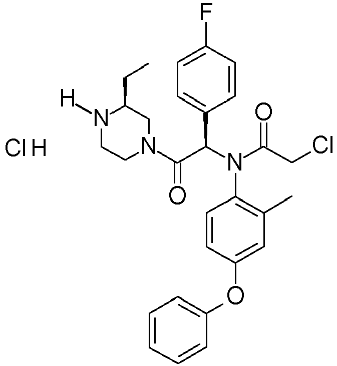 | 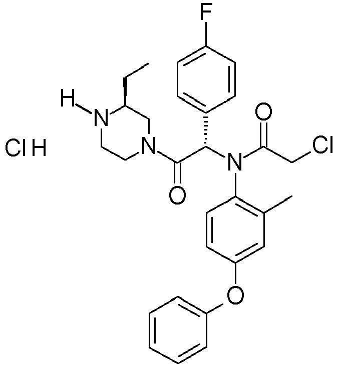 |
| I-17a | I-17b |
| 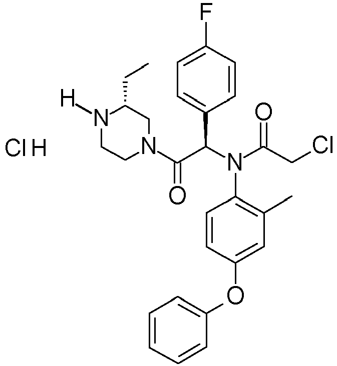 | 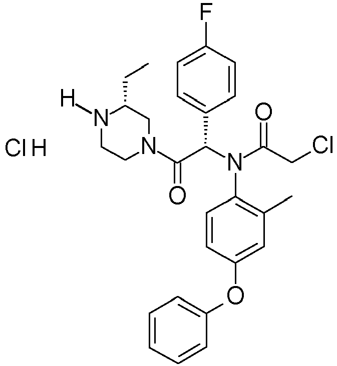 |
| I-18a | I-18b |
| 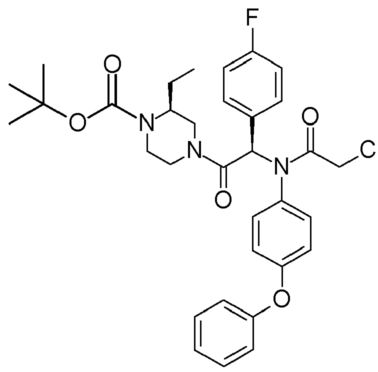 | 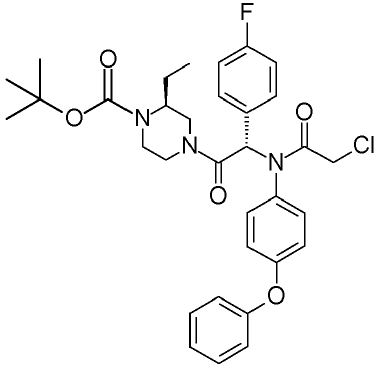 |
| I-19a | I-19b |
| 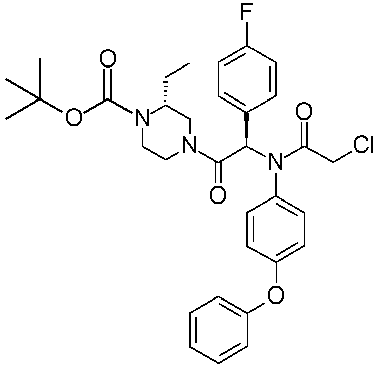 | 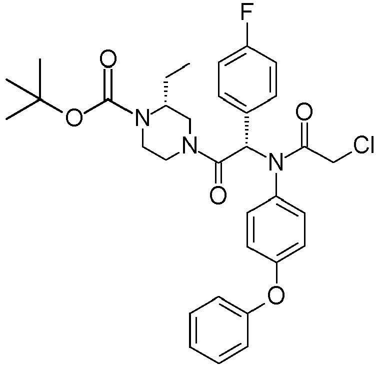 |
| I-20a | I-20b |
| 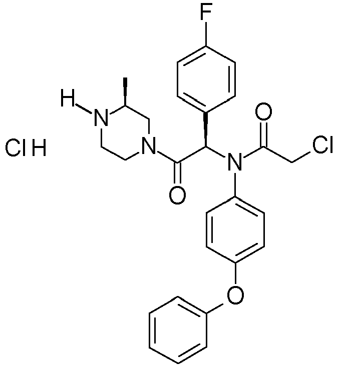 | 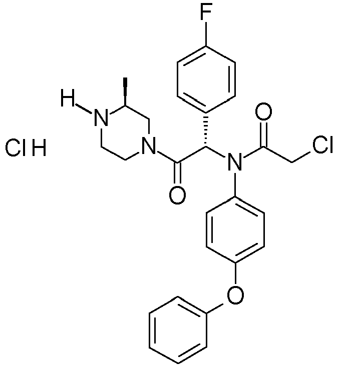 |
| I-21a | I-21b |
| 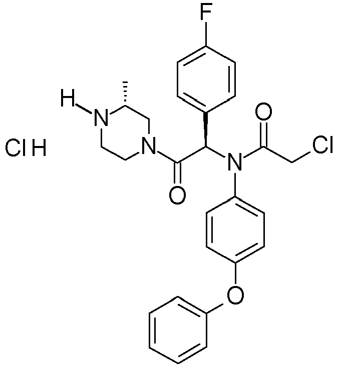 | 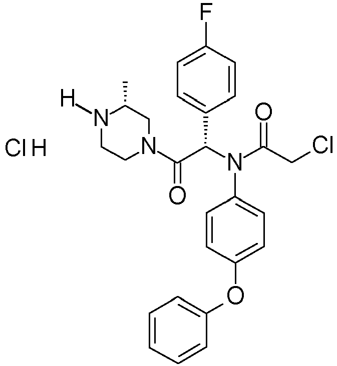 |
| I-22a | I-22b |
| 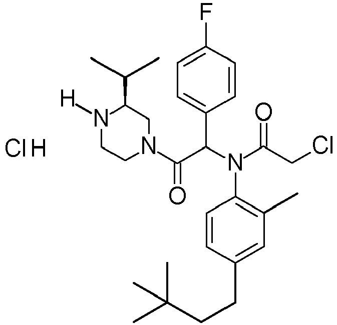 | 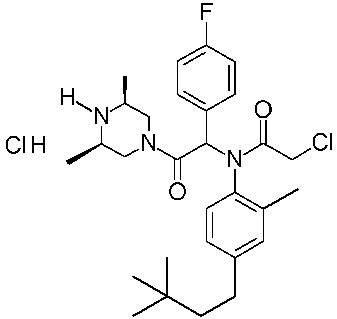 |
| I-23 | I-24 |
| 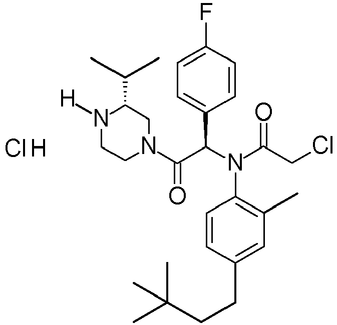 | 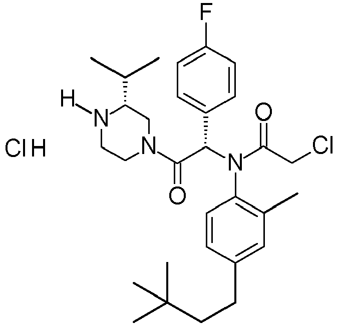 |
| I-25a | I-25b |
| 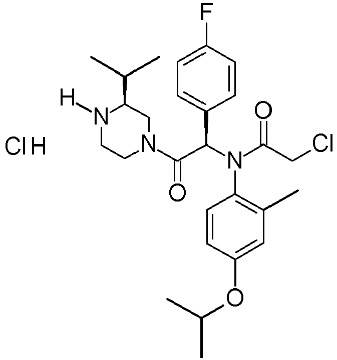 | 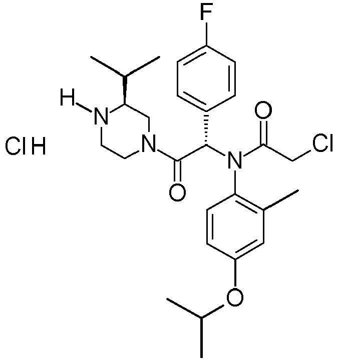 |
| I-26a | I-26b |
| 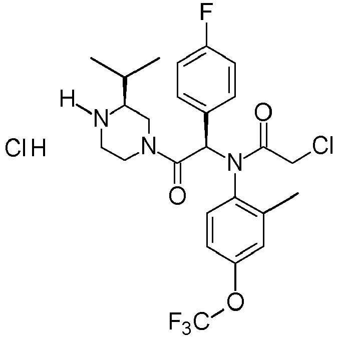 | 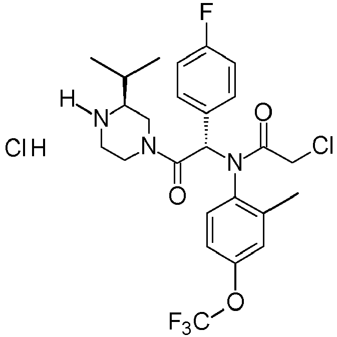 |
| I-27a | I-27b |

| | |
|---|---|
| Les exemples suivants ont été obtenus en remplaçant l'ester éthylique de l'acide (4-fluoro-phényl)-oxo-acétique par le thiophène-2-glyoxylate d'éthyle et en utilisant les mêmes modes opératoires que précédemment. | |

| | |
|---|---|
| 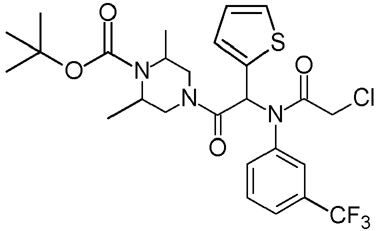 | 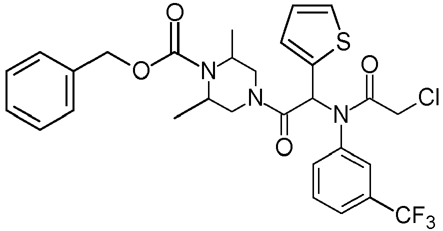 |
| I-28 | I-29 |
| 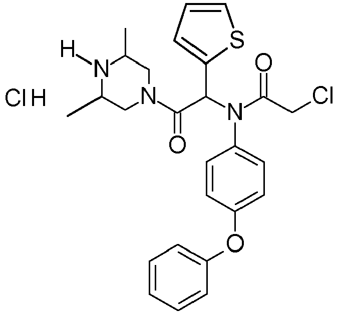 | 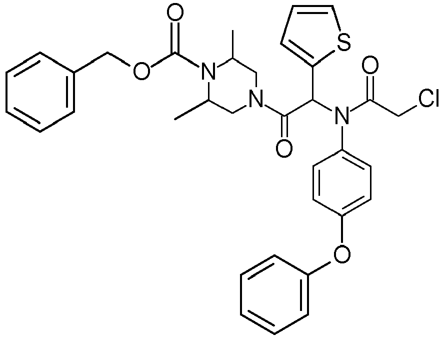 |
| I-30 | I-31 |
| 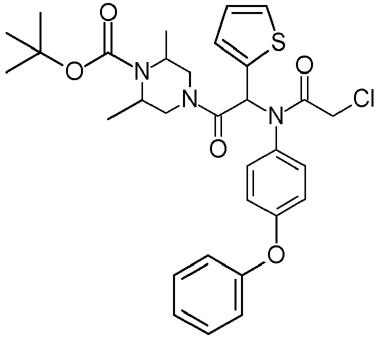 | 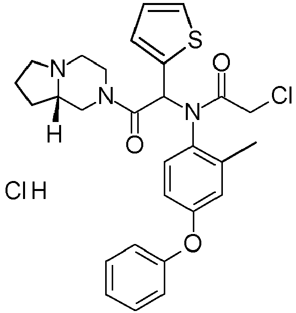 |
| I-32 | I-33 |
| 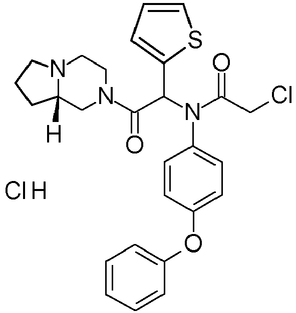 | 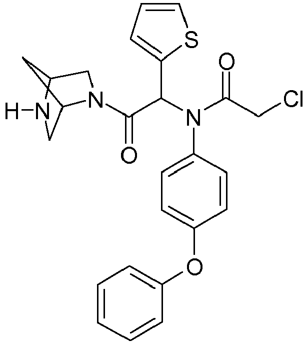 |
| I-34 | I-35 |
| 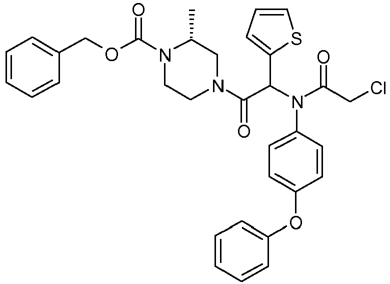 | 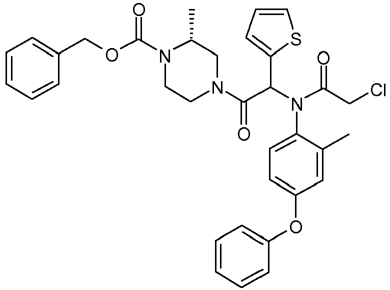 |
| I-36 | I-37 |
| 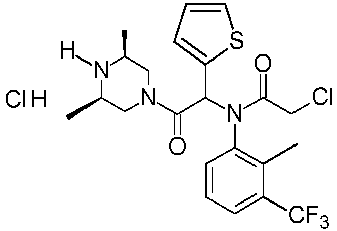 | 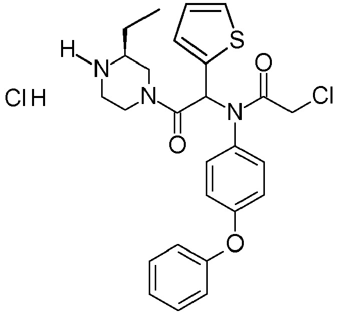 |
| I-3 | I-39 |
| 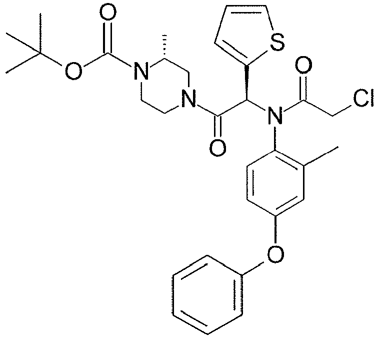 | 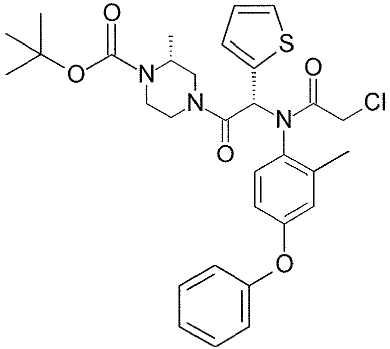 |
| I-40a | I-40b |
| 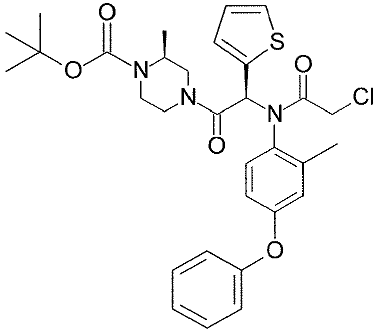 | 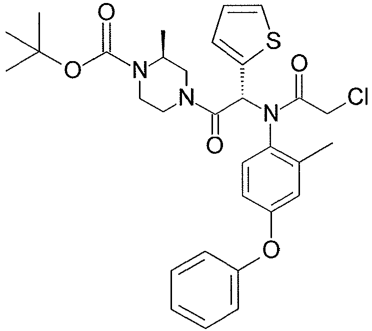 |
| I-41a | I-41b |
| 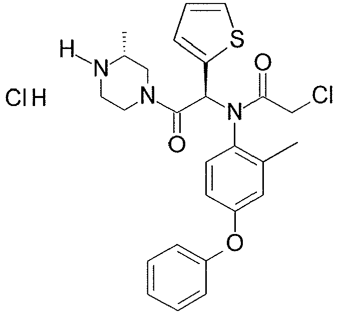 | 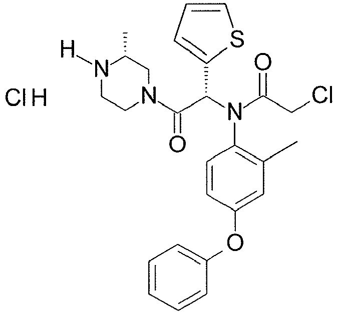 |
| I-42a | I-42b |
| 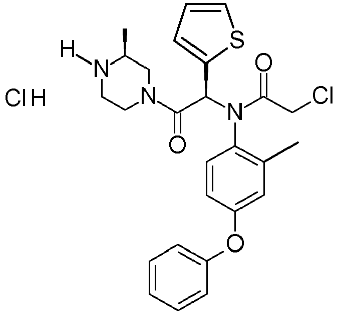 | 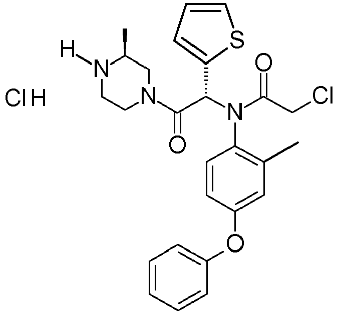 |
| I-43a | I-43b |
| 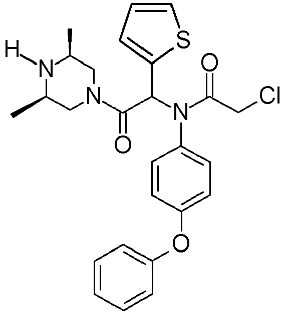 | 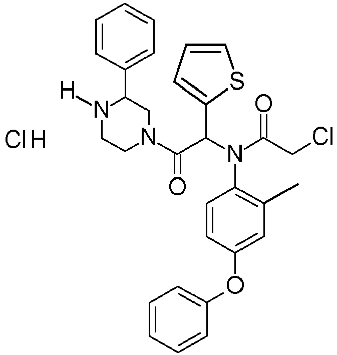 |
| I-44 | I-45 |
| 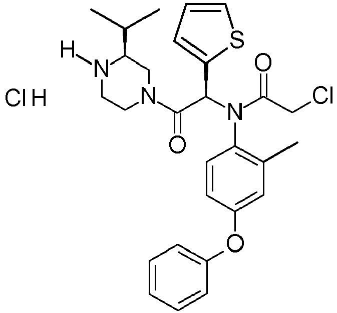 | 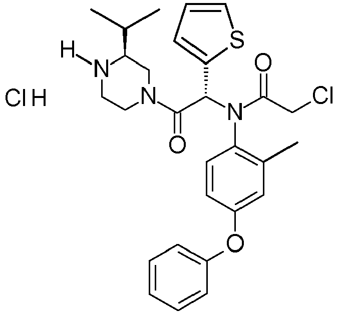 |
| I-46a | I-46b |
| 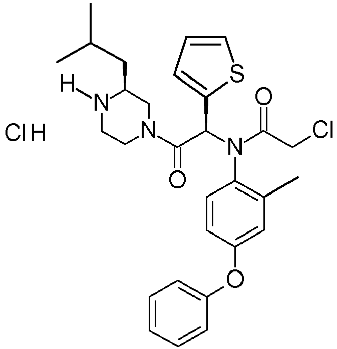 | 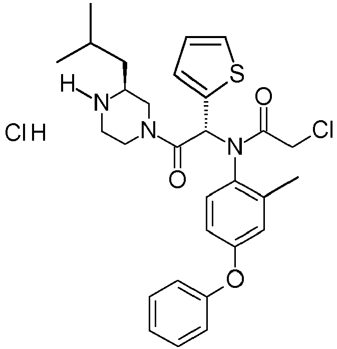 |
| I-47a | I-47b |
| 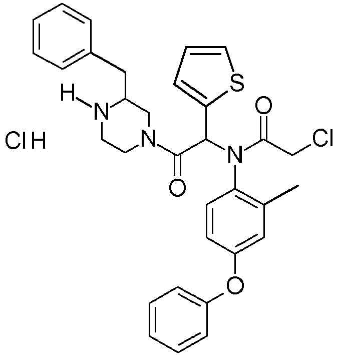 | 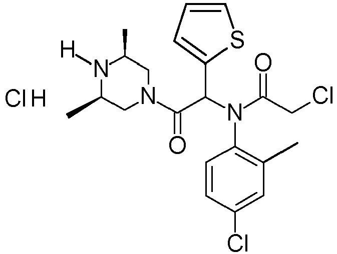 |
| I-48 | I-49 |
| 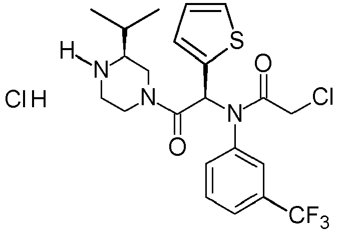 | 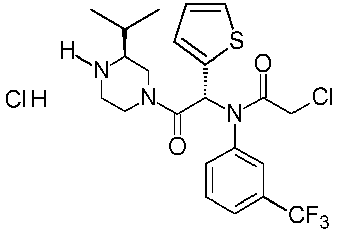 |
| I-50a | I-50b |
| 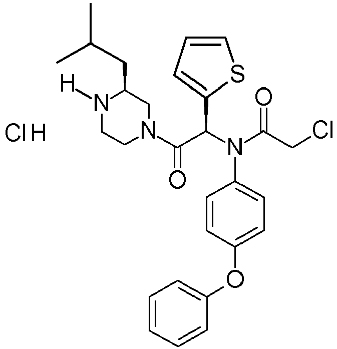 | 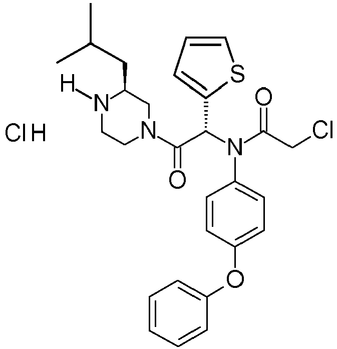 |
| I-51a | I-51b |
| 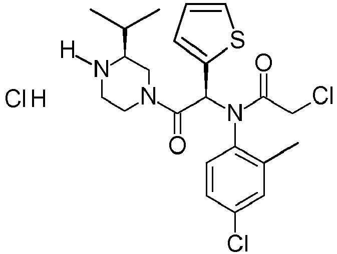 | 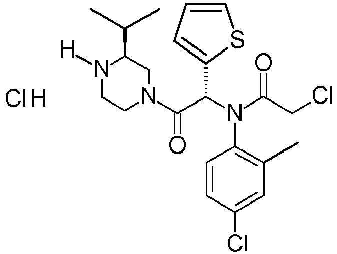 |
| I-52a | I-52b |
| 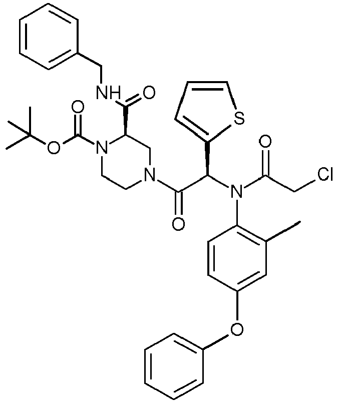 | 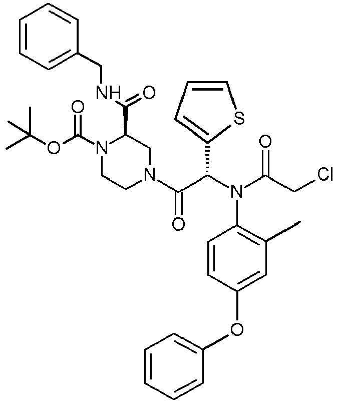 |
| I-53a | I-53b |
| 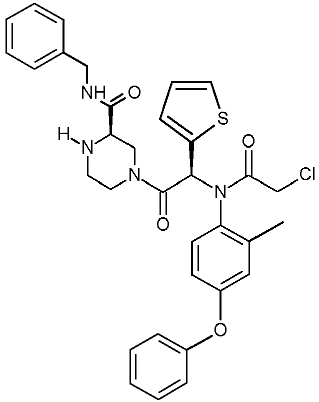 | 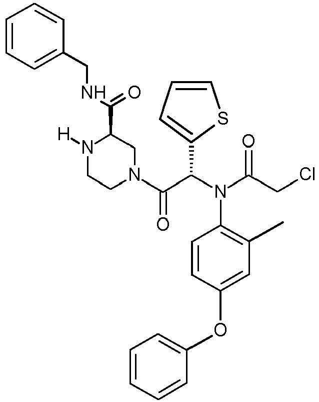 |
| I-54a | I-54b |
| 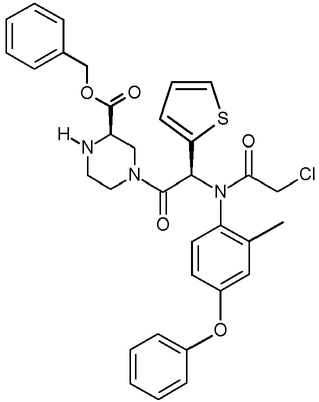 | 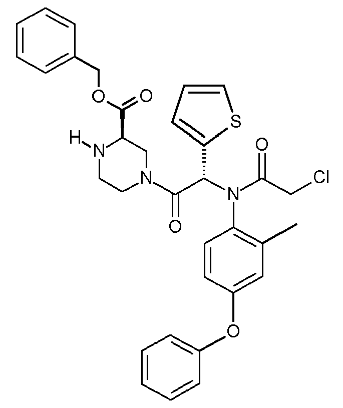 |
| I-55a | I-55b |
| 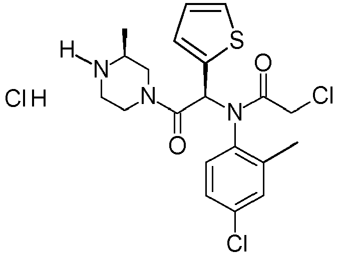 | 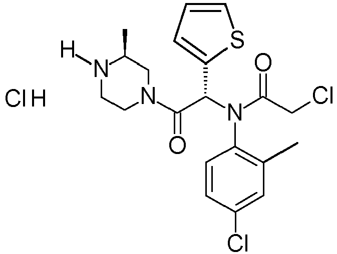 |
| I-56a | I-56b |
| 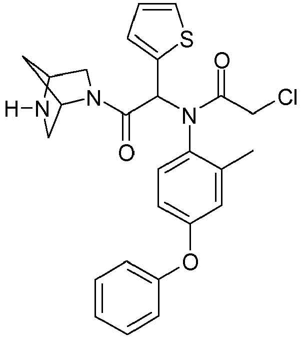 | 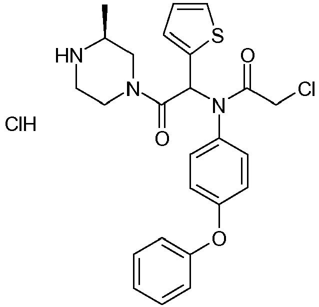 |
| I-57 | I-58 |
| 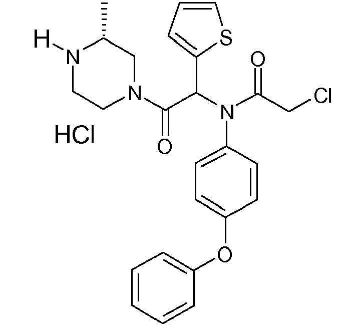 | 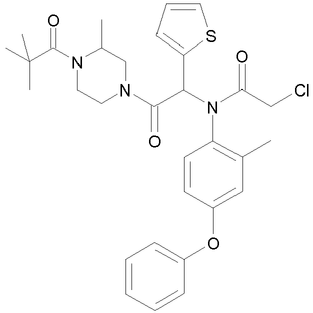 |
| I-59 | I-60 |
| 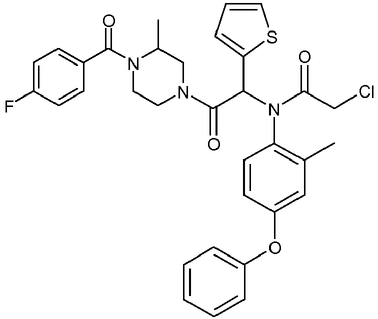 | 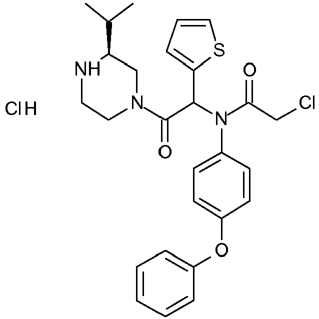 |
| I-61 | I-62 |
| 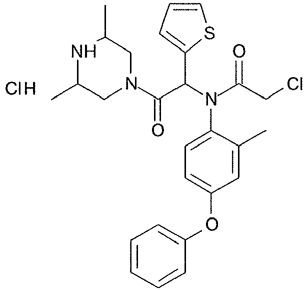 | |
| 1-63 | |

### II - Etude pharmacologique des composés selon la présente invention

### 1) Tests de cytotoxicité

Les effets des composés de l'invention sur la prolifération de cellules cancéreuses ont été étudiés sur différentes lignées cellulaires cancéreuses humaines d'origines tissulaires différentes (MCF-7 : cancer du sein, MCF-7/adr : cancer du sein résistant à l'adriamycine, HL-60 : leucémie aigüe à promyélocyte, HL-60/R10 : leucémie aigüe à promyélocyte résistant à la doxorubicine, HT29 : adénocarcinome du côlon, Mia Paca2 : tumeur du pancréas, Panc-1 : tumeur du pancréas exocrine, SK-OV-3 : tumeur de l'ovaire résistante au cis-platine et à l'adriamycine). Les cellules cancéreuses utilisées pour cette étude ont été incubées à 37°C en présence de l'un des composés de l'invention ajouté dans le milieu de culture à différentes concentrations.

Les lignées cellulaires cancéreuses proviennent de l'ATCC (American Type Culture Collection) dans le cas de MCF-7, de l'Hôpital de la Pitié Salpetrière pour MCF-7/adr et d'Oncodesign (Dijon, France) pour HL-60, HL-60/R10, HT29, MiaPaCa2, Panc-1 et SK-OV-3. Elles ont été cultivées en milieu RPMI 1640 contenant 2 mM L-Glutamine et supplémenté avec 10 % de sérum de veau foetal (Lonza ; Verviers, Belgique). Toutes les lignées cellulaires ont été maintenues en culture à 37°C dans une atmosphère humide contenant 5 % de CO₂. La prolifération cellulaire a été évaluée en utilisant le réactif ViaLight® Plus Assay Kit (Lonza ; Verviers, Belgique) en respectant les instructions du fabriquant. Les cellules ont été ensemencées dans des plaques de culture de 96 puits compatibles à la lecture de la luminescence (plaques blanches à fond transparent) à raison de 5 000 à 10 000 cellules par puits dans 100 µl de milieu de culture. Après 24 heures de préincubation à 37°C, les composés de l'invention dissous dans du diméthylsulfoxyde (DMSO) ont été ajoutés individuellement dans chacun des puits à raison de 0,5 µl par puits. Après 72 heures d'incubation à 37°C dans une atmosphère humide contenant 5 % de CO₂, 50 µl de tampon de lyse sont ajoutés dans chaque puits, 15 minutes après, 100 µl d'agent de mesure d'ATP ont été ajoutés. Les plaques ont été lues au luminomètre afin d'évaluer la viabilité cellulaire. Les données obtenues ont été traitées par ordinateur afin d'obtenir l'EC₅₀, c'est à dire la valeur de la concentration de chacun des composés qui induit 50 % de viabilité cellulaire en comparaison du control (0,5 % DMSO seul).

Les résultats obtenus sont présentés dans les tableaux 1 et 2 suivants.

**Tableau 1 : Résultats obtenus avec les lignées cellulaires HL-60, HL60/R10, MCF-7 et MCF-7/adr (EC₅₀ exprimées en nM)**

| **Exemple N°** | **EC₅₀ (nM)** | | | |
|---|---|---|---|---|
| | **HL-60** | **HL60/R10** | **MCF-7** | **MCF-7 /adr** |
| I-1 dia1 | 1799 | -219 | -2500 | 338 |
| I-1 dia2 | 824 | 23 | 1304 | 39 |
| I-2 dial | 728 | 40 | 2091 | 49 |
| I-2 dia2 | 2061 | 472 | 2500 | 645 |
| I-3 dia1 | 1311 | 15 | 927 | 55 |
| I-3 dia2 | 504 | 2 | 301 | 9 |
| I-4 dia1 | 648 | 4 | 315 | 13 |
| I-4 dia2 | 1321 | 62 | 863 | 166 |
| I-7 dia1 | 2500 | 604 | 2500 | 618 |
| I-7 dia2 | 1938 | 26 | 1954 | 98 |
| I-8 dia1 | 2029 | 54 | 1740 | 148 |
| I-8 dia2 | 1737 | 312 | 2410 | 477 |
| I-9 | 771 | 99 | 2500 | 108 |
| I-10 | 641 | 131 | 2500 | 151 |
| I-11 | 939 | 68 | 2500 | 102 |
| I-12 | 2321 | 82 | 1787 | 421 |
| I-13 | 1989 | 88 | 2500 | 532 |
| I-14 | 1848 | 95 | 2500 | 232 |
| I-16 dial | 1478 | 450 | 2500 | 527 |
| I-16 dia2 | 1913 | 317 | 2500 | 443 |
| I-17 dia1 | 2313 | 353 | 2500 | 898 |
| I-17 dia2 | 1604 | 49 | 1994 | 152 |
| I-18 dia1 | 1596 | 140 | 2500 | 485 |
| I-18 dia2 | 352 | 44 | 591 | 152 |
| I-19 dia1 | 516 | 97 | 2500 | 128 |
| I-19 dia2 | 453 | 24 | 2500 | 35 |
| I-20 dia1 | 196 | 80 | 1571 | 73 |
| I-20 dia2 | 1478 | 139 | 2500 | 165 |
| I-21 dia1 | 2447 | 13 | 1950 | 93 |
| I-21 dia2 | 630 | 8 | 869 | 10 |
| I-22 dia1 | 1995 | 17 | 569 | 131 |
| I-22 dia2 | 1149 | 18 | 430 | 34 |
| I-23 | 559 | 12 | nd | nd |
| I-24 | 626 | 15 | nd | nd |
| I-25 dia1 | 1122 | 43 | nd | nd |
| I-25 dia2 | 819 | 103 | nd | nd |
| I-26 dia1 | 2447 | 13 | 1950 | 93 |
| I-26 dia2 | 630 | 8 | 869 | 10 |
| I-27 dia1 | 1995 | 17 | 569 | 131 |
| I-27 dia2 | 1149 | 18 | 430 | 34 |
| I-28 | 623 | 206 | nd | nd |
| I-29 | 939 | 38 | 1032 | 54 |
| I-30 | 202 | 10 | 1006 | 44 |
| I-31 | 766 | 64 | 2500 | 89 |
| I-32 | 175 | 78 | 2500 | 58 |
| I-33 | 2500 | 157 | 2500 | 394 |
| I-34 | 1123 | 30 | 1775 | 100 |
| I-35 | 300 | 249 | 632 | 863 |
| I-36 | 372 | 28 | 2500 | 24 |
| I-37 | 967 | 154 | 2500 | 156 |
| I-38 | 2500 | 189 | 2500 | 851 |
| I-39 | 1814 | 9 | 776 | 52 |
| I-40 dia1 | 2129 | 248 | 2500 | 371 |
| I-40 dia2 | 2500 | 487 | 2500 | 827 |
| I-43 dia2 | 1886 | 20 | 1424 | 126 |
| I-44 | 215 | 7 | nd | nd |
| I-45 | 2136 | 17 | nd | nd |
| I-46 dia1 | 2500 | 318 | 2500 | 544 |
| I-46 dia2 | 449 | 10 | 1184 | 58 |
| I-47 dia1 | 936 | 54 | nd | nd |
| I-47 dia2 | 849 | 8 | nd | nd |
| I-48 | 1991 | 10 | nd | nd |
| I-49 | 1166 | 321 | nd | nd |
| I-50 dia1 | 2102 | 259 | nd | Nd |
| I-50 dia2 | 841 | 8 | nd | nd |
| I-51 dia1 | 145 | 96 | nd | nd |
| I-51 dia2 | 3 | 1 | nd | nd |
| I-52 dia1 | 2500 | 194 | nd | nd |
| I-52 dia2 | 223 | 24 | nd | nd |
| I-53 dia1 | 1981 | 645 | 2064 | 462 |
| I-53 dia2 | 547 | 305 | nd | nd |
| I-58 | 1058 | 11 | 870 | 88 |
| I-59 | 956 | 16 | 745 | 106 |

| | | | | |
|---|---|---|---|---|
| nd = non déterminé | | | | |

**Tableau 2 : Résultats obtenus avec d'autres lignées cellulaires (EC₅₀ exprimées en nM)**

| **Exemple N°** | **EC₅₀ (nM)** | | | |
|---|---|---|---|---|
| | **HT29** | **Mia PaCa2** | **Panc-1** | **SK-OV-3** |
| I-3 dia2 | 1 | 2 | 1 | 1 |
| I-7 dia2 | 8 | 13 | 5 | 7 |
| I-43 dia2 | 10 | 18 | 7 | 9 |
| I-46 dia2 | 6 | 10 | 4 | 6 |
| I-50 dia2 | 9 | 2200 | 9 | 11 |

| | | | | |
|---|---|---|---|---|
| Les tableaux 3 et 4 suivants illustrent le gain d'activité cytotoxique sur la lignée résistante HL60/R10 obtenu avec les composés possédant une pipérazine substituée en position alpha de l'azote 4 de la pipérazine par rapport à une pipérazine non substituée et/ou à une autre position de la pipérazine. La meilleure activité cytotoxique est obtenue par la configuration absolue (S) de cette substitution. | | | | |

**Tableau 3 : Résultats obtenus avec différentes substitutions de la pipérazine**

| **Exemple** | **EC₅₀ (nM)** | |
|---|---|---|
| | **HL-60** | **HL-60/R10** |
| 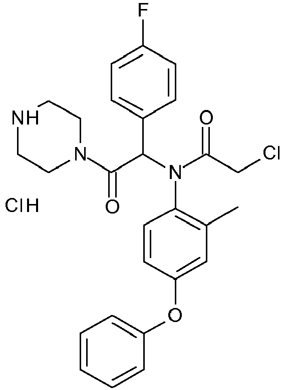 | 1627 | 226 |
| Ex. comparatif | | |
| 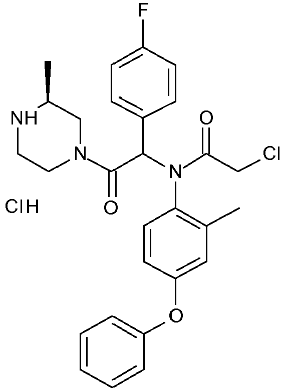 | 2321 | 82 |
| I-12 | | |
| 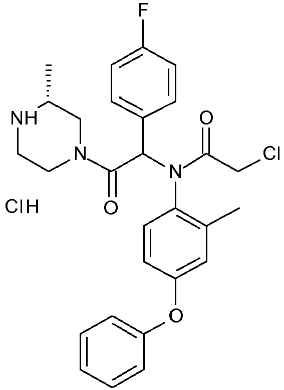 | 1989 | 88 |
| I-14 | | |
| 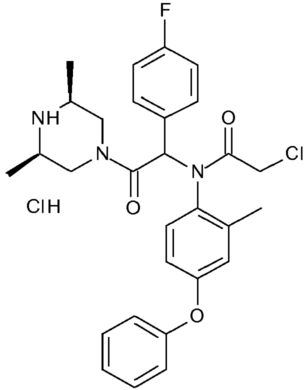 | 1848 | 95 |
| I-13 | | |

**Tableau 4 : Résultats obtenus avec différentes substitutions de la pipérazine**

| **Exemple** | **EC₅₀ (nM)** | |
|---|---|---|
| | **HL-60** | **HL-60/R10** |
| 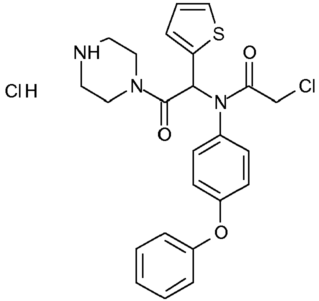 | 579 | 21 |
| Ex. comparatif | | |
| 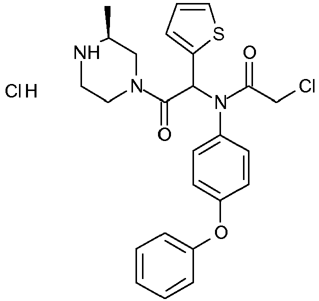 | 1058 | 11 |
| I-58 | | |
| 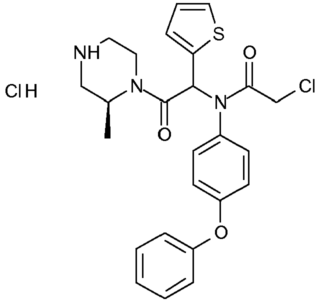 | 662 | 65 |
| Ex. comparatif | | |
| 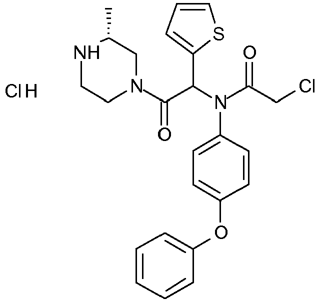 | 966 | 16 |
| I-59 | | |
| 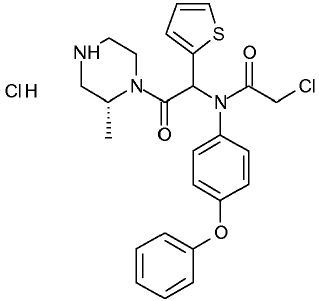 | 311 | 38 |
| Ex. comparatif | | |

### 2) Détermination de la solubilité aqueuse

La solubilité aqueuse est un paramètre physicochimique important pour améliorer les propriétés ADME (Absorption, Distribution, Métabolisme et Excrétion) des molécules (Drug-like properties: concepts, structure design and methods, Edward Harvel Kerns, Li Di ; Academic Press, 2008).

La solubilité aqueuse de chaque composé a été mesurée à pH 7,4. Elle a été mesurée par HPLC sur les surnageants obtenus par centrifugation après saturation des milieux par un excès de composé après 24 h d'agitation et à une température de 20°C. La préparation et le traitement des échantillons sont réalisés par un robot.

Le tableau 5 montre le gain de solubilité aqueuse obtenu pour un composé selon l'invention I-58 par rapport à une pipérazine non substituée ou substituée sur une autre position.

**Tableau 5 : Solubilité aqueuse obtenus avec différentes substitutions de la pipérazine**

| **Exemple** | **Solubilité (µg/mL)** |
|---|---|
| 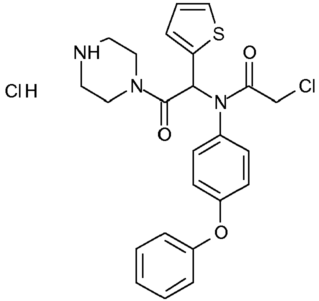 | 68 |
| Ex. comparatif | |
| 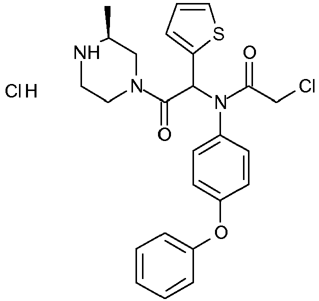 | 203 |
| I-58 | |
| 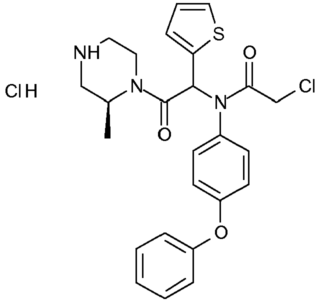 | 105 |
| Ex. comparatif | |
| 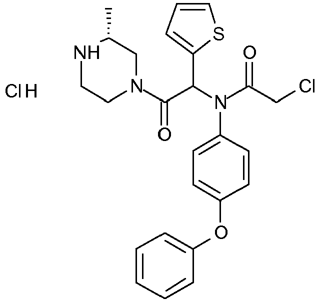 | 152 |
| I-59 | |
| 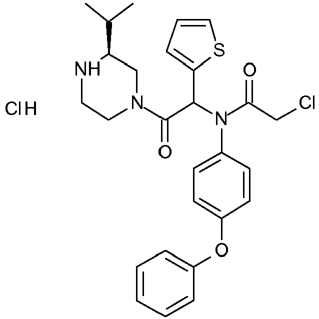 | 50 |
| I-62 | |

### 3) Paramètres pharmacocinétiques chez la souris

Le comportement pharmacocinétique des composés est un pré-requis nécessaire à son utilisation raisonnée en expérimentation *in vivo.* Les composés sont administrés en solution DMSO par voie intraveineuse (IV) ou voie orale (PO) chez des souris balb/c. Des échantillons de sang sont prélevés à des temps allant de 5 minutes à 6 heures, les plasmas sont récupérés et la concentration des composés dans chaque échantillon est dosée par LC/MS/MS. Les données obtenues permettent l'élaboration de courbes temps-concentration et la détermination des paramètres fondamentaux tels que le temps de ½ vie plasmatique du composé (T½), l'aire sous la courbe à un temps donné (AUCt) et la concentration maximale obtenue (Cmax). Le tableau 6 montre le gain apporté par une substitution de la pipérazine sur les paramètres pharmacocinétiques des composés administrés par voie intraveineuse à la dose de 10 mg/kg.

Sur la figure 1, sont données les courbes temps-concentration plasmatique pour une souris suite à l'administration de I-43 dia2 par voie IV et PO. Le composé I-43 dia2 présente ainsi une bonne biodisponiblité chez la souris, particulièrement par voie orale.

**Tableau 6 : Paramètres pharmacocinétiques obtenus avec diverses substitutions de la pipérazine**

| **Exemple** | **Cmax** (**ng**/**mL**) | **AUCt** (**ng**/**mL*****h**) | **AUCinf** (**ng**/**mL*****h**) | **t**_{**1/**2} **(h)** |
|---|---|---|---|---|
| 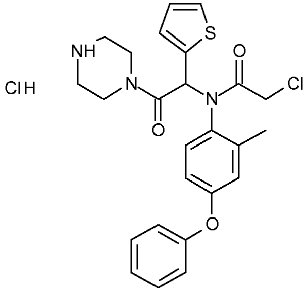 | 1469,50 | 1278,25 | 1321,68 | 1,38 |
| Ex. comparatif | | | | |
| 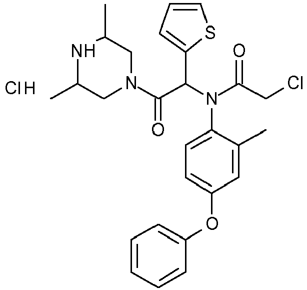 | 3797,6 | 3287,08 | 3837,61 | 2,64 |
| I-63 | | | | |
| 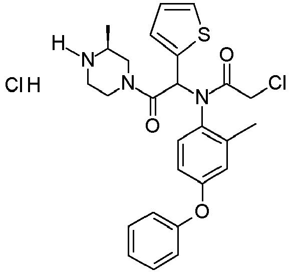 | 1424,37 | 1677,94 | 2057,38 | 2,47 |
| I-43 dia 2 | | | | |

## Revendications

1. Composé de formule générale (I) suivante : 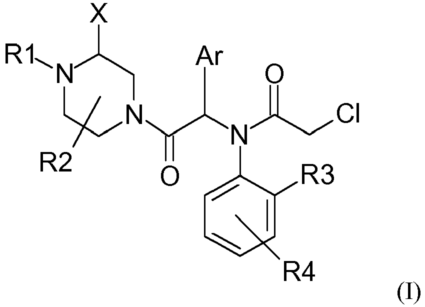 ainsi que ses sels pharmaceutiquement acceptables, ses stéréoisomères ou mélanges de stéréoisomères en toutes proportions, en particulier un mélange d'énantiomères, et notamment un mélange racémique,
pour laquelle :
- X représente un groupement (C₁-C₆)alkyle, phényle, benzyle, C(O)OR5, ou C(O)NHR5,
- R1 représente un atome d'hydrogène ou un groupement C(O)H, C(O)R6, ou C(O)OR6,
- R2 représente un atome d'hydrogène ou un groupement (C₁-C₆)alkyle, ou R2 forme avec R1 ou X une chaîne hydrocarbonée saturée de manière à former un cycle à 5 ou 6 chaînons, notamment à 5 chaînons,
- R3 représente un atome d'hydrogène ou d'halogène ou un groupement (C₁-C₆)alkyle, ou (C₁-C₆)alkoxy,
- R4 représente un atome d'hydrogène ou d'halogène, CN, NO₂, ou un groupement (C₁-C₆)alkyle, (C₁-C₆)alkoxy, aryloxy, benzyloxy, ou hétéroaryloxy, ledit groupement étant éventuellement substitué par un ou plusieurs atomes d'halogène,
- Ar représente un groupement thiophényle ou un groupement phényle éventuellement substitué par un ou plusieurs atomes d'halogène, et
- R5 et R6 représentent, indépendamment l'un de l'autre, un groupement (C₁-C₆)alkyle, aryl-(C₁-C₆)alkyle ou aryle, ledit groupement étant éventuellement substitué par un ou plusieurs atomes d'halogène.

2. Composé selon la revendication 1, **caractérisé en ce qu'**il répond à la formule (I-bis) suivante :

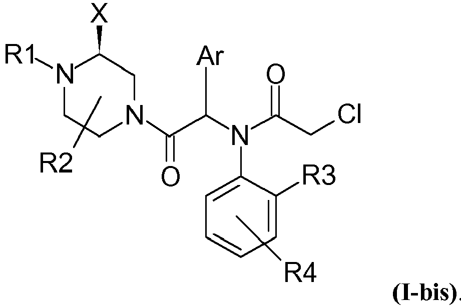

3. Composé selon l'une quelconque des revendications 1 et 2, **caractérisé en ce que** Ar représente un groupement thiophényle ou un groupement phényle substitué par un ou plusieurs atomes de fluor tel que le 4-fluoro-phényle.

4. Composé selon l'une quelconque des revendications 1 à 3, **caractérisé en ce que** R4 représente un atome d'hydrogène ou d'halogène, ou un groupement (C₁-C₆)alkyle, (C₁-C₆)alkoxy, ou aryloxy, ledit groupement étant éventuellement substitué par un ou plusieurs atomes d'halogène, en particulier de fluor.

5. Composé selon l'une quelconque des revendications 1 à 4, **caractérisé en ce que** R3 représente un atome d'hydrogène ou un groupement (C₁-C₆)alkyle, tel que méthyle.

6. Composé selon l'une quelconque des revendications 1 à 5, **caractérisé en ce que** X représente un groupement (C₁-C₆)alkyle, phényle, ou benzyle; R1 et R2 représentent un atome d'hydrogène ; R3 représente un atome d'hydrogène ou un groupement (C₁-C₆)alkyle, en particulier (C₁-C₃)alkyle; R4 représente un atome d'halogène, ou un groupement (C₁-C₆)alkyle, (C₁-C₆)alkoxy, aryloxy, ou benzyloxy, ledit groupement étant éventuellement substitué par un ou plusieurs atomes d'halogène ; Ar représente un groupement thiophényle ou un groupement phényle éventuellement substitué par un atome de fluor tel que le 4-fluoro-phényle ; et R5 et R6 représentent, indépendamment l'un de l'autre, un groupement (C₁-C₆)alkyle, aryl-(C₁-C₆)alkyle, ou aryle, ledit groupement étant éventuellement substitué par un ou plusieurs atomes de fluor.

7. Composé selon la revendication 1, **caractérisé en ce que** le composé est choisi parmi :
| | |
|---|---|
| 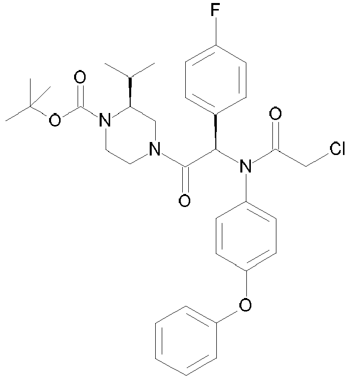 | 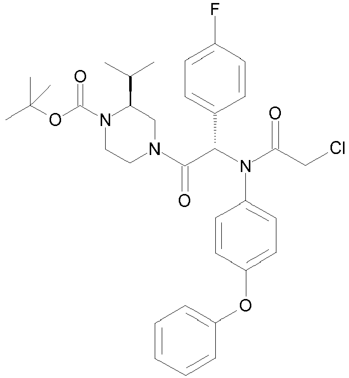 |
| I-1a | I-1b |
| 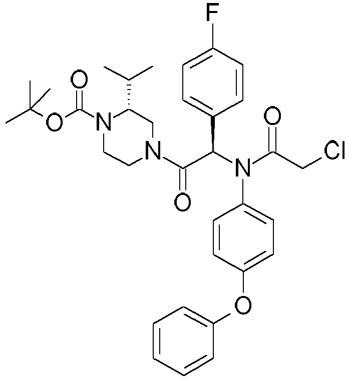 | 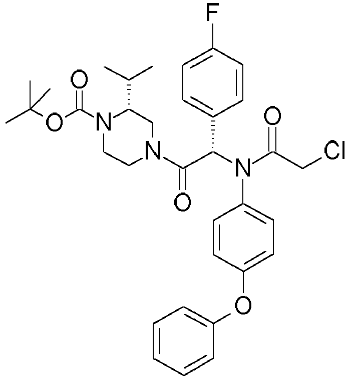 |
| I-2a | I-2b |
| 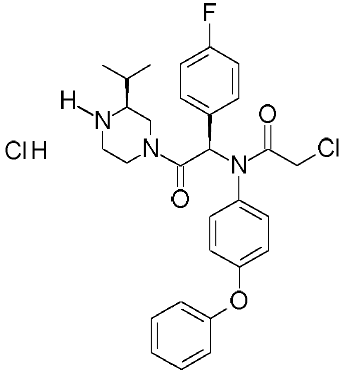 | 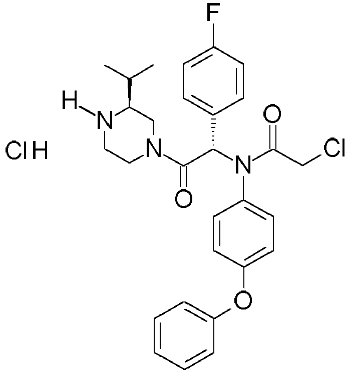 |
| I-3a | I-3b |
| 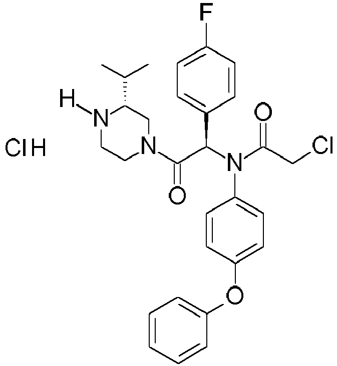 | 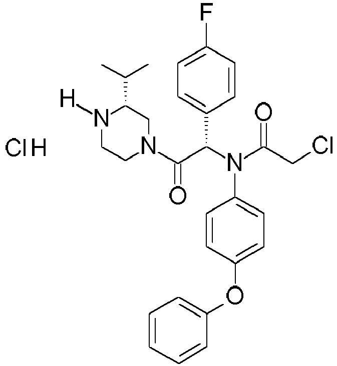 |
| I-4a | I-4b |
| 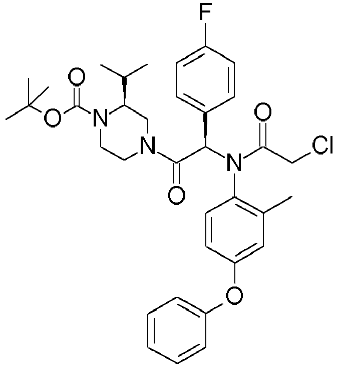 | 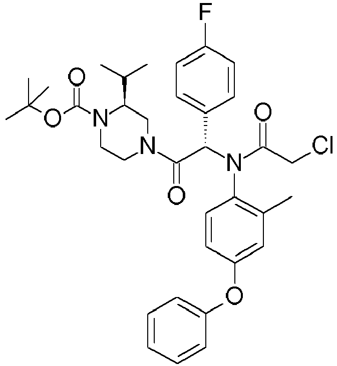 |
| I-5a | I-5b |
| 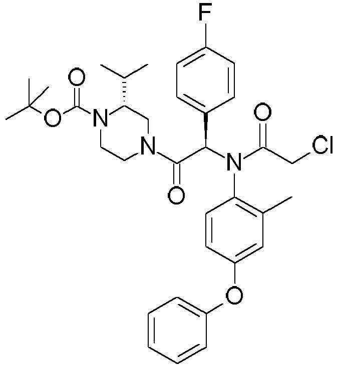 | 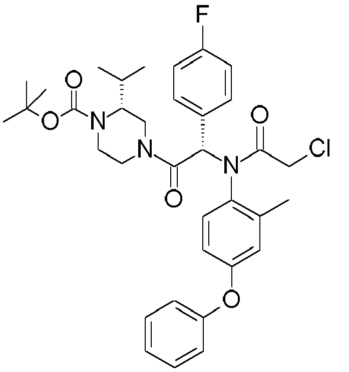 |
| I-6a | I-6b |
| 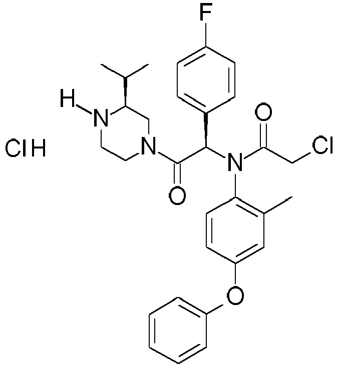 | 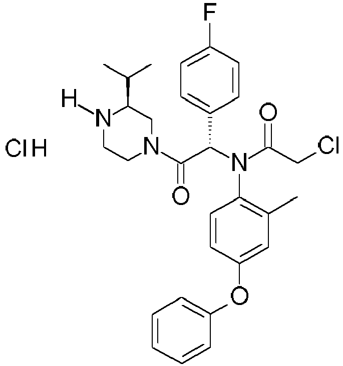 |
| I-7a | I-7b |
| 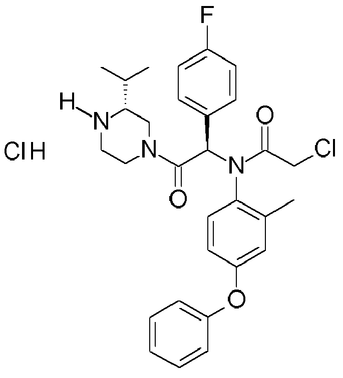 | 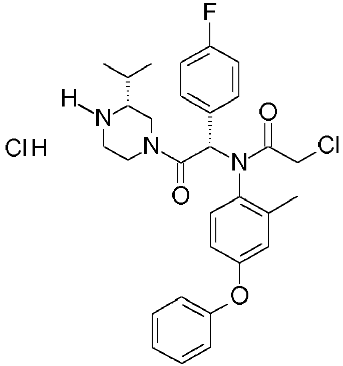 |
| I-8a | I-8b |
| 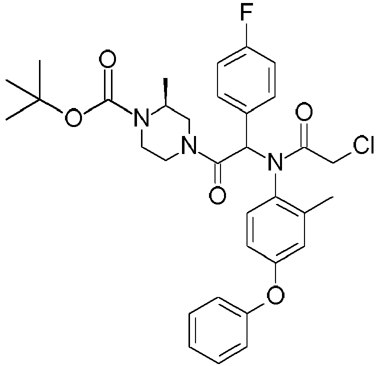 | 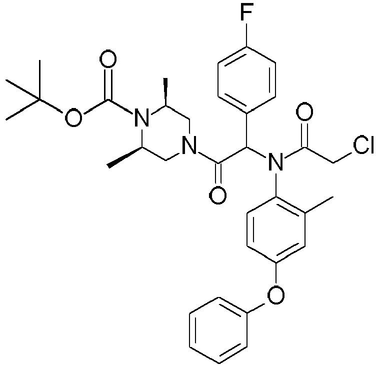 |
| I-9 | I-10 |
| 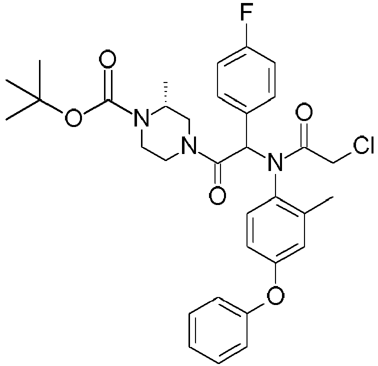 | 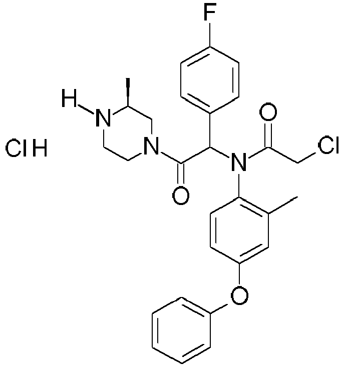 |
| I-11 | I-12 |
| 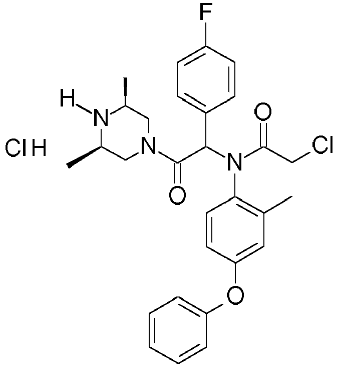 | 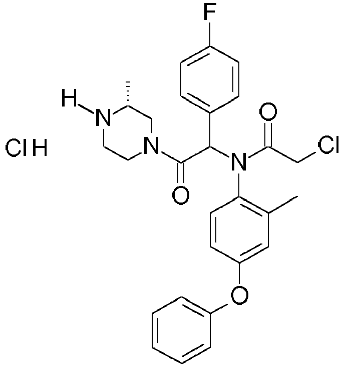 |
| I-13 | I-14 |
| 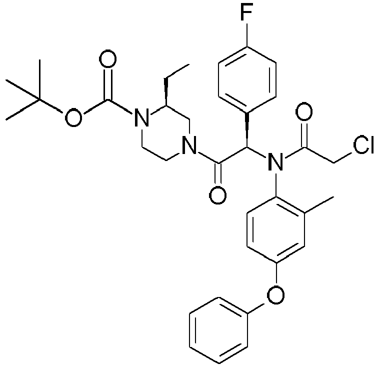 | 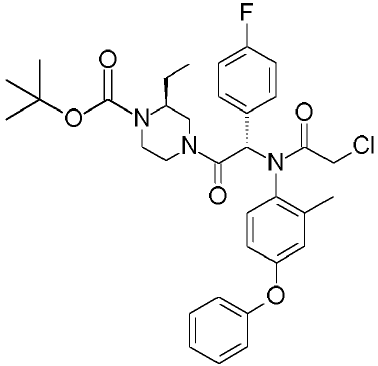 |
| I-15a | I-15b |
| 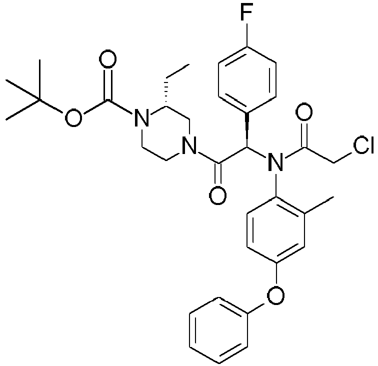 | 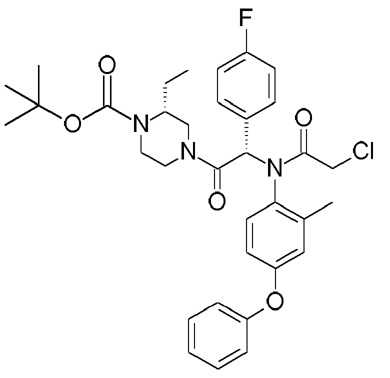 |
| I-16a | I-16b |
| 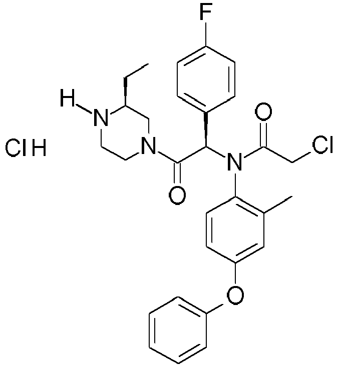 | 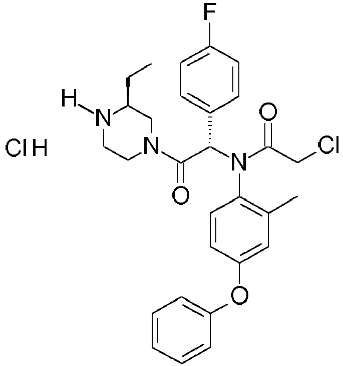 |
| I-17a | I-17b |
| 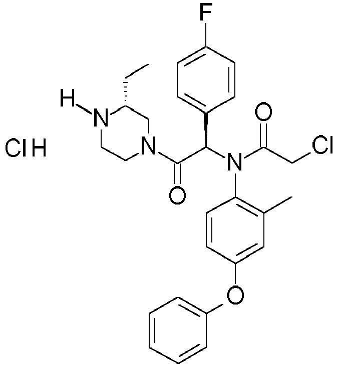 | 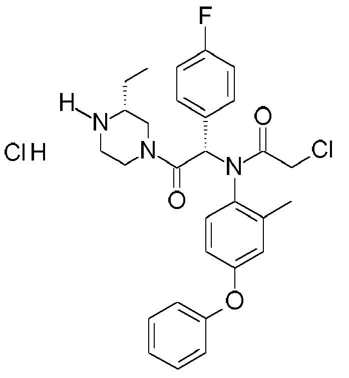 |
| I-18a | I-18b |
| 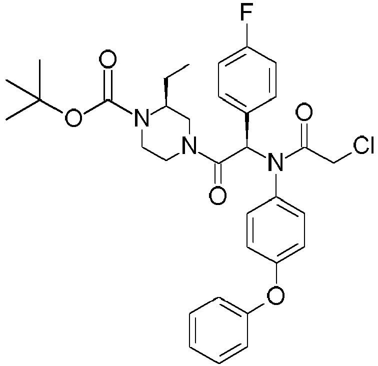 | 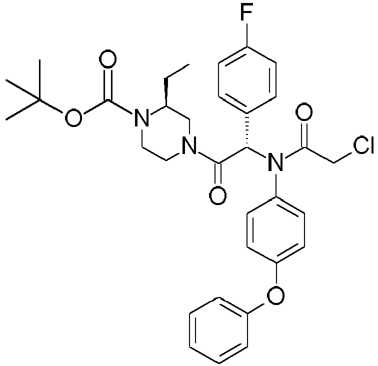 |
| I-19a | I-19b |
| 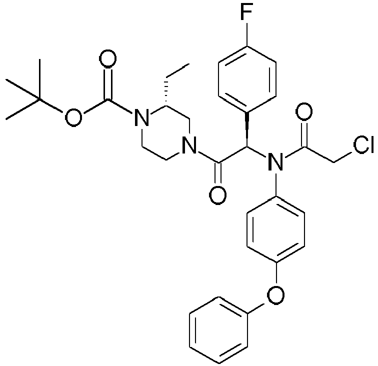 | 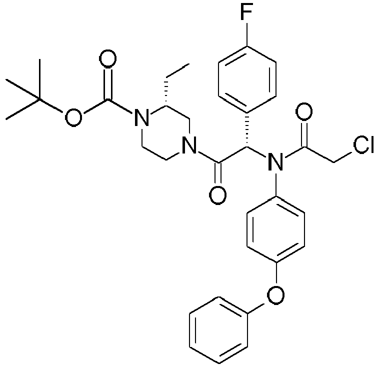 |
| I-20a | I-20b |
| 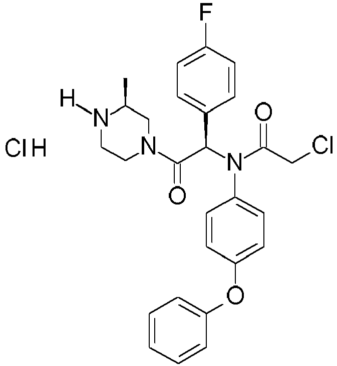 | 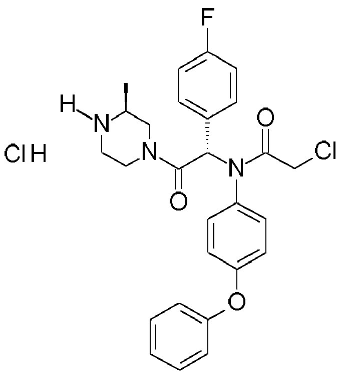 |
| I-21a | I-21b |
| 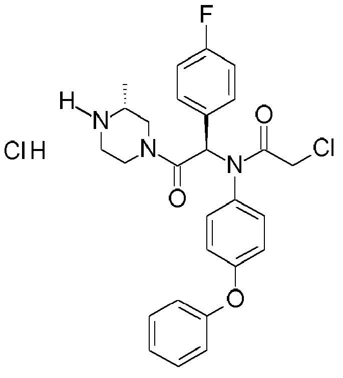 | 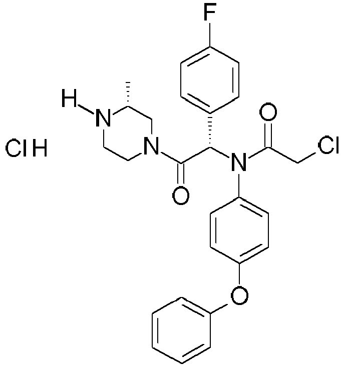 |
| I-22a | I-22b |
| 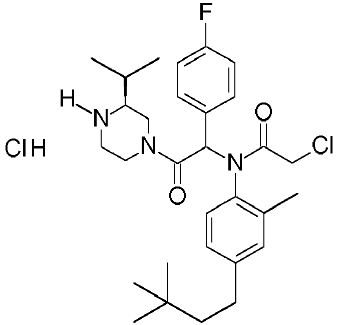 | 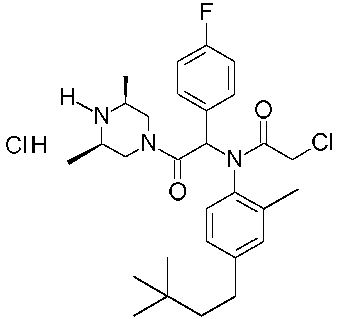 |
| I-23 | I-24 |
| 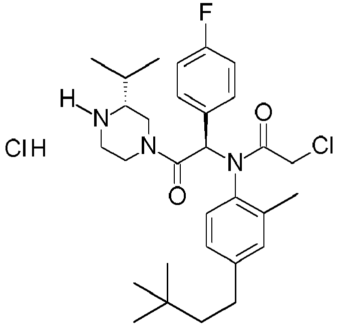 | 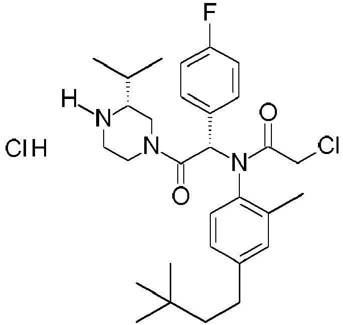 |
| I-25a | I-25b |
| 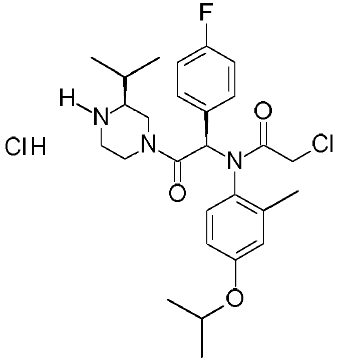 | 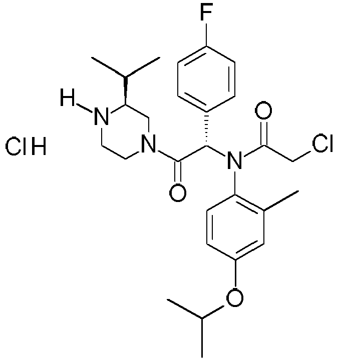 |
| I-26a | I-26b |
| 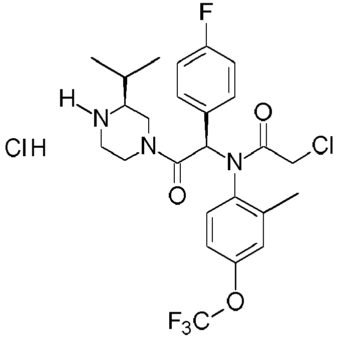 | 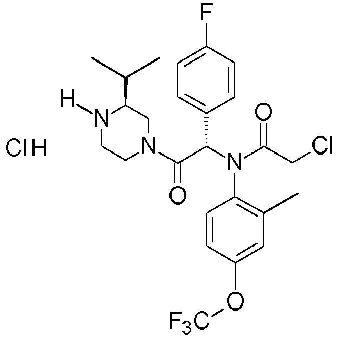 |
| I-27a | I-27b |
| 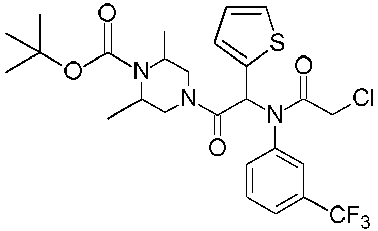 | 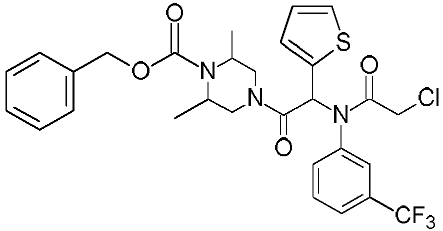 |
| I-28 | I-29 |
| 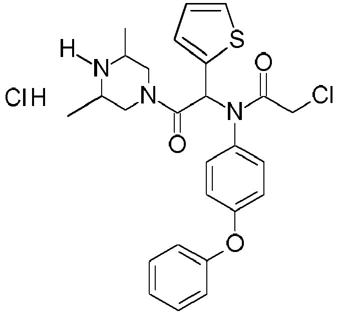 | 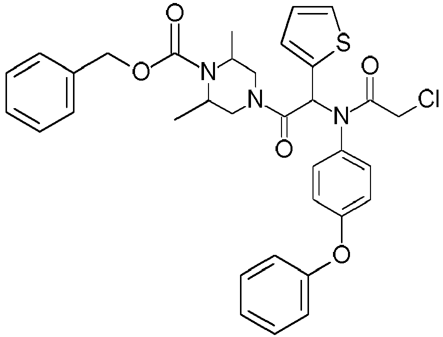 |
| I-30 | I-31 |
| 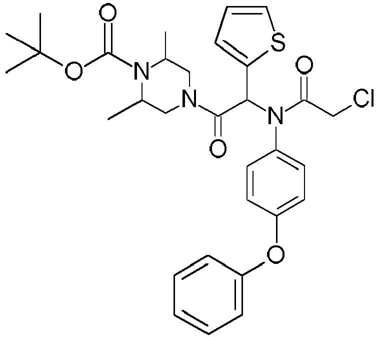 | 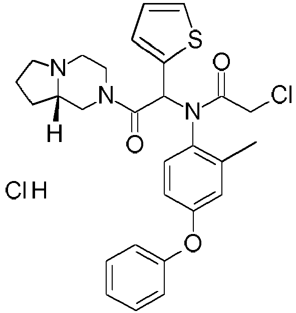 |
| I-32 | I-33 |
| 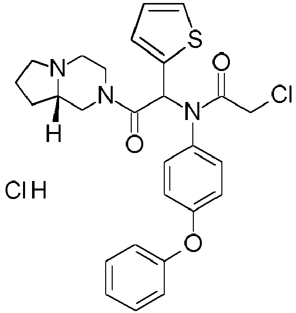 | 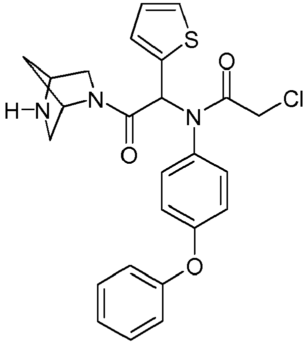 |
| I-34 | I-35 |
| 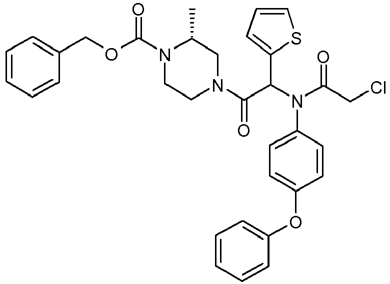 | 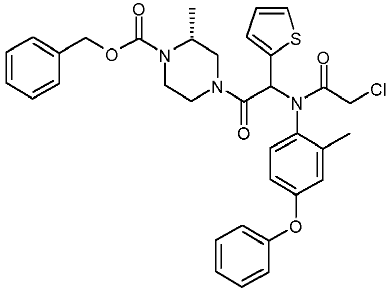 |
| I-36 | I-37 |
| 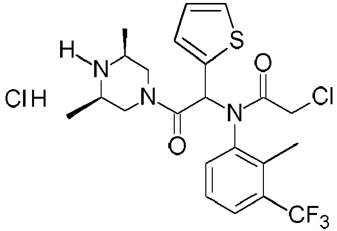 | 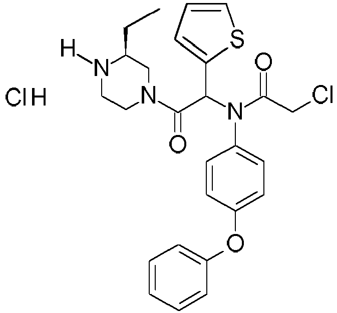 |
| I-3 | I-39 |
| 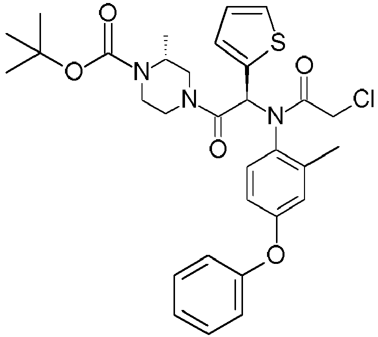 | 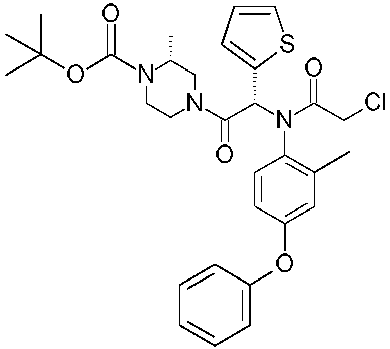 |
| I-40a | I-40b |
| 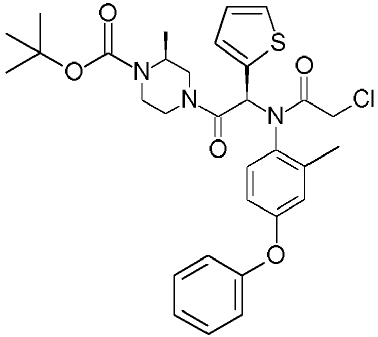 | 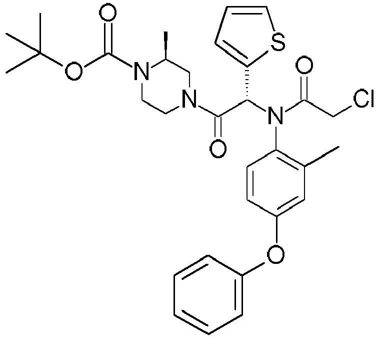 |
| I-41a | I-41b |
| 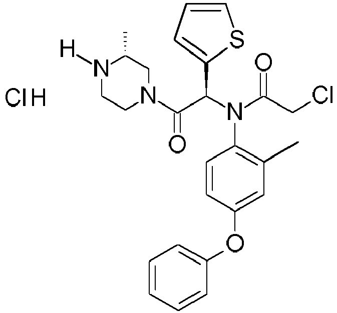 | 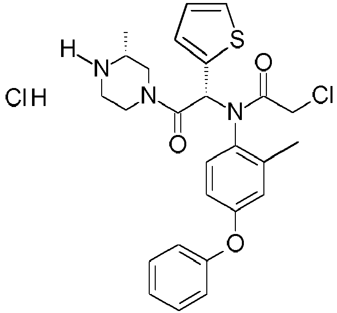 |
| I-42a | I-42b |
| 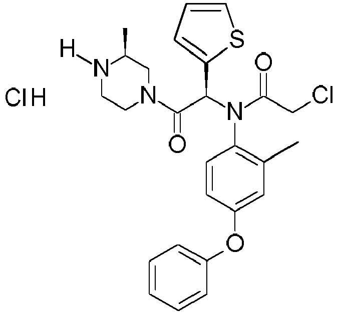 | 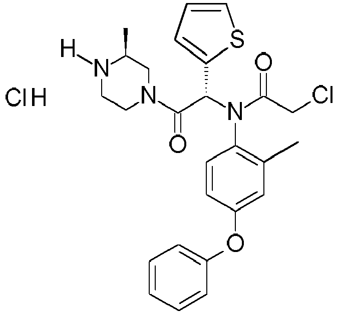 |
| I-43a | I-43b |
| 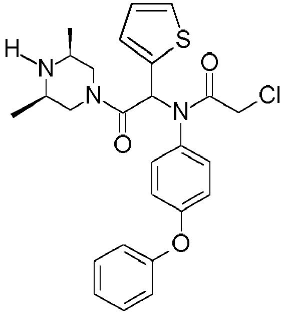 | 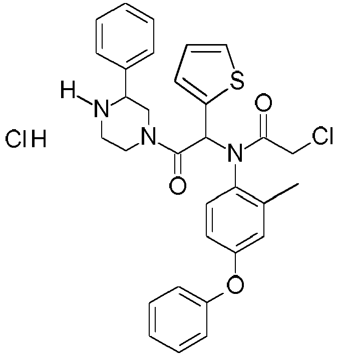 |
| I-44 | I-45 |
| 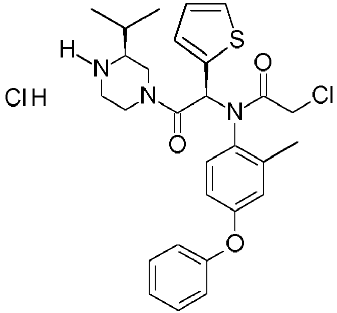 | 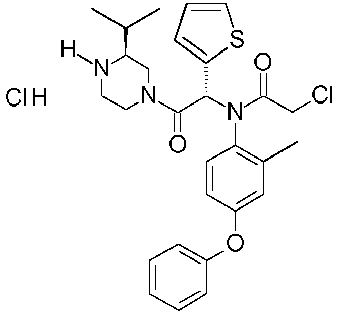 |
| I-46a | I-46b |
| 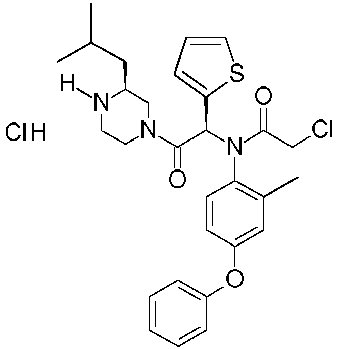 | 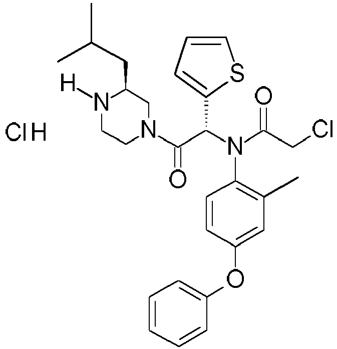 |
| I-47a | I-47b |
| 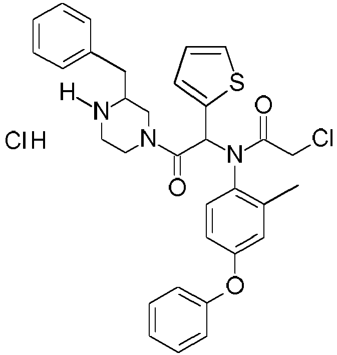 | 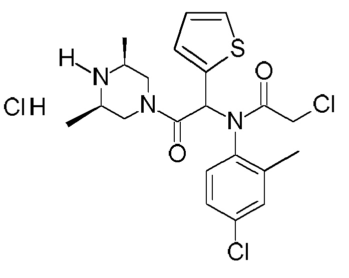 |
| 1-48 | I-49 |
| 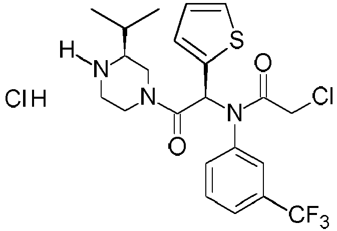 | 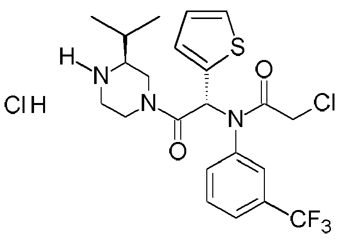 |
| I-50a | I-50b |
| 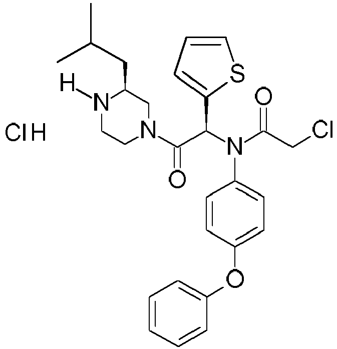 | 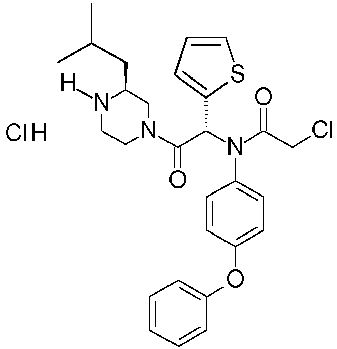 |
| I-51a | I-51b |
| 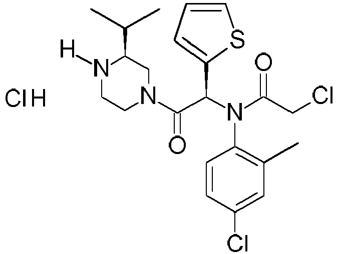 | 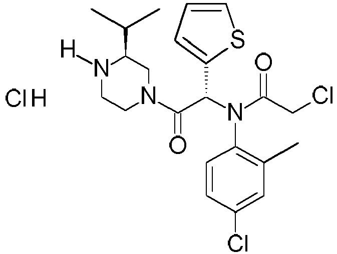 |
| I-52a | I-52b |
| 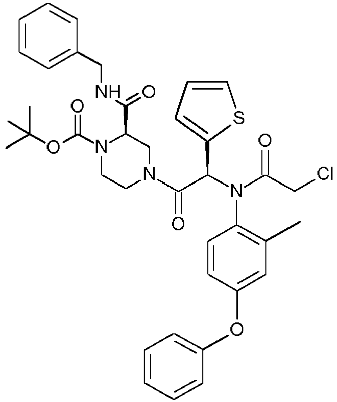 | 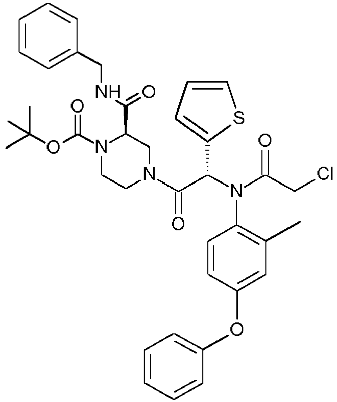 |
| I-53a | I-53b |
| 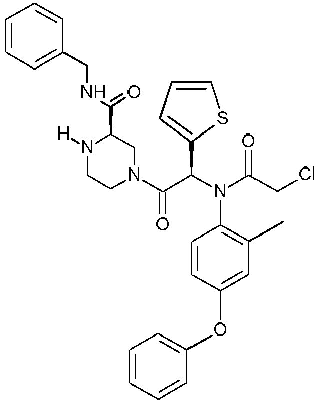 | 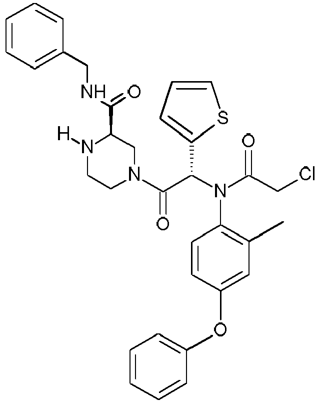 |
| I-54a | I-54b |
| 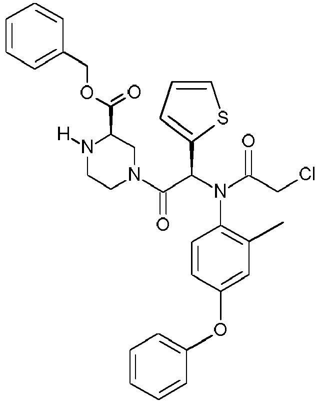 | 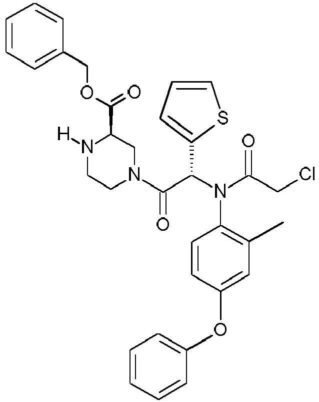 |
| I-55a | I-55b |
| 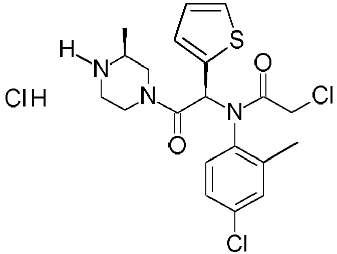 | 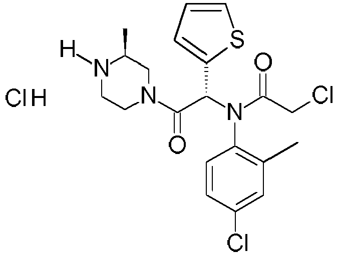 |
| I-56a | I-56b |
| 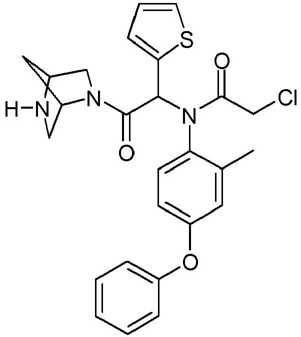 | 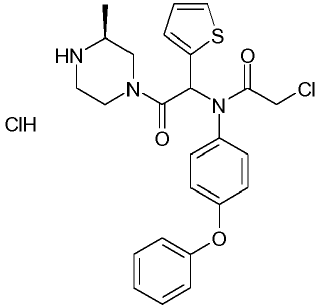 |
| I-57 | I-58 |
| 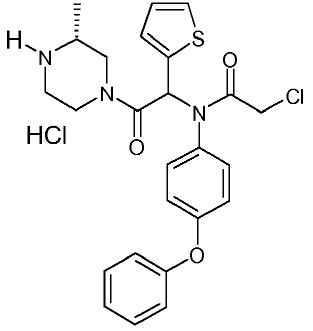 | 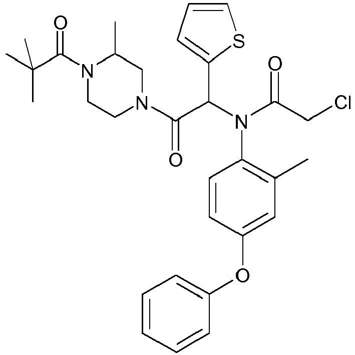 |
| I-59 | I-60 |
| 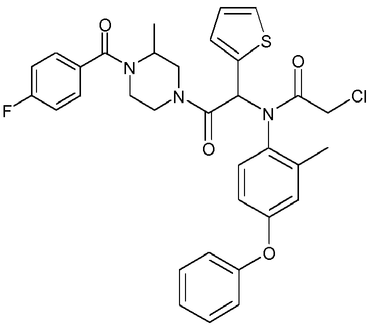 | 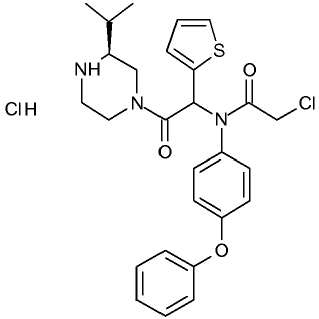 |
| I-61 | I-62 |
| 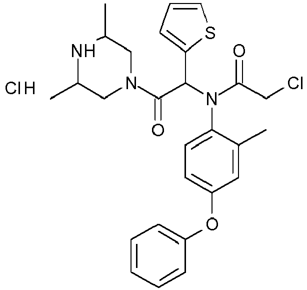 | |
| I-63 | |

8. Composé de formule générale (I) selon l'une quelconque des revendications 1 à 7, pour son utilisation à titre de médicament.

9. Composé de formule générale (I) selon l'une quelconque des revendications 1 à 7, pour son utilisation dans le traitement ou la prévention d'un cancer et en particulier d'un cancer résistant à une chimiothérapie.

10. Composition pharmaceutique comprenant au moins un composé de formule générale (I) selon l'une quelconque des revendications 1 à 7, en association avec un ou plusieurs excipients pharmaceutiquement acceptables.

11. Composition pharmaceutique selon la revendication 10, **caractérisée en ce qu'**elle comprend au moins un autre principe actif, tel qu'un agent anticancéreux.

12. Composition pharmaceutique selon la revendication 11, **caractérisée en ce que** le(s) principe(s) actif(s) est(sont) choisi(s) parmi le cisplatine et ses dérivés tels que le carboplatine et l'oxalyplatine; des taxanes tels que le taxol, le taxotère, le paclitaxel et le docétaxel ; des alcaloïdes de vinca tels que la vinblastine, la vincristine et la vinorelbine; des analogues de purine tels que la mercaptopurine, la thioguanine, la pentostatine et la 2-chlorodésoxyadénosine ; des inhibiteurs de topoisomerase I tels que des composés de la camptothécine comme l'irinotecan et le topotecan ; des inhibiteurs de topoisomerase II tels que l'épipodophyllotoxine, la podophyllotoxine et leurs dérivés comme l'étoposide et le téniposide ; des dérivés nucléosides antitumoraux tels que le 5-fluorouracile, la leucovorine, la gemcitabine ou la capécitabine ; des agents alkylants tels que des moutardes à l'azote comme la cyclophosphamide, la mechloréthamine, la chlorambucile et le melphalan, des nitroso-urées comme la carmustine, la lomustine et la streptozocine, des alkylsulfonates comme le busulfan, des éthylènimines et des méthylmélamines comme la thiotépa et l'hexaméthylmélamine, et des tétrazines comme la dacarbazine ; des dérivés d'antracyclines anti-tumoraux tels que la daunorubicine, l'adriamycine, le doxil, l'idarubicine et la mitoxantrone; des molécules ciblant le récepteur IGF-I telles que la picropodophylline ; des dérivés de tetracarcin tels que le tetrocarcin A ; des corticostéroïdes tels que la prednisone ; des anticorps tels que le trastuzumab (anticorps anti-HER2), le rituximab (anticorps anti-CD20), le gemtuzamab, le cetuximab, le pertuzumab et le bevacizumab; des antagonistes ou des modulateurs sélectifs des récepteurs des oestrogènes tels que le tamoxifen, le fulvestrant, le toremifène, le droloxifène, le faslodex et le raloxifène ; des inhibiteurs d'aromatase tels que l'exémestane, l'anastrozole, le letrozole et le vorozole; des agents de différenciation tels que les rétinoïdes comme l'acide rétinoïque et la vitamine D et des agents bloquant le métabolisme de l'acide rétinoïque tels que l'accutane ; des inhibiteurs d'ADN méthyle-transférase tels que l'azacytidine et le decitabine ; des antifolates tels que le permetrexed disodique; des antibiotiques tels que l'antinomycine D, la bléomycine, la mitomycine C, l'actinomycine D, la carminomycine, la daunomycine et la plicamycine ; des antimétabolites tels que la chlofarabine, l'aminoptérine, la cytosine arabinoside, la floxuridine et le méthotrexate; des agents induisant l'apoptose et des agents antiangiogéniques des inhibiteurs de Bcl-2 tels que YC 137, BH 312, ABT 737, le gossypol, HA 14-1, TW 37 et l'acide décanoïque ; des agents se liant à la tubuline tels que la combrestatine, des dérivés de colchicine et le nocodazole ; des inhibiteurs de kinase tels que le flavoperidol, le mésylate d'imatinib, l'erlotinib et le gefitinib; des inhibiteurs de farnésyl transférase tels que le tipifarnib; des inhibiteurs des histone-désacétylases tels que le butyrate de sodium, l'acide suberoylanilide hydroxamique, le depsipeptide, NVP- LAQ824, R306465, JNJ-26481585 et la trichostatine A; des inhibiteurs du système ubiquitine-protéasome tels que MLN .41, le bortezomib et l'yondelis ; et des inhibiteurs de télomérase tels que la telomestatine.

13. Composition pharmaceutique comprenant :
(i) au moins un composé de formule (I) selon l'une quelconque des revendications 1 à 7, et
(ii) au moins un autre principe actif, tel qu'un agent anticancéreux,
en tant que produits de combinaison pour une utilisation simultanée, séparée ou étalée dans le temps.

14. Composition pharmaceutique selon la revendication 13, **caractérisée en ce que** le(s) principe(s) actif(s) est(sont) choisi(s) parmi le cisplatine et ses dérivés tels que le carboplatine et l'oxalyplatine; des taxanes tels que le taxol, le taxotère, le paclitaxel et le docétaxel ; des alcaloïdes de vinca tels que la vinblastine, la vincristine et la vinorelbine; des analogues de purine tels que la mercaptopurine, la thioguanine, la pentostatine et la 2-chlorodésoxyadénosine ; des inhibiteurs de topoisomerase I tels que des composés de la camptothécine comme l'irinotecan et le topotecan ; des inhibiteurs de topoisomerase II tels que l'épipodophyllotoxine, la podophyllotoxine et leurs dérivés comme l'étoposide et le téniposide ; des dérivés nucléosides antitumoraux tels que le 5-fluorouracile, la leucovorine, la gemcitabine ou la capécitabine ; des agents alkylants tels que des moutardes à l'azote comme la cyclophosphamide, la mechloréthamine, la chlorambucile et le melphalan, des nitroso-urées comme la carmustine, la lomustine et la streptozocine, des alkylsulfonates comme le busulfan, des éthylènimines et des méthylmélamines comme la thiotépa et l'hexaméthylmélamine, et des tétrazines comme la dacarbazine ; des dérivés d'antracyclines anti-tumoraux tels que la daunorubicine, l'adriamycine, le doxil, l'idarubicine et la mitoxantrone; des molécules ciblant le récepteur IGF-I telles que la picropodophylline ; des dérivés de tetracarcin tels que le tetrocarcin A ; des corticostéroïdes tels que la prednisone ; des anticorps tels que le trastuzumab (anticorps anti-HER2), le rituximab (anticorps anti-CD20), le gemtuzamab, le cetuximab, le pertuzumab et le bevacizumab; des antagonistes ou des modulateurs sélectifs des récepteurs des oestrogènes tels que le tamoxifen, le fulvestrant, le toremifène, le droloxifène, le faslodex et le raloxifène ; des inhibiteurs d'aromatase tels que l'exémestane, l'anastrozole, le letrozole et le vorozole ;des agents de différenciation tels que les retinoïdes comme l'acide rétinoïque et la vitamine D et des agents bloquant le métabolisme de l'acide rétinoïque tels que l'accutane; des inhibiteurs d'ADN méthyle-transférase tels que l'azacytidine et le decitabine ; des antifolates tels que le permetrexed disodique ; des antibiotiques tels que l'antinomycine D, la bléomycine, la mitomycine C, l'actinomycine D, la carminomycine, la daunomycine et la plicamycine ; des antimétabolites tels que la chlofarabine, l'aminoptérine, la cytosine arabinoside, la floxuridine et le méthotrexate; des agents induisant l'apoptose et des agents antiangiogéniques des inhibiteurs de Bcl-2 tels que YC 137, BH 312, ABT 737, le gossypol, HA 14-1, TW 37 et l'acide décanoïque ; des agents se liant à la tubuline tels que la combrestatine, des dérivés de colchicine et le nocodazole ; des inhibiteurs de kinase tels que le flavoperidol, le mésylate d'imatinib, l'erlotinib et le gefitinib ; des inhibiteurs de farnésyl transférase tels que le tipifarnib; des inhibiteurs des histone-désacétylases tels que le butyrate de sodium, l'acide suberoylanilide hydroxamique, le depsipeptide, NVP- LAQ824, R306465, JNJ-26481585 et la trichostatine A; des inhibiteurs du système ubiquitine-protéasome tels que MLN .41, le bortezomib et l'yondelis ; et des inhibiteurs de télomérase tels que la telomestatine.

15. Composition pharmaceutique selon l'une quelconque des revendications 10 à 14, pour son utilisation à titre de médicament destiné au traitement ou à la prévention d'un cancer, et en particulier au traitement d'un cancer résistant à une chimiothérapie.

16. Procédé de préparation d'un composé de formule (I) selon l'une quelconque des revendications 1 à 7 comprenant les étapes successives suivantes:
a) réaction d'une amine de formule (II) suivante : 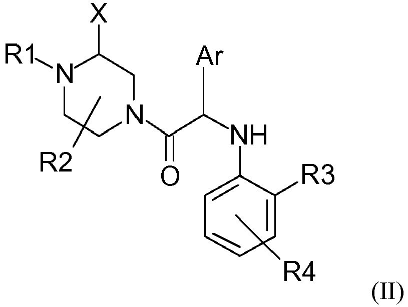
avec X, R1, R2, R3, R4 et Ar tels que définis à la revendication 1, R1 ne représentant pas un atome d'hydrogène,
avec du chlorure de chloroacétyle en présence d'une base pour donner un composé de formule (I) avec R1 ≠ H, et
b) éventuellement déprotection de l'atome d'azote portant le groupement R1 ≠ H pour donner un composé de formule (I) avec R1 = H.

17. Procédé selon la revendication 16 comprenant les étapes successives suivantes :
i) réaction d'un cétoester de formule (V) suivante : 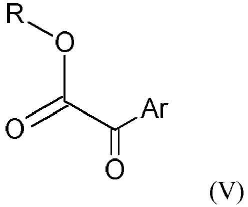
avec Ar tel que défini à la revendication 1 et R représentant un groupe (C₁-C₆)alkyle, tel qu'éthyle,
avec une aniline de formule (VI) suivante : 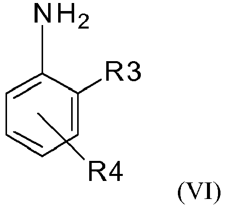 avec R3 et R4 tels que définis à la revendication 1,
pour donner une imine de formule (VII) suivante : 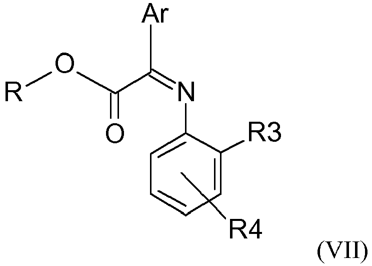 avec R tel que défini précédemment et R3, R4 et Ar tels que définis à la revendication 1,
ii) réduction de l'imine de formule (VII) obtenue à l'étape précédente pour donner une amine de formule (VIII) suivante : 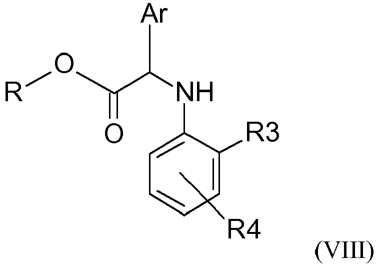 avec R tel que défini précédemment et R3, R4 et Ar tels que définis à la revendication 1,
iii) saponification de la fonction ester du composé de formule (VIII) obtenu à l'étape précédente pour donner un acide de formule (IV) suivante : 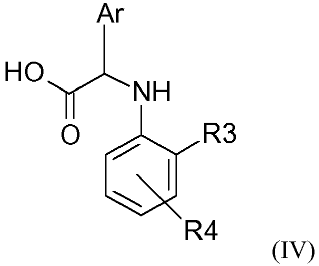 avec R3, R4 et Ar tels que définis à la revendication 1,
iv) réaction de l'acide de formule (IV) obtenu à l'étape précédente avec une pipérazine de formule (III) suivante : 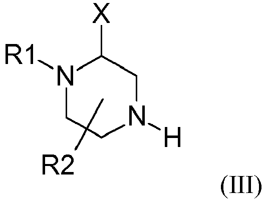 avec X, R1 et R2 tels que définis à la revendication 1, R1 ne représentant pas un atome d'hydrogène,
pour donner une amine de formule (II) selon la revendication 16,
v) réaction de l'amine de formule (II) obtenue à l'étape précédente avec du chlorure de chloroacétyle en présence d'une base pour donner un composé de formule (I) avec R1 ≠ H, et
vi) éventuellement déprotection de l'atome d'azote portant le groupement R1 ≠ H pour donner un composé de formule (I) avec R1 = H.

## Patentansprüche

1. Verbindung mit der folgenden allgemeinen Formel (I): 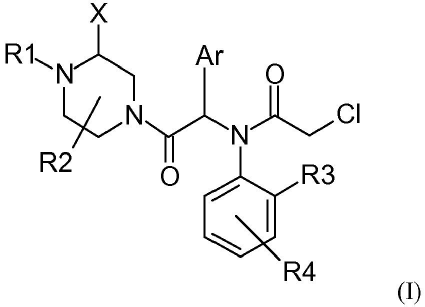 sowie ihre pharmazeutisch verträglichen Salze, ihre Stereoisomere oder Gemische von Stereoisomeren in sämtlichen Verhältnissen, insbesondere ein Gemisch aus Enantiomeren und insbesondere ein racemisches Gemisch,
für das:
- X eine (C₁-C₆)-Alkyl-, Phenyl-, Benzyl-, C(O)OR5- oder C(O)NHR5-Gruppe darstellt,
- R1 ein Wasserstoffatom oder eine C(O)H-, C(O)R6-oder C(O)OR6-Gruppe darstellt,
- R2 ein Wasserstoffatom oder eine (C₁-C₆) - Alkylgruppe darstellt,
oder R2 mit R1 oder X eine gesättigte Kohlenwasserstoffkette bildet, derart, dass ein fünf- oder sechsgliedriger, insbesondere ein fünfgliedriger Ring, gebildet wird,
- R3 ein Wasserstoff- oder Halogenatom oder eine (C₁-C₆)-Alkyl- oder (C₁-C₆)-Alkoxy-Gruppe darstellt,
- R4 ein Wasserstoff- oder Halogenatom, CN, NO₂, oder eine (C₁-C₆)-Alkyl-, (C₁-C₆)-Alkoxy-, Aryloxy-, Benzyloxy- oder Heteroaryloxy-Gruppe darstellt, wobei die Gruppe gegebenenfalls durch ein oder mehrere Halogenatome ersetzt ist,
- Ar eine Thiophenylgruppe oder eine Phenyl-Gruppe ist, die gegebenenfalls durch ein oder mehrere Halogenatome ersetzt ist, und
- R5 und R6 unabhängig voneinander eine (C₁-C₆)-Alkyl-, Aryl-(C₁-C₆)-Alkyl- oder Aryl-Gruppe darstellen, wobei die Gruppe gegebenenfalls durch ein oder mehrere Halogenatome ersetzt ist.

2. Verbindung nach Anspruch 1, **dadurch gekennzeichnet, dass** sie die folgende Formel (I-bis) erfüllt:

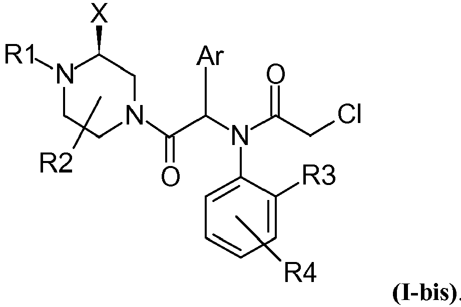

3. Verbindung nach einem der Ansprüche 1 und 2, **dadurch gekennzeichnet, dass** Ar eine Thiophenylgruppe oder eine durch ein oder mehrere Fluoratome ersetzte Phenylgruppe darstellt, wie beispielsweise 4-Fluorphenyl.

4. Verbindung nach einem der Ansprüche 1 bis 3, **dadurch gekennzeichnet, dass** R4 ein Wasserstoff- oder Halogenatom oder eine (C₁-C₆)-Alkyl-, (C₁-C₆)-Alkoxy- oder Aryloxy-Gruppe ist, wobei die Gruppe gegebenenfalls durch ein oder mehrere Halogenatome, insbesondere aus Fluor, ersetzt ist.

5. Verbindung nach einem der Ansprüche 1 bis 4, **dadurch gekennzeichnet, dass** R3 ein Wasserstoffatom oder eine (C₁-C₆)-Alkyl-Gruppe, wie beispielsweise Methyl, darstellt.

6. Verbindung nach einem der Ansprüche 1 bis 5, **dadurch gekennzeichnet, dass** X eine (C₁-C₆)-Alkyl-, Phenyl- oder Benzyl-Gruppe darstellt; R1 und R2 ein Wasserstoffatom darstellen; R3 ein Wasserstoffatom oder eine (C₁-C₆)-Alkyl-Gruppe, insbesondere (C₁-C₃)-Alkyl, darstellt; R4 ein Halogenatom oder eine (C₁-C₆)-Alkyl-, (C₁-C₆)-Alkoxy-, Aryloxy- oder Benzyloxy-Gruppe darstellt, wobei die Gruppe gegebenenfalls durch ein oder mehrere Halogenatome ersetzt ist; Ar eine Thiophenyl-Gruppe oder eine gegebenenfalls durch ein Fluoratomersetzte Phenylgruppe, wie beispielsweise 4-Fluorphenyl, darstellt; und R5 und R6 unabhängig voneinander eine (C₁-C₆)-Alkyl-, Aryl- (C₁-C₆) -Alkyl- oder Aryl-Gruppe darstellen, wobei die Gruppe gegebenenfalls durch ein oder mehrere Fluoratome ersetzt ist.

7. Verbindung nach Anspruch 1, **dadurch gekennzeichnet, dass** die Verbindung unter Folgendem ausgewählt ist:
| | |
|---|---|
| 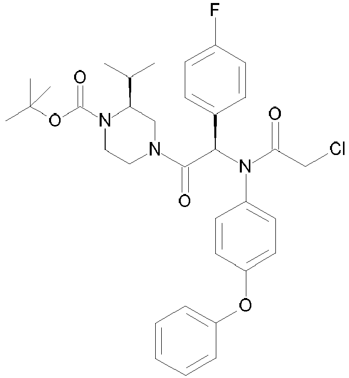 | 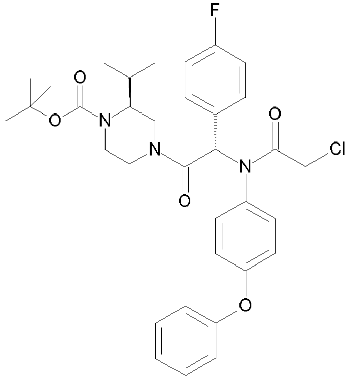 |
| I-1a | I-1b |
| 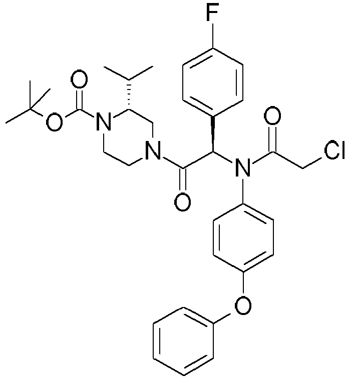 | 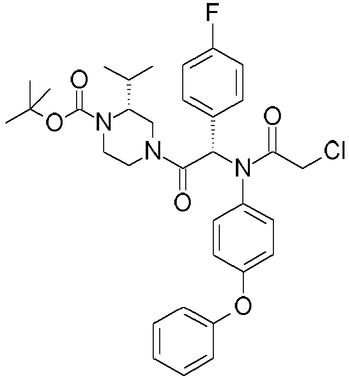 |
| I-2a | I-2b |
| 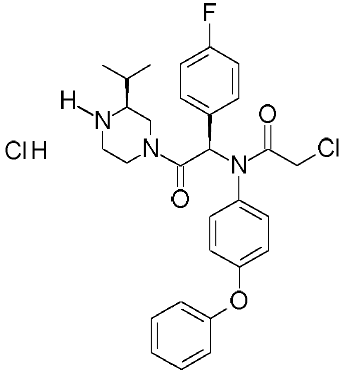 | 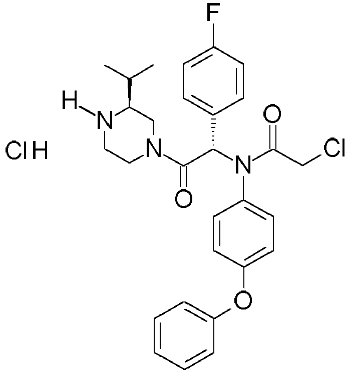 |
| I-3a | I-3b |
| 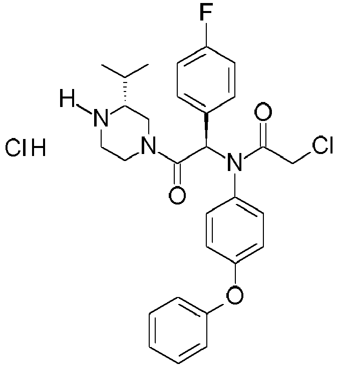 | 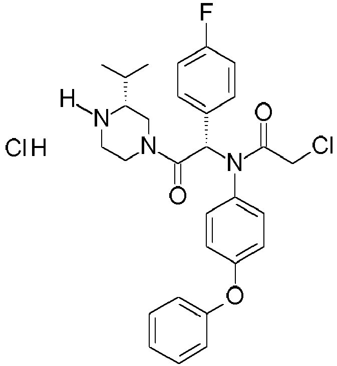 |
| I-4a | I-4b |
| 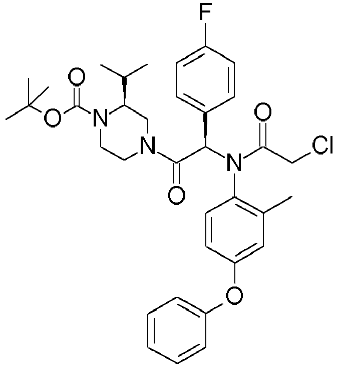 | 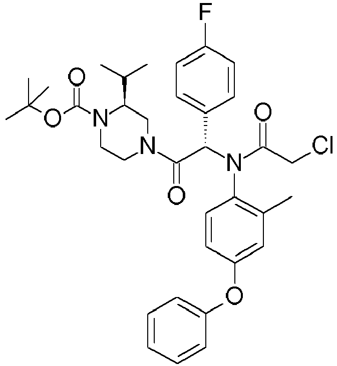 |
| I-5a | I-5b |
| 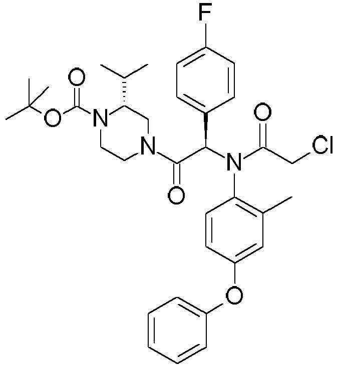 | 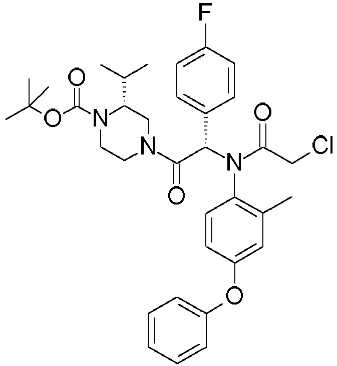 |
| I-6a | I-6b |
| 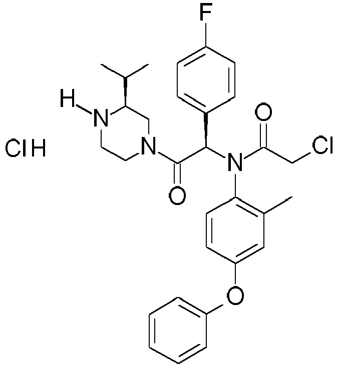 | 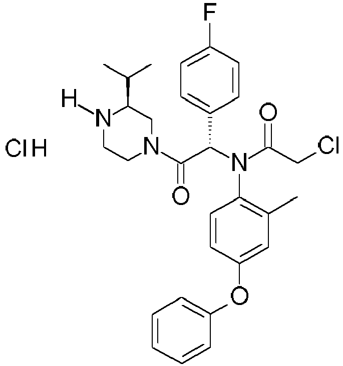 |
| I-7a | I-7b |
| 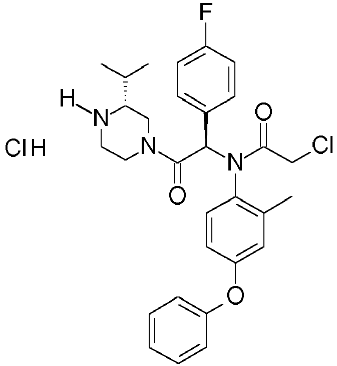 | 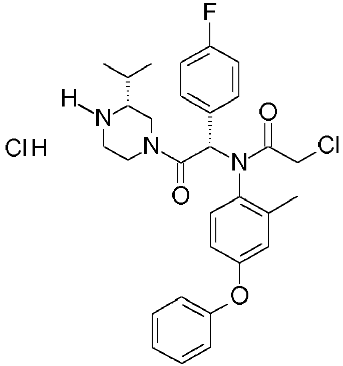 |
| I-8a | I-8b |
| 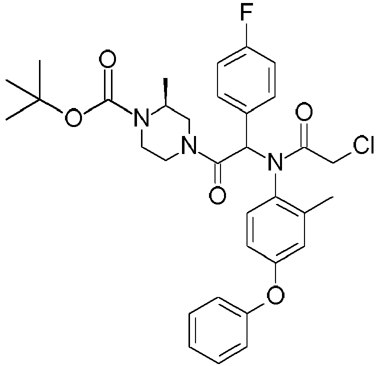 | 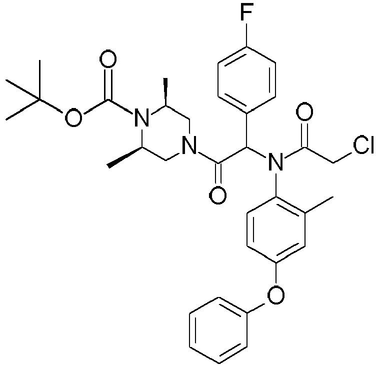 |
| I-9 | I-10 |
| 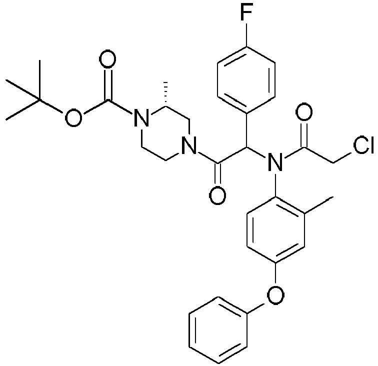 | 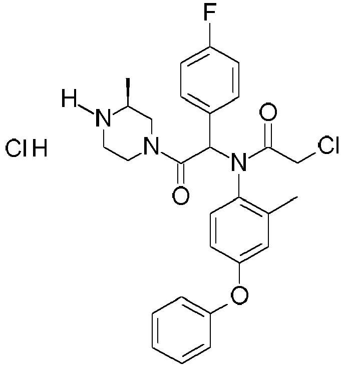 |
| I-11 | I-12 |
| 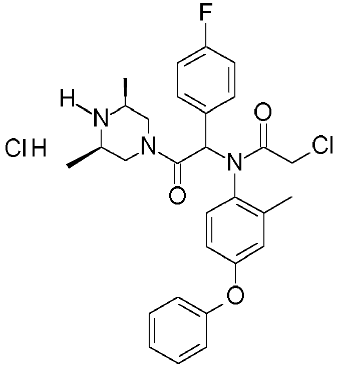 | 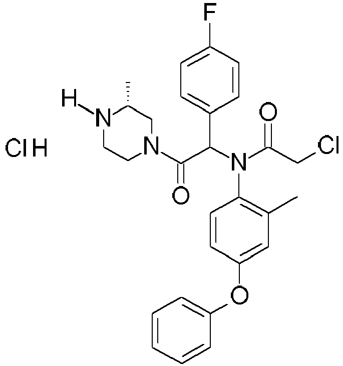 |
| I-13 | I-14 |
| 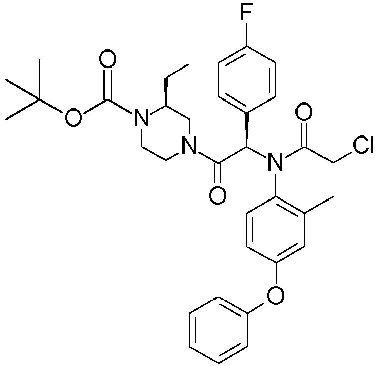 | 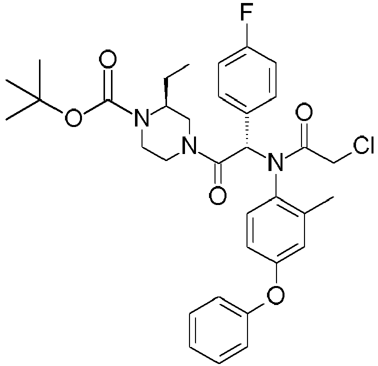 |
| I-15a | I-15b |
| 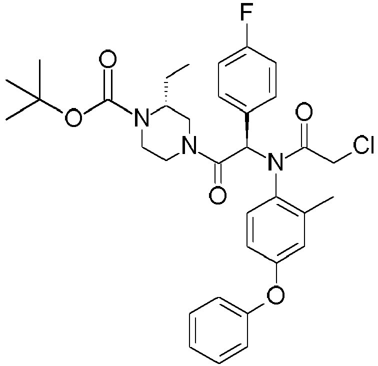 | 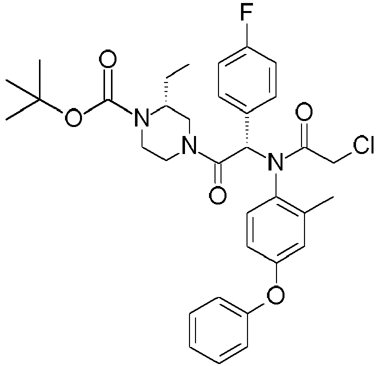 |
| I-16a | I-16b |
| 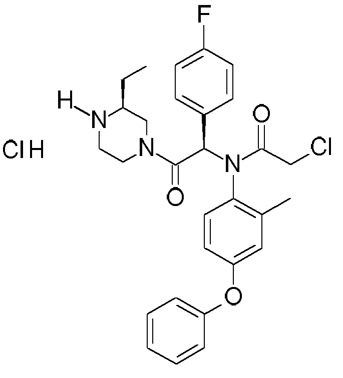 | 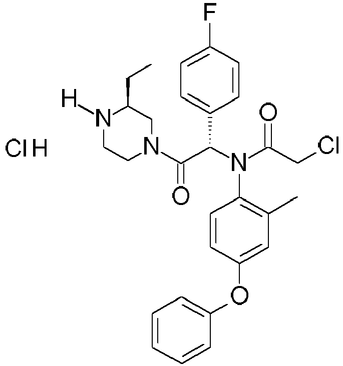 |
| I-17a | I-17b |
| 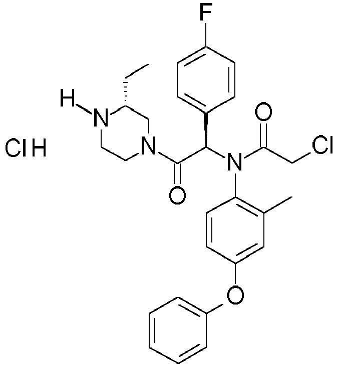 | 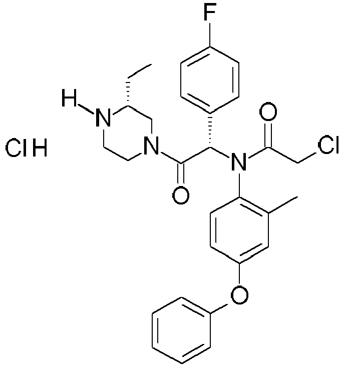 |
| I-18a | I-18b |
| 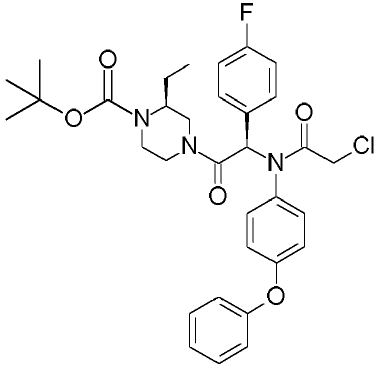 | 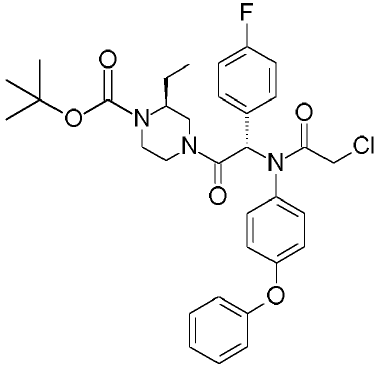 |
| I-19a | I-19b |
| 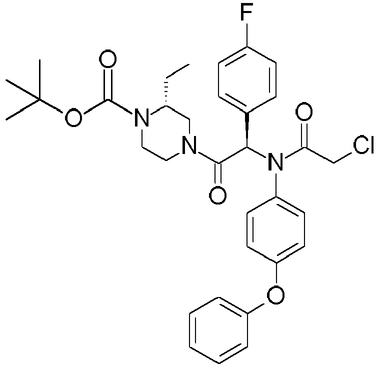 | 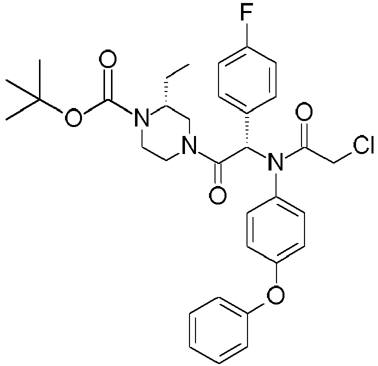 |
| I-20a | I-20b |
| 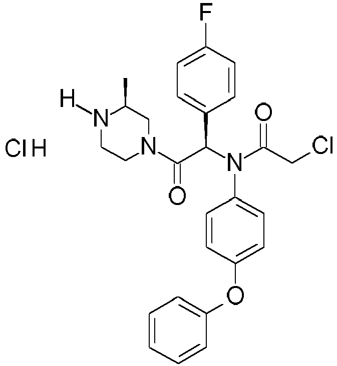 | 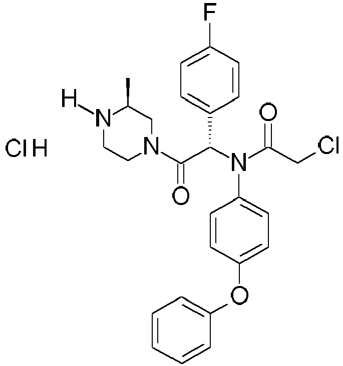 |
| I-21a | I-21b |
| 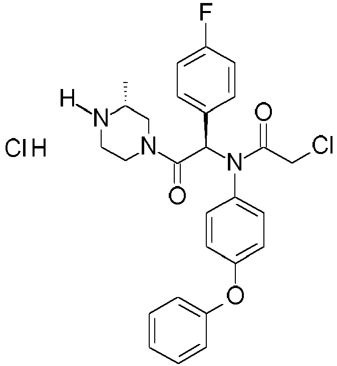 | 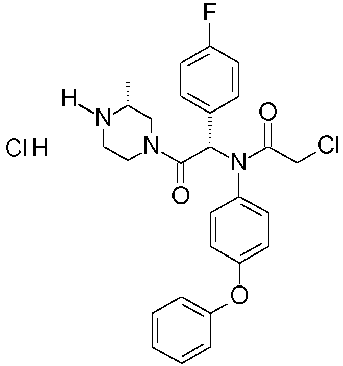 |
| I-22a | I-22b |
| 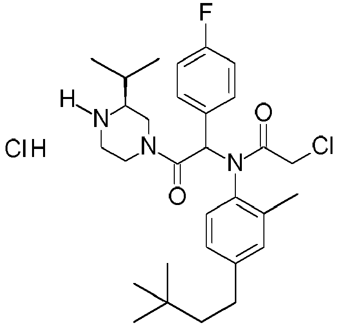 | 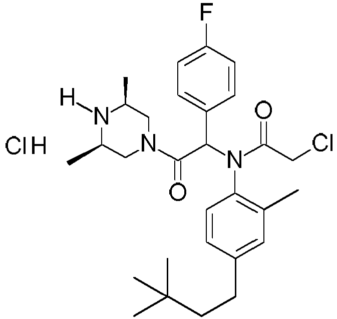 |
| I-23 | I-24 |
| 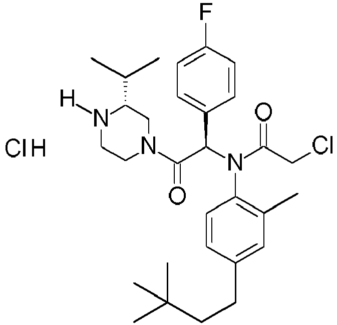 | 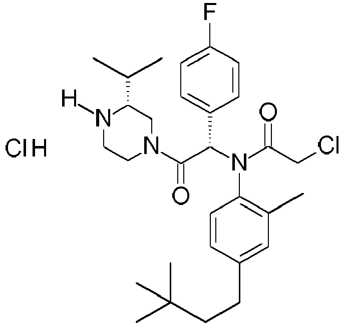 |
| I-25a | I-25b |
| 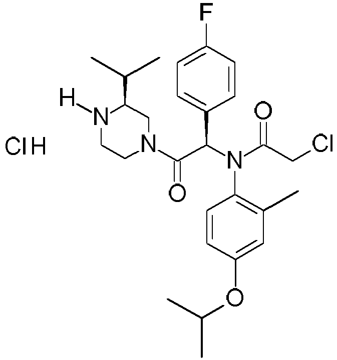 | 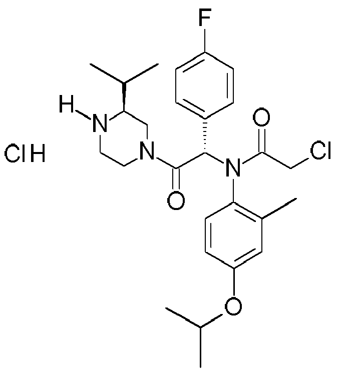 |
| I-26a | I-26b |
| 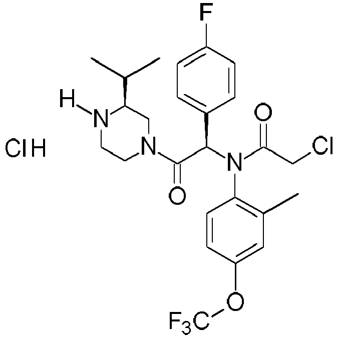 | 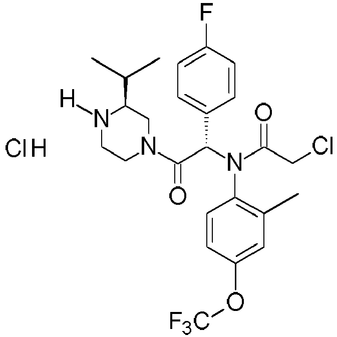 |
| I-27a | I-27b |
| 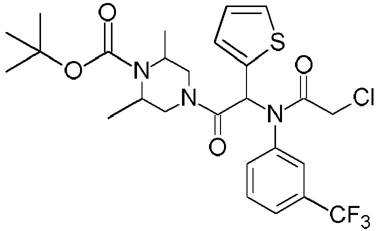 | 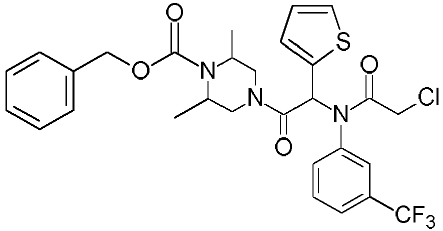 |
| I-28 | I-29 |
| 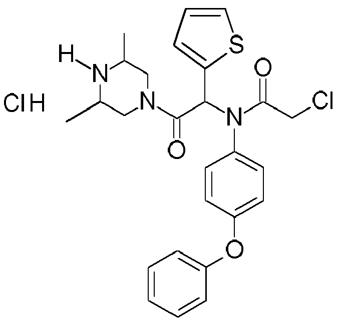 | 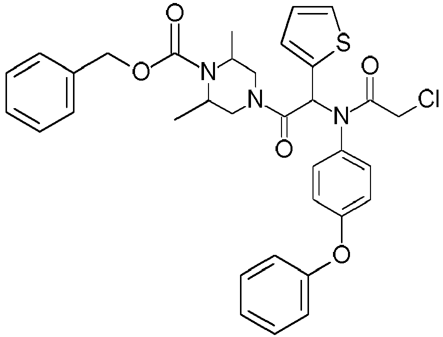 |
| I-30 | I-31 |
| 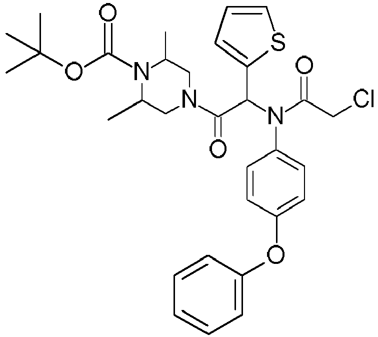 | 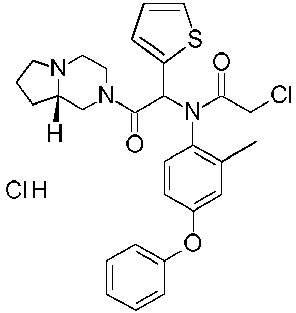 |
| I-32 | I-33 |
| 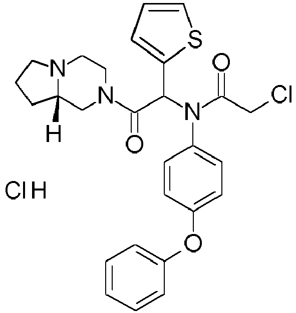 | 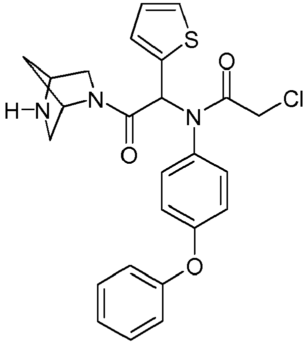 |
| I-34 | I-35 |
| 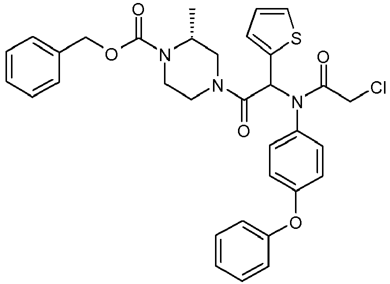 | 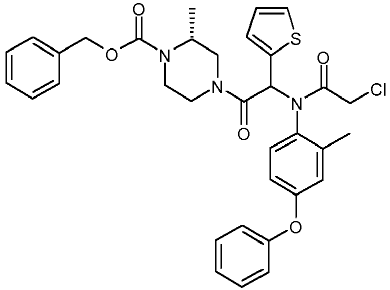 |
| I-36 | I-37 |
| 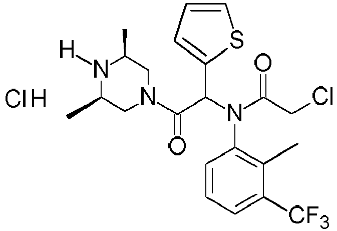 | 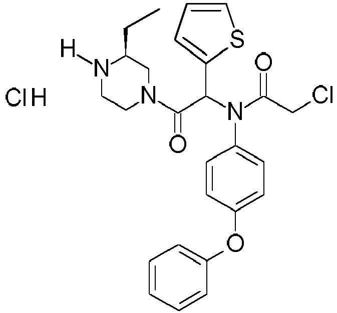 |
| I-38 | I-39 |
| 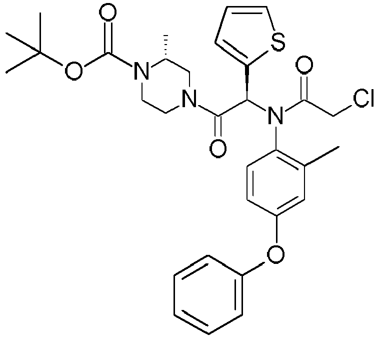 | 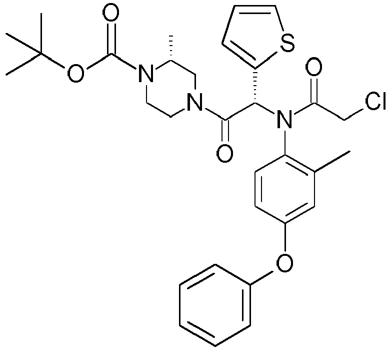 |
| I-40a | I-40b |
| 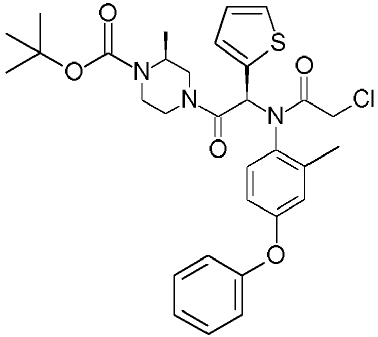 | 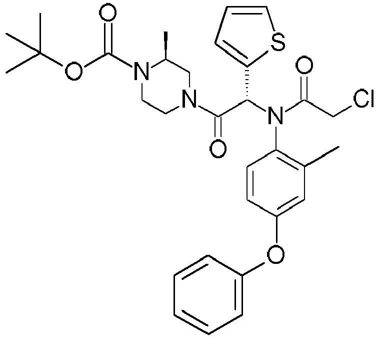 |
| I-41a | I-41b |
| 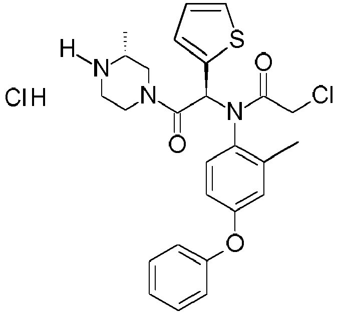 | 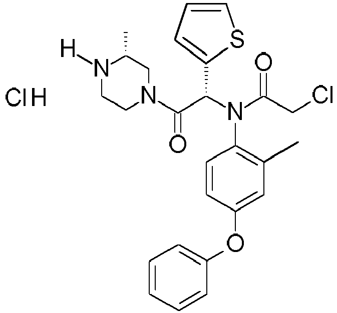 |
| I-42a | I-42b |
| 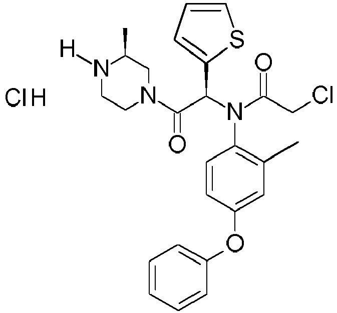 | 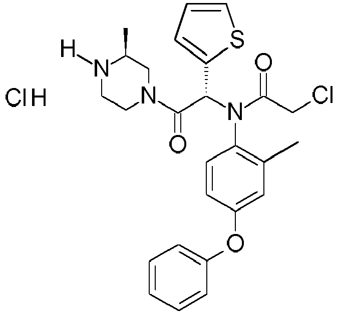 |
| I-43a | I-43b |
| 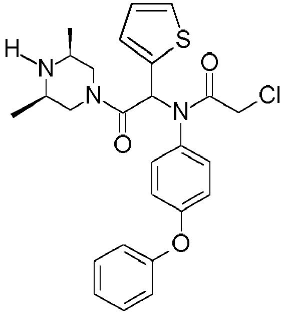 | 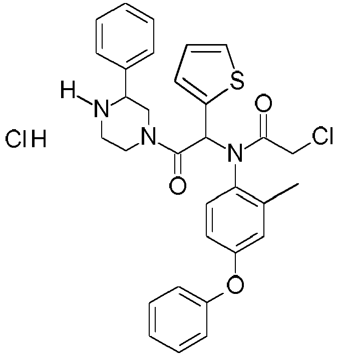 |
| I-44 | I-45 |
| 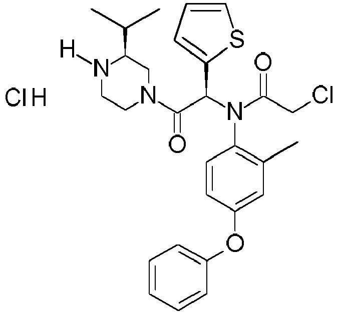 | 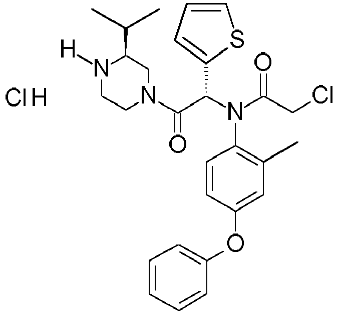 |
| I-46a | I-46b |
| 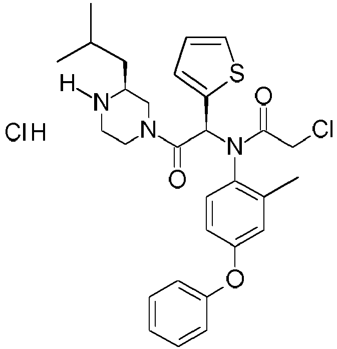 | 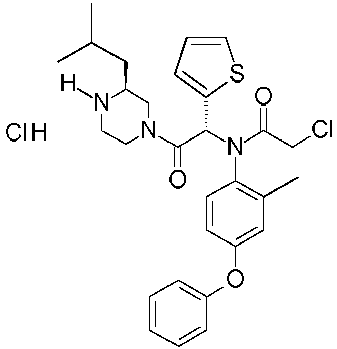 |
| I-47a | I-47b |
| 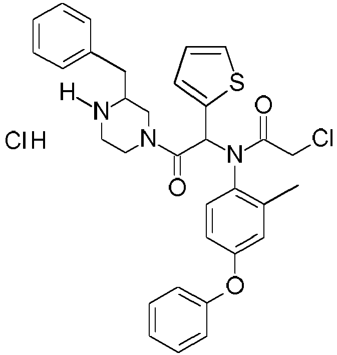 | 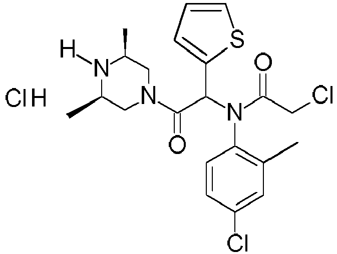 |
| I-48 | I-49 |
| 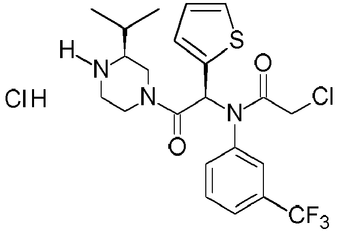 | 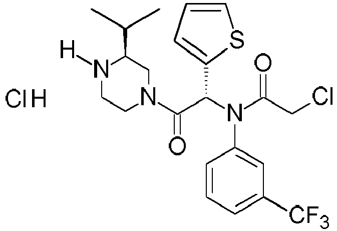 |
| I-50a | I-50b |
| 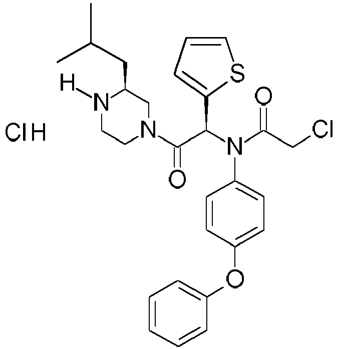 | 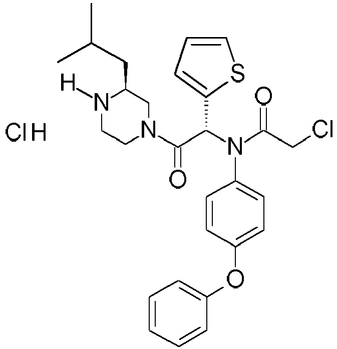 |
| I-51a | I-51b |
| 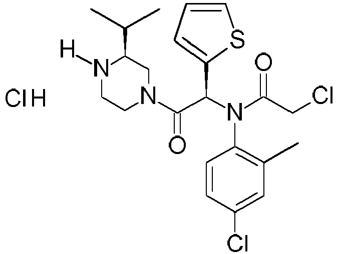 | 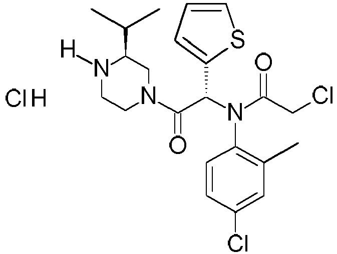 |
| I-52a | I-52b |
| 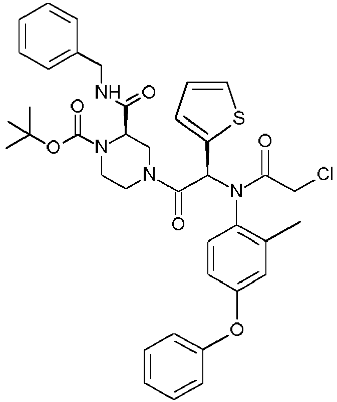 | 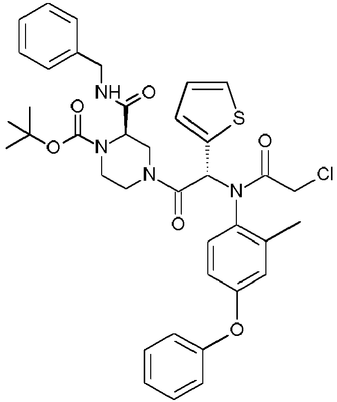 |
| I-53a | I-53b |
| 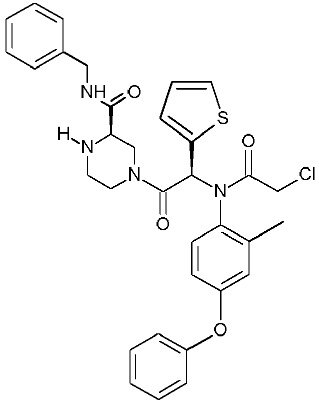 | 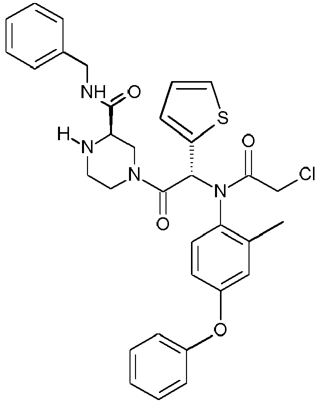 |
| I-54a | I-54b |
| 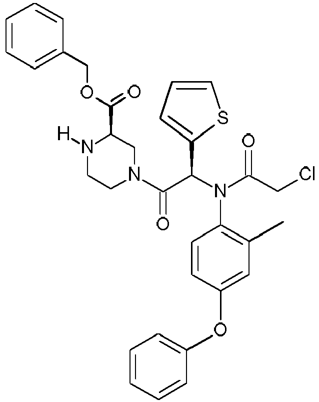 | 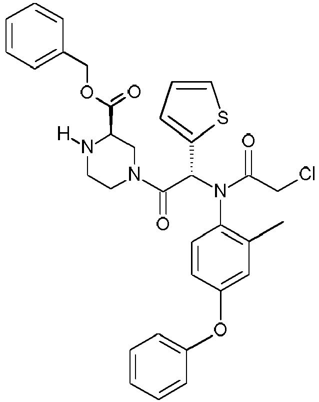 |
| I-55a | I-55b |
| 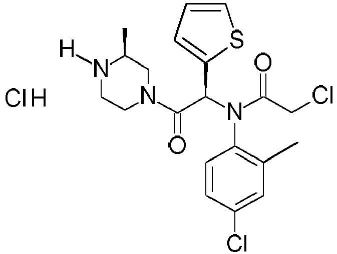 | 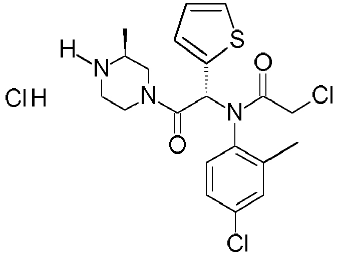 |
| I-56a | I-56b |
| 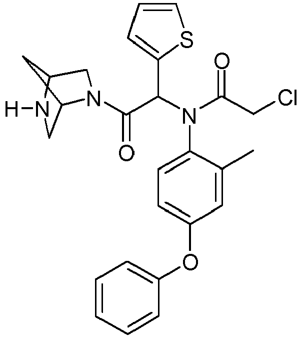 | 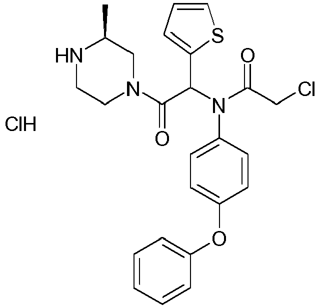 |
| I-57 | I-58 |
| 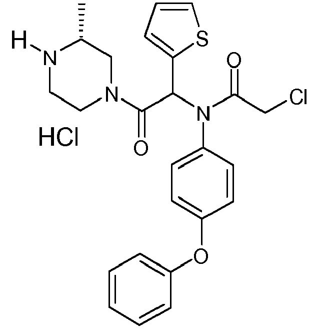 | 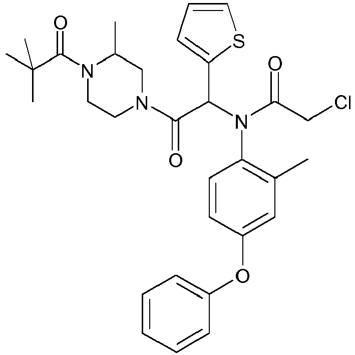 |
| I-59 | I-60 |
| 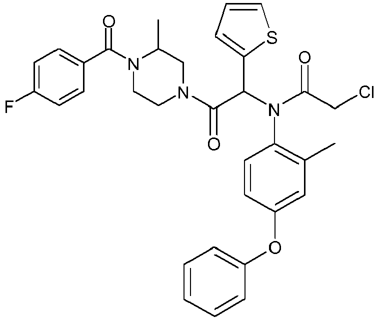 | 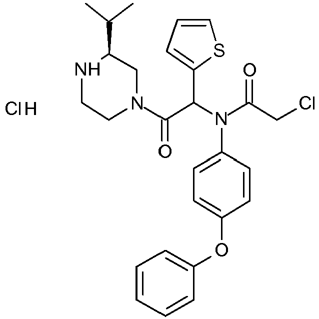 |
| I-61 | I-62 |
| 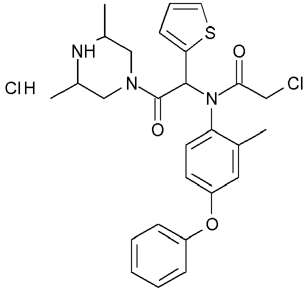 | |
| I-63 | |

8. Verbindung mit der allgemeinen Formel (I) nach einem der Ansprüche 1 bis 7, für ihre Verwendung als Medikament.

9. Verbindung mit der allgemeinen Formel (I) nach einem der Ansprüche 1 bis 7 für ihre Verwendung bei der Behandlung oder der Prävention eines Krebses und insbesondere eines chemotherapieresistenten Krebses.

10. Pharmazeutische Zusammensetzung, die mindestens eine Verbindung mit der allgemeinen Formel (I) nach einem der Ansprüche 1 bis 7 in Verbindung mit einem oder mehreren pharmazeutisch verträglichen Trägern umfasst.

11. Pharmazeutische Zusammensetzung nach Anspruch 10, **dadurch gekennzeichnet, dass** sie mindestens einen weiteren Wirkstoff, wie beispielsweise einen krebshemmenden Stoff, umfasst.

12. Pharmazeutische Zusammensetzung nach Anspruch 11, **dadurch gekennzeichnet, dass** der/die Wirkstoff/e unter Cisplatin und seinen Derivaten, wie beispielsweise Carboplatin und Oxalyplatin; Taxanen, wie beispielsweise Taxol, Taxoter, Paclitaxel und Docetaxel; Vincaalkaloiden, wie beispielsweise Vinblastin, Vincristin und Vinorelbin; Purinanaloga, wie beispielsweise Mercaptopurin, Thioguanin, Pentostatin und 2-Chlordesoxyadenosin; Topoisomerase-I-Inhibitoren, wie beispielsweise Camptothecinverbindungen wie Irinotecan und Topotecan; Topoisomerase-II-Inhibitoren, wie beispielsweise Epipodophyllotoxin, Podophyllotoxin und ihre Derivate, wie Etoposid und Teniposid; antitumoralen Nukleosidderivaten, wie beispielsweise 5-Fluoruracil, Leucovorin, Gemcitabin oder Capecitabin; alkylierenden Mitteln, wie beispielsweise Stickstoffloste, wie Cyclophosphamid, Mechlorethamin, Chlorambucil und Melphalan, Nitrosoharnstoffe, wie Carmustin, Lomustin und Streptozocin, Alkylsulfonaten, wie Busulfan, Ethylenimine und Methylmelamine, wie Thiotepa und Hexamethylmelamin, und Tetrazine, wie Dacarbazin; antitumoralen Antracyclin-Derivaten, wie beispielsweise Daunorubicin, Adriamycin, Doxil, Idarubicin und Mitoxantron; Molekülen, die auf den IGF-I-Rezeptor abzielen, wie beispielsweise Picropodophyllin; Tetracarcin-Derivaten, wie beispielsweise Tetrocarcin A; Kortikosteroiden, wie beispielsweise Prednison; Antikörpern, wie beispielsweise Trastuzumab (Anti-HER2-Antikörper), Rituximab (Anti-CD20-Antikörper), Gemtuzamab, Cetuximab, Pertuzumab und Bevacizumab; Antagonisten oder selektiven Modulatoren von Östrogenrezeptoren, wie beispielsweise Tamoxifen, Fulvestrant, Toremifen, Droloxifen, Faslodex und Raloxifen; Aromataseinhibitoren, wie beispielsweise Exemestan, Anastrozol, Letrozol und Vorozol; Differenzierungsmitteln, wie beispielsweise Retinoide, wie Retinolsäure und Vitamin D und Mittel zum Blockieren des Retinolsäuremetabolismus, wie beispielsweise Accutan; Methyltransferase-DNA-Inhibitoren, wie beispielsweise Azacytidin und Decitabin; Antifolaten, wie beispielsweise Pemetrexed-Dinatrium; Antibiotika, wie beispielsweise Antinomycin D, Bleomycin, Mitomycin C, Actinomycin D, Carminomycin, Daunomycin und Plicamycin; Antimetaboliten, wie beispielsweise Chlofarabin, Aminopterin, Cytosinarabinosid, Floxuridin und Methotrexat; Apoptose induzierenden Mitteln und antiangiogenetischen Mitteln der Bcl-2-Inhibitoren, wie beispielsweise YC 137, BH 312, ABT 737, Gossypol, HA 14-1, TW 37 und Decansäure; tubulin-bindenden Mitteln, wie beispielsweise Combrestatin, Colchicin- und Nocodazol-Derivate; Kinaseinhibitoren, wie beispielsweise Flavoperidol, Imatinib-Mesylat, Erlotinib und Gefitinib; Farnesyltransferease-Inhibitoren, wie beispielsweise Tipifarnib; Histonedesacetylaseinhibitoren, wie beispielsweise Natriumbutyrat, Suberoylanilid-Hydroxamsäure, Depsipeptid, NVP-LAQ824, B306465, JNJ-26481585 und Trichostatin A; Ubiquitin-Proteasom-System-Inhibitoren, wie beispielsweise MLN .41, Bortezomib und Yondelis; und Telomerase-Inhibitoren, wie beispielsweise Telomestatin, ausgewählt ist/sind.

13. Pharmazeutische Zusammensetzung, die Folgendes umfasst:
(i) mindestens eine Verbindung der Formel (I) nach einem der Ansprüche 1 bis 7, und
(ii) mindestens einen anderen Wirkstoff, wie beispielsweise einen krebshemmenden Stoff,
als Kombinationsprodukte für eine gleichzeitige, getrennte oder zeitliche verteilte Verwendung.

14. Pharmazeutische Zusammensetzung nach Anspruch 13, **dadurch gekennzeichnet, dass** der/die Wirkstoff/e unter Cisplatin und seinen Derivaten, wie beispielsweise Carboplatin und Oxalyplatin; Taxanen, wie beispielsweise Taxol, Taxoter, Paclitaxel und Docetaxel; Vincaalkaloiden, wie beispielsweise Vinblastin, Vincristin und Vinorelbin; Purinanaloga, wie beispielsweise Mercaptopurin, Thioguanin, Pentostatin und 2-Chlordesoxyadenosin; Topoisomerase-I-Inhibitoren, wie beispielsweise Camptothecinverbindungen wie Irinotecan und Topotecan; Topoisomerase-II-Inhibitoren, wie beispielsweise Epipodophyllotoxin, Podophyllotoxin und ihre Derivate, wie Etoposid und Teniposid; antitumoralen Nukleosidderivaten, wie beispielsweise 5-Fluoruracil, Leucovorin, Gemcitabin oder Capecitabin; alkylierenden Mitteln, wie beispielsweise Stickstoffloste, wie Cyclophosphamid, Mechlorethamin, Chlorambucil und Melphalan, Nitrosoharnstoffe, wie Carmustin, Lomustin und Streptozocin, Alkylsulfonaten, wie Busulfan, Ethylenimine und Methylmelamine, wie Thiotepa und Hexamethylmelamin, und Tetrazinen, wie Dacarbazin; antitumoralen Antracyclin-Derivaten, wie beispielsweise Daunorubicin, Adriamycin, Doxil, Idarubicin und Mitoxantron; Molekülen, die auf den IGF-I-Rezeptor abzielen, wie beispielsweise Picropodophyllin; Tetracarcin-Derivaten, wie beispielsweise Tetrocarcin A; Kortikosteroiden, wie beispielsweise Prednison; Antikörpern, wie beispielsweise Trastuzumab (Anti-HER2-Antikörper), Rituximab (Anti-CD20-Antikörper), Gemtuzamab, Cetuximab, Pertuzumab und Bevacizumab; Antagonisten oder selektiven Modulatoren von Östrogenrezeptoren, wie beispielsweise Tamoxifen, Fulvestrant, Toremifen, Droloxifen, Faslodex und Raloxifen; Aromataseinhibitoren, wie beispielsweise Exemestan, Anastrozol, Letrozol und Vorozol; Differenzierungsmitteln, wie beispielsweise Retinoide, wie Retinolsäure und Vitamin D und Mittel zum Blockieren des Retinolsäuremetabolismus, wie beispielsweise Accutan; Methyltransferase-DNA-Inhibitoren, wie beispielsweise Azacytidin und Decitabin; Antifolaten, wie beispielsweise Pemetrexed-Dinatrium; Antibiotika, wie beispielsweise Antinomycin D, Bleomycin, Mitomycin C, Actinomycin D, Carminomycin, Daunomycin und Plicamycin; Antimetaboliten, wie beispielsweise Chlofarabin, Aminopterin, Cytosinarabinosid, Floxuridin und Methotrexat; Apoptose induzierenden Mitteln und antiangiogenetischen Mitteln der Bcl-2-Inhibitoren, wie beispielsweise YC 137, BH 312, ABT 737, Gossypol, HA 14-1, TW 37 und Decansäure; tubulin-bindenden Mitteln, wie beispielsweise Combrestatin, Colchicin- und Nocodazol-Derivate; Kinaseinhibitoren, wie beispielsweise Flavoperidol, Imatinib-Mesylat, Erlotinib und Gefitinib; Farnesyltransferease-Inhibitoren, wie beispielsweise Tipifarnib; Histonedesacetylaseinhibitoren, wie beispielsweise Natriumbutyrat, Suberoylanilid-Hydroxamsäure, Depsipeptid, NVP-LAQ824, B306465, JNJ-26481585 und Trichostatin A; Ubiquitin-Proteasom-System-Inhibitoren, wie beispielsweise MLN .41, Bortezomib und Yondelis; und Telomerase-Inhibitoren, wie beispielsweise Telomestatin, ausgewählt ist/sind.

15. Pharmazeutische Zusammensetzung nach einem der Ansprüche 10 bis 14 für ihre Verwendung als Arzneimittel, das zur Behandlung oder zur Prävention eines Krebses und insbesondere zur Behandlung eines chemotherapieresistenten Krebses bestimmt ist.

16. Verfahren zur Herstellung einer Verbindung mit der Formel (I) nach einem der Ansprüche 1 bis 7, das die folgenden aufeinanderfolgenden Schritte umfasst:
a) Reaktion eines Amins mit der folgenden Formel (II) : 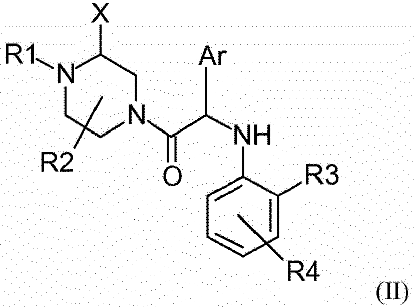
mit X, R1, R2, R3, R4 und Ar, wie in Anspruch 1 definiert, wobei R1 kein Wasserstoffatom darstellt,
mit Chloracetylchlorid bei Vorhandensein einer Base, um eine Verbindung der Formel (I) mit R1 ≠ H zu ergeben, und
b) gegebenenfalls Entschützung des Stickstoffatoms, das die Gruppe R1 ≠ H trägt, um eine Verbindung mit der Formel (I) mit R1 = H zu ergeben.

17. Verfahren nach Anspruch 16, das die folgenden aufeinanderfolgenden Schritte umfasst:
i) Reaktion eines Ketoesters mit der folgenden Formel(V): 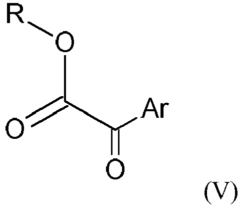
mit Ar, wie in Anspruch 1 definiert, wobei R eine (C₁-C₆)-Alkylgruppe, wie beispielsweise Ethyl, darstellt,
mit einem Anilin mit der folgenden Formel (VI): 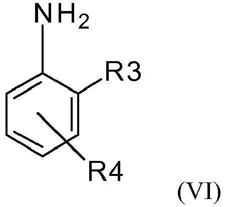 mit R3 und R4, wie in Anspruch 1 definiert, um ein Imin mit der folgenden Formel (VII) zu ergeben: 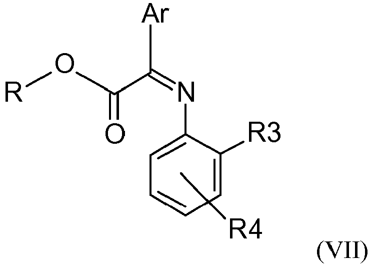 mit R, wie vorhergehend definiert, und R3, R4 und Ar wie in Anspruch 1 definiert,
ii) Reduktion des Imins mit der Formel (VII), das im vorhergehenden Schritt erhalten wurde, um ein Amin mit der folgenden Formel (VIII) zu ergeben: 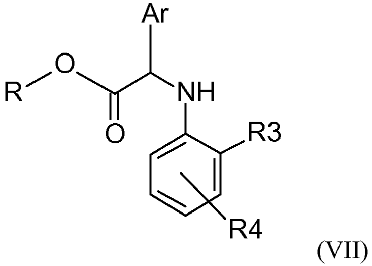 mit R, wie vorhergehend definiert, und R3, R4 und Ar, wie in Anspruch 1 definiert,
iii) Verseifung der Esterfunktion der Verbindung mit der Formel (VIII), die im vorhergehenden Schritt erhalten wurde, um eine Säure mit der folgenden Formel (IV) zu ergeben: 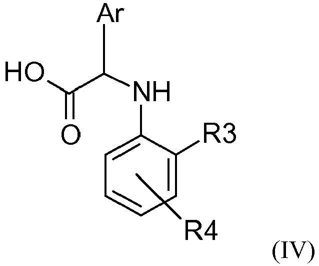 mit R3, R4 und Ar, wie im Anspruch 1 definiert,
iv) Reaktion der Säure mit der Formel (IV), die im vorhergehenden Schritt erhalten wurde, mit einem Piperazin mit der folgenden Formel (III): 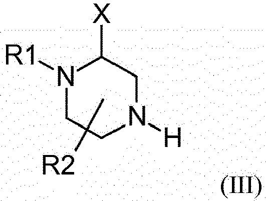
mit X, R1 und R2, wie in Anspruch 1 definiert, wobei R1 kein Wasserstoffatom darstellt,
um ein Amin mit der Formel (II) nach Anspruch 16 zu ergeben,
v) Reaktion des Amins mit der Formel (II), die im vorhergehenden Schritt erhalten wurde, mit Chloracetylchlorid beim Vorhandensein einer Base, um eine Verbindung der Formel (I) mit R1 ≠ H zu ergeben, und
vi) gegebenenfalls Entschützung des Stickstoffatoms, das die Gruppe R1 ≠ H trägt, um eine Verbindung mit der Formel (I) mit R1 = H zu ergeben.

## Claims

1. A compound of following general formula (I): 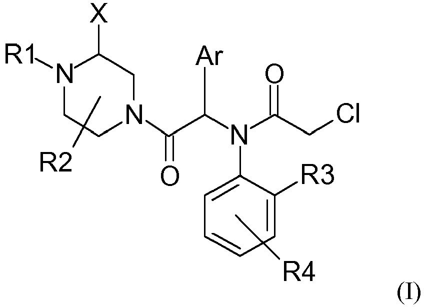 and the pharmaceutically acceptable salts thereof, its stereoisomers or mixtures of stereoisomers in any proportion, in particular an enantiomer mixture, and particularly a racemic mixture,
where:
- X is a (C₁-C₆)alkyl, phenyl, benzyl, C(O)OR5 or C(O)NHR5 group;
- R1 is a hydrogen atom or a C(O)H, C(O)R6 or C(O)OR6 group;
- R2 is a hydrogen atom or a (C₁-C₆)alkyl group; or R2 together with R1 or X forms a saturated hydrocarbon chain to form a 5- or 6-membered ring, in particular a 5-membered ring;
- R3 is a hydrogen or halogen atom or a (C₁-C₆)alkyl or (C₁-C₆)alkoxy group;
- R4 is a hydrogen or halogen atom, CN, NO₂, or a (C₁-C₆)alkyl, (C₁-C₆)alkoxy, aryloxy, benzyloxy or heteroaryloxy group, the said group optionally being substituted by one or more halogen atoms;
- Ar is a thiophenyl group or a phenyl group optionally substituted by one or more halogen atoms; and
- R5 and R6 independently of one another are a (C₁-C₆)alkyl, aryl-(C₁-C₆)alkyl or aryl group, the said group optionally being substituted by one or more halogen atoms.

2. The compound according to claim 1, **characterized in that** it meets the following formula (I-bis):

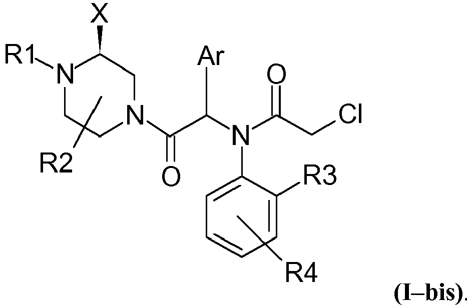

3. The compound according to any one of claims 1 and 2, **characterized in that** Ar is a thiophenyl group or a phenyl group substituted by one or more fluorine atoms such as 4-fluoro-phenyl.

4. The compound according to any one of claims 1 to 3, **characterized in that** R4 is a hydrogen or halogen atom or a (C₁-C₆)alkyl, (C₁-C₆)alkoxy or aryloxy group, the said group optionally being substituted by one or more halogen atoms, fluorine in particular.

5. The compound according to any one of claims 1 to 4, **characterized in that** R3 is a hydrogen atom or a (C₁-C₆)alkyl group e.g. methyl.

6. The compound according to any one of claims 1 to 5, **characterized in that** X is a (C₁-C₆)alkyl, phenyl or benzyl group; R1 and R2 are a hydrogen atom; R3 is a hydrogen atom or a (C₁-C₆)alkyl group, in particular (C₁-C₃)alkyl; R4 is a halogen atom or a (C₁-C₆)alkyl, (C₁-C₆)alkoxy, aryloxy or benzyloxy group, the said group optionally being substituted by one or more halogen atoms; Ar is a thiophenyl group or a phenyl group optionally substituted by a fluorine atom such as 4-fluoro-phenyl; and R5 and R6 independently of one another are a (C₁-C₆)alkyl, aryl-(C₁-C₆)alkyl or aryl group, the said group optionally being substituted by one or more fluorine atoms.

7. The compound according to claim 1, **characterized in that** the compound is selected from among:
| | |
|---|---|
| 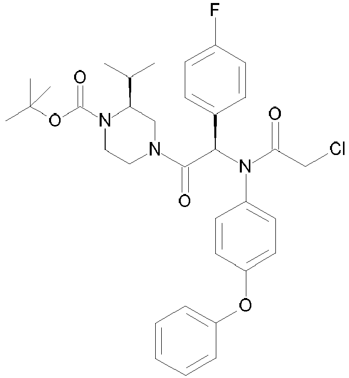 | 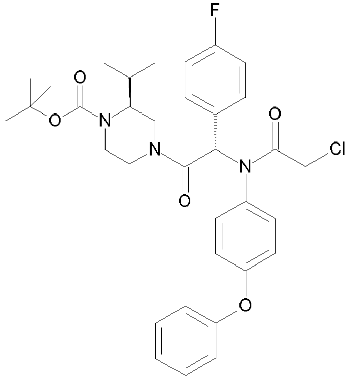 |
| I-1a | I-1b |
| 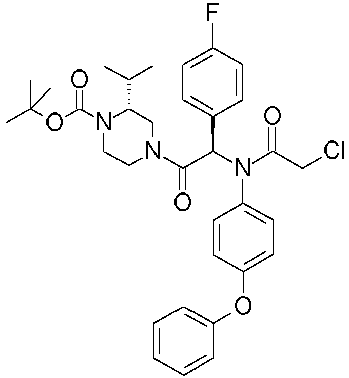 | 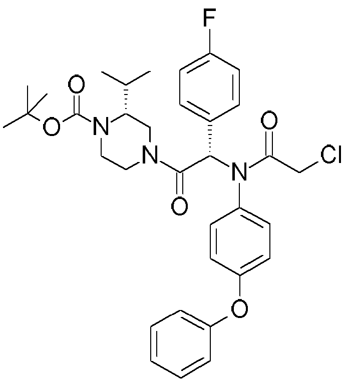 |
| I-2a | I-2b |
| 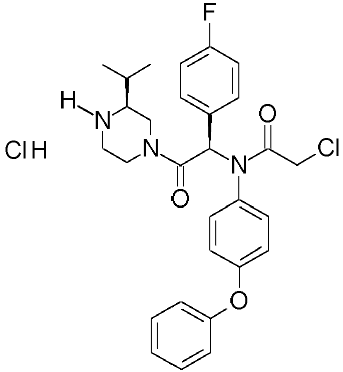 | 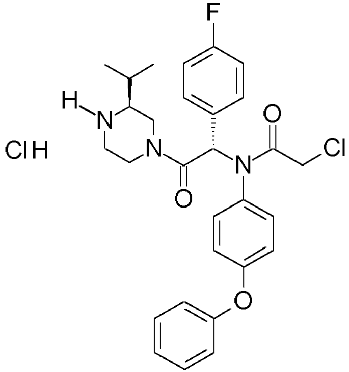 |
| I-3a | I-3b |
| 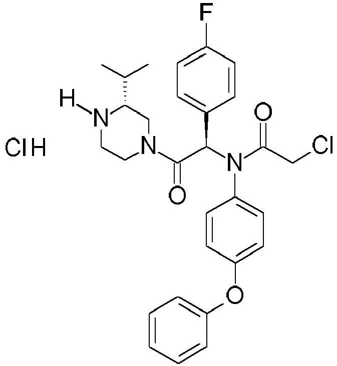 | 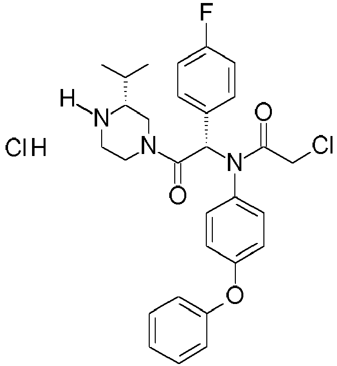 |
| I-4a | I-4b |
| 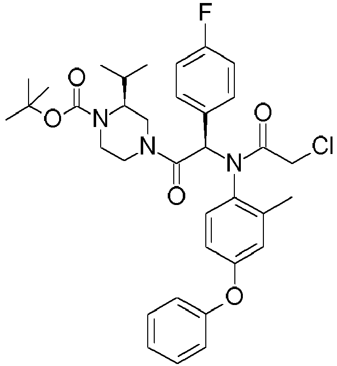 | 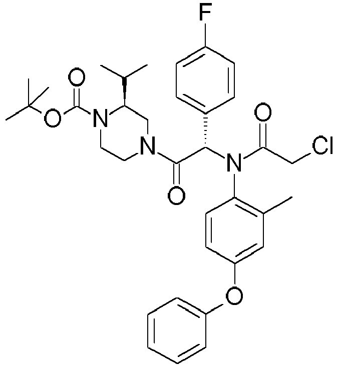 |
| I-5a | I-5b |
| 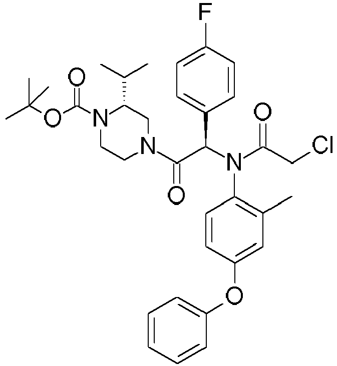 | 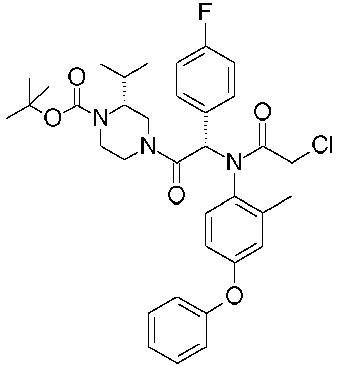 |
| I-6a | I-6b |
| 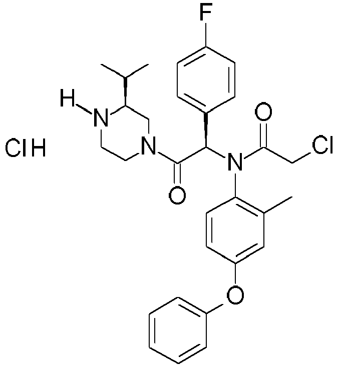 | 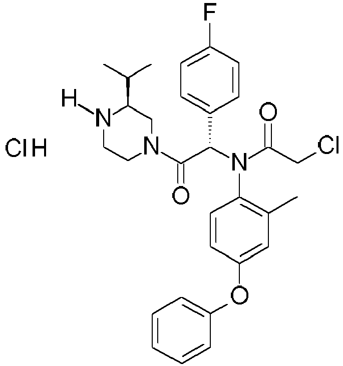 |
| I-7a | I-7b |
| 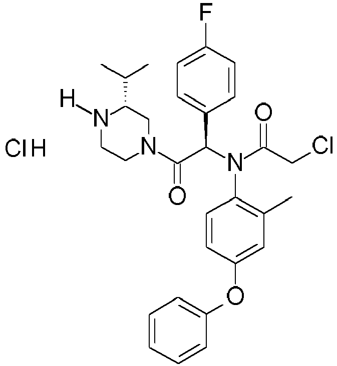 | 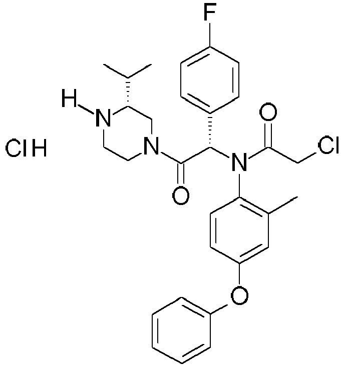 |
| I-8a | I-8b |
| 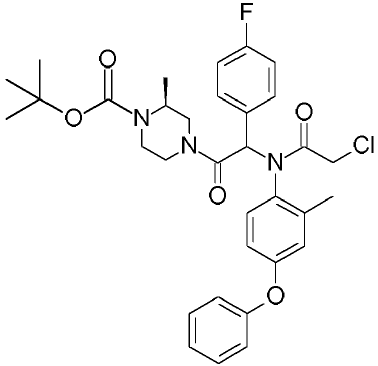 | 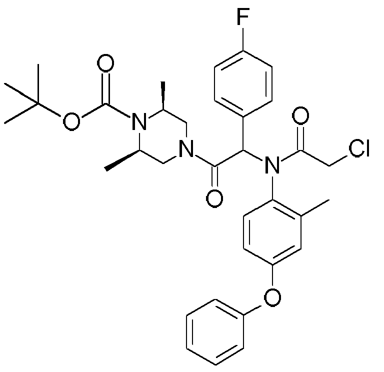 |
| I-9 | I-10 |
| 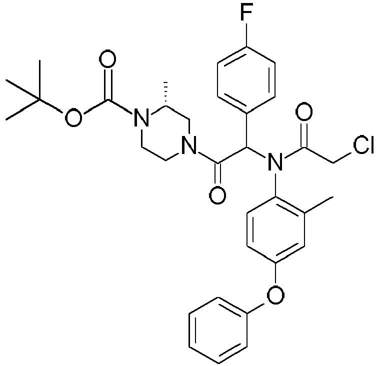 | 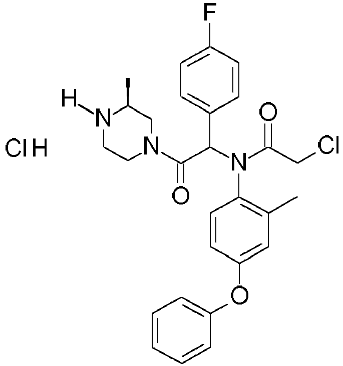 |
| I-11 | I-12 |
| 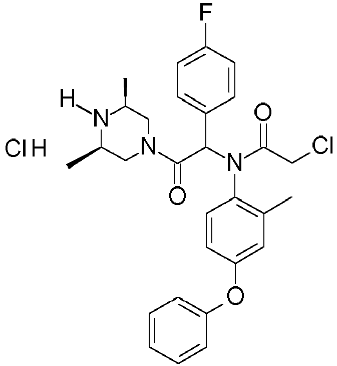 | 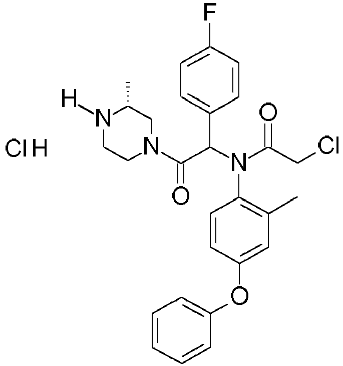 |
| I-13 | I-14 |
| 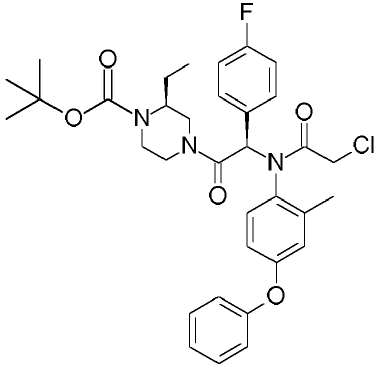 | 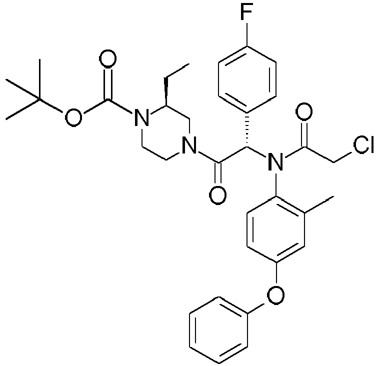 |
| I-15a | I-15b |
| 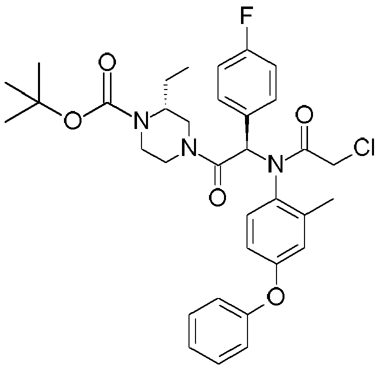 | 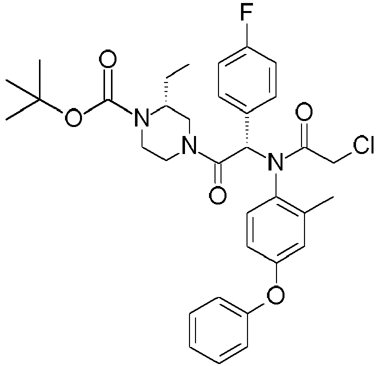 |
| I-16a | I-16b |
| 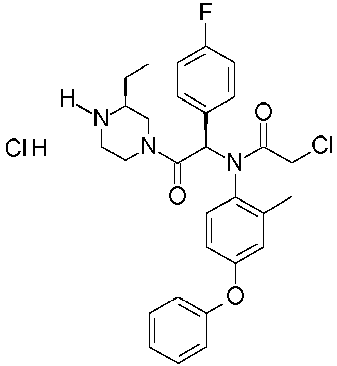 | 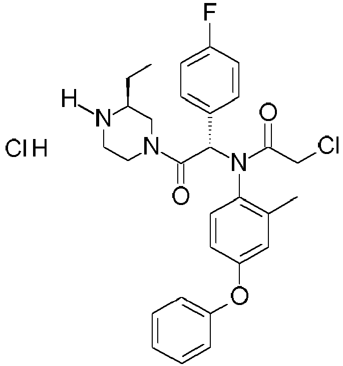 |
| I-17a | I-17b |
| 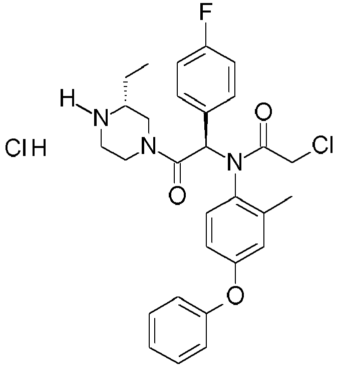 | 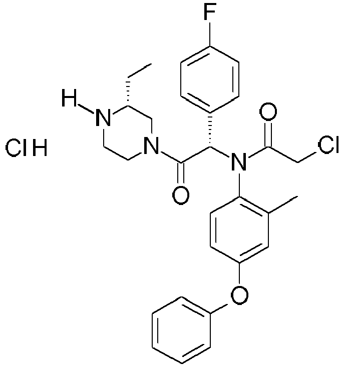 |
| I-18a | I-18b |
| 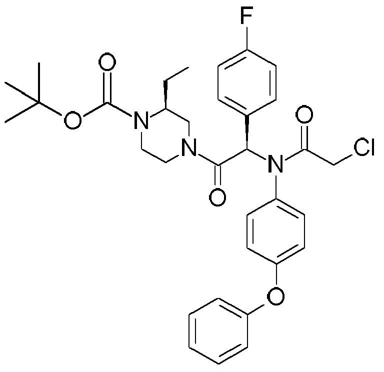 | 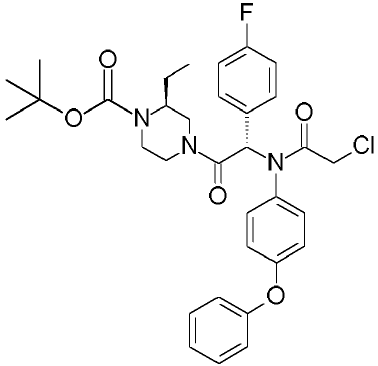 |
| I-19a | I-19b |
| 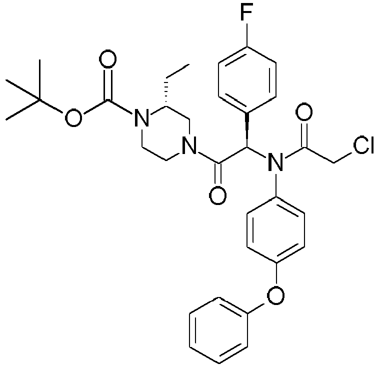 | 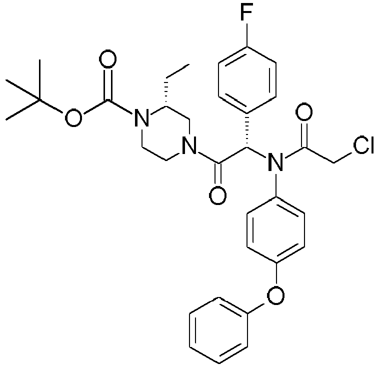 |
| I-20a | I-20b |
| 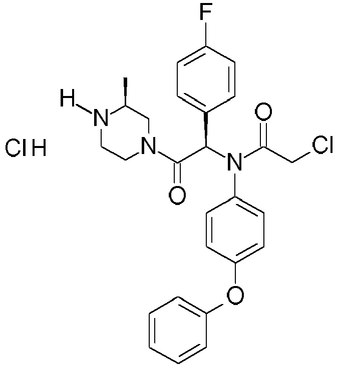 | 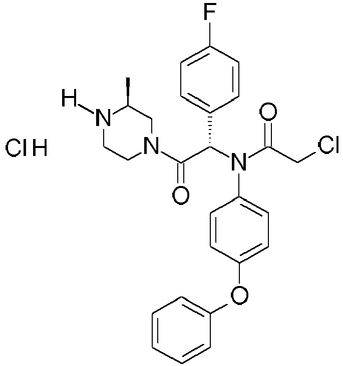 |
| I-21a | I-21b |
| 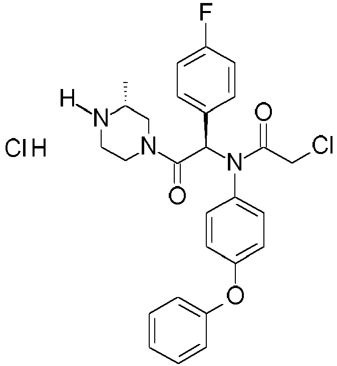 | 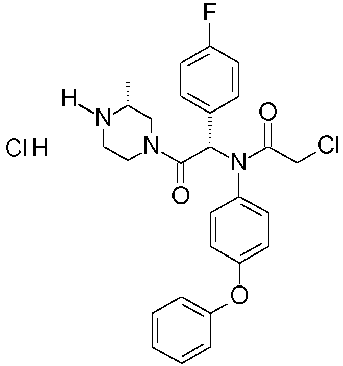 |
| I-22a | I-22b |
| 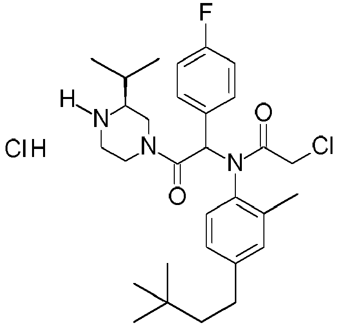 | 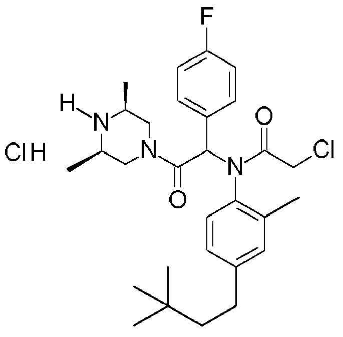 |
| I-23 | I-24 |
| 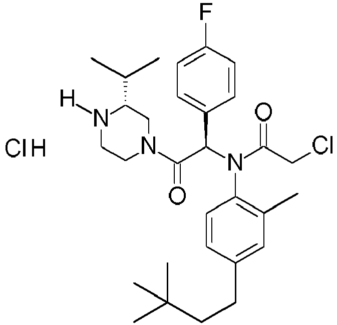 | 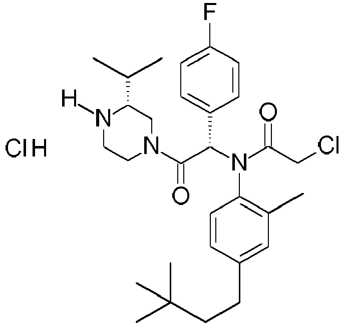 |
| I-25a | I-25b |
| 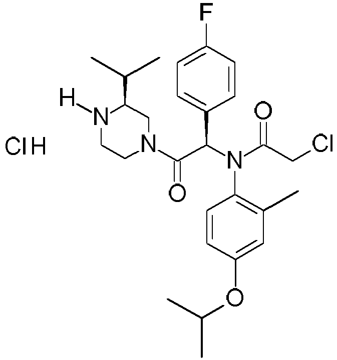 | 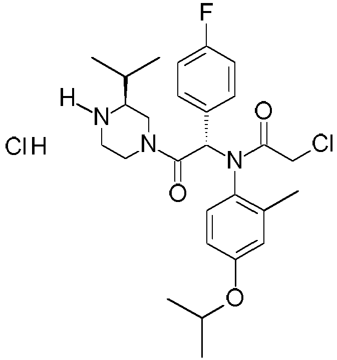 |
| I-26a | I-26b |
| 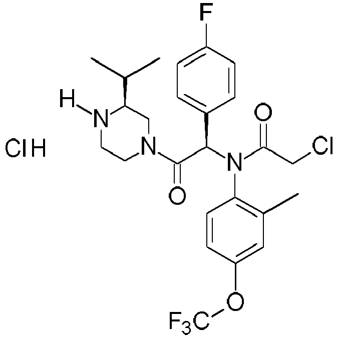 | 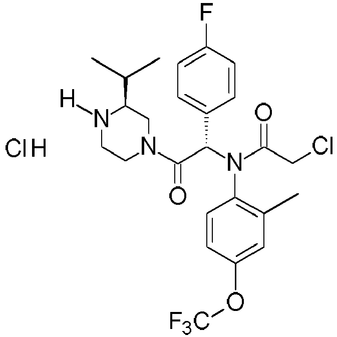 |
| I-27a | I-27b |
| 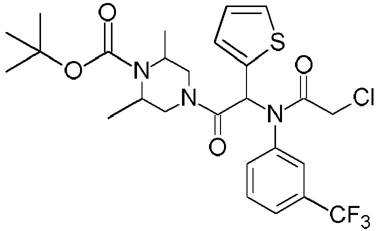 | 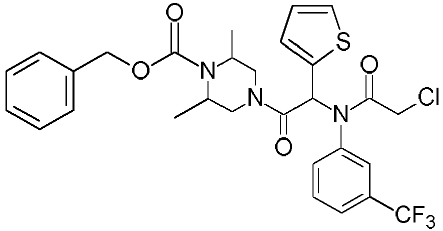 |
| I-28 | I-29 |
| 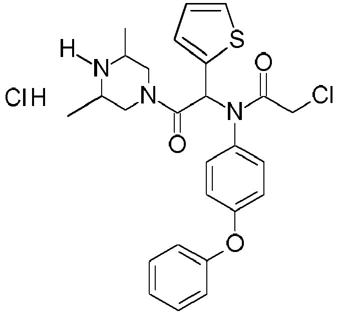 | 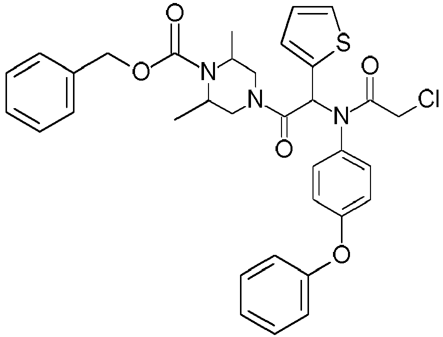 |
| I-30 | I-31 |
| 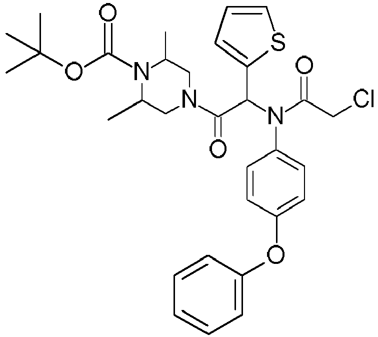 | 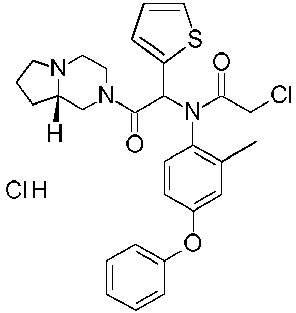 |
| I-32 | I-33 |
| 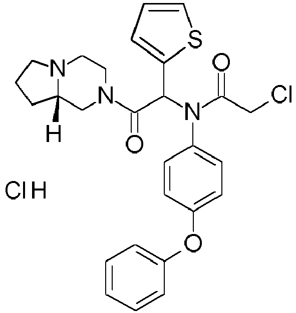 | 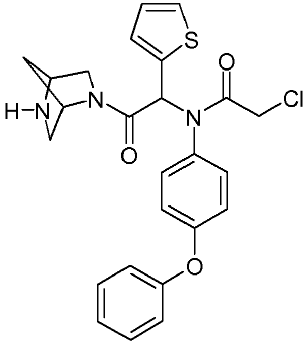 |
| I-34 | I-35 |
| 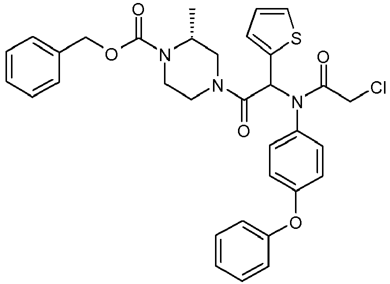 | 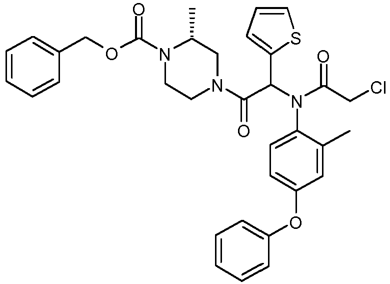 |
| I-36 | I-37 |
| 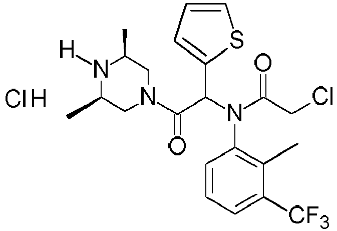 | 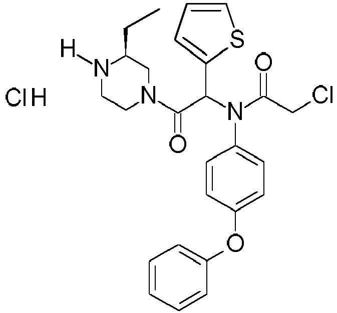 |
| I-38 | I-39 |
| 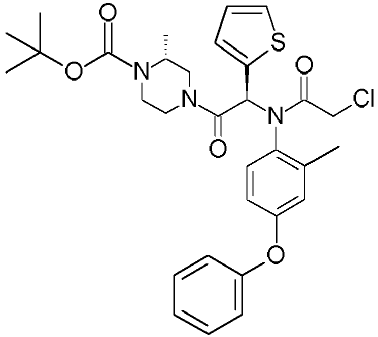 | 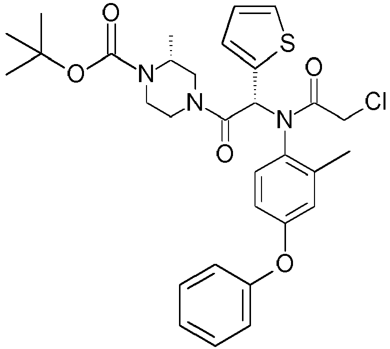 |
| I-40a | I-40b |
| 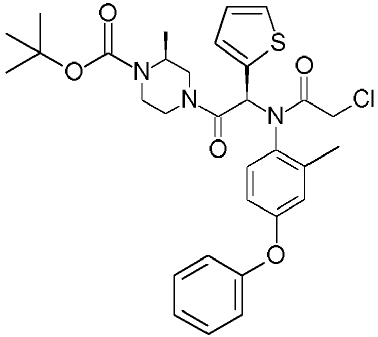 | 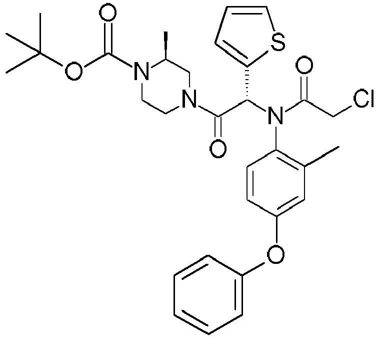 |
| I-41a | I-41b |
| 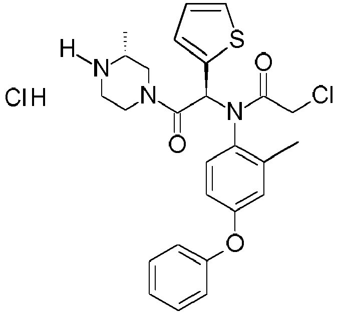 | 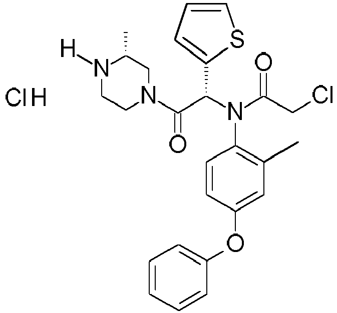 |
| I-42a | I-42b |
| 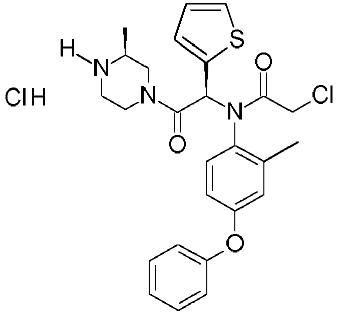 | 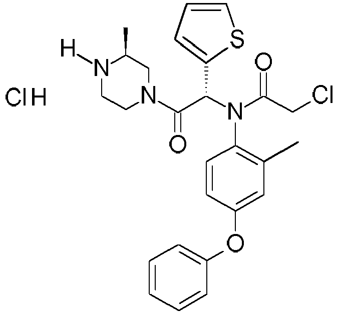 |
| I-43a | I-43b |
| 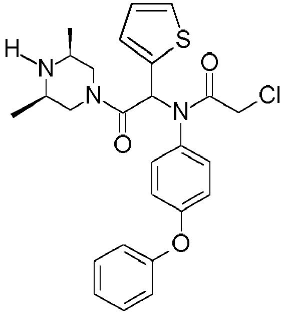 | 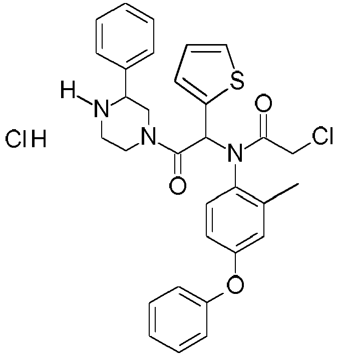 I-45 |
| I-44 | |
| 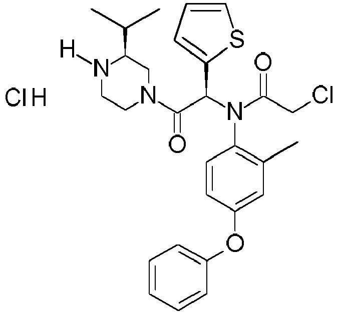 | 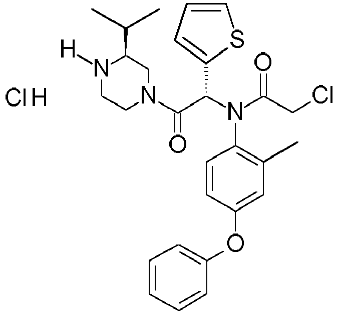 |
| I-46a | I-46b |
| 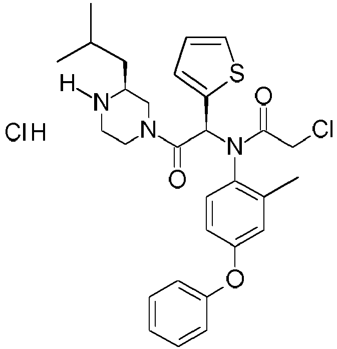 | 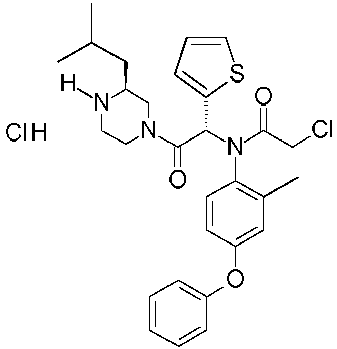 |
| I-47a | I-47b |
| 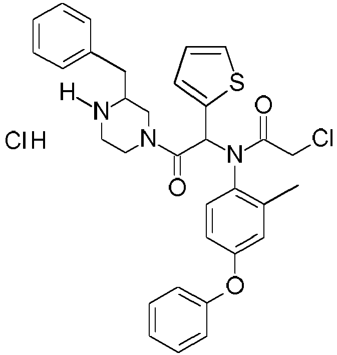 | 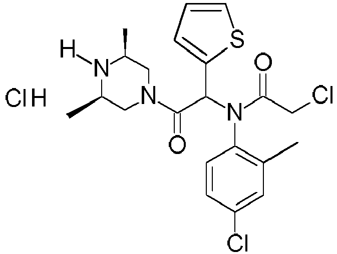 |
| I-48 | I-49 |
| 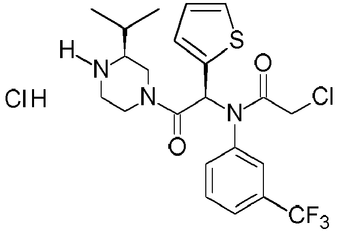 | 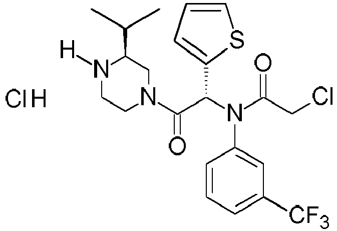 |
| I-50a | I-50b |
| 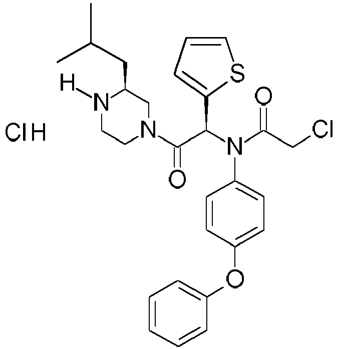 | 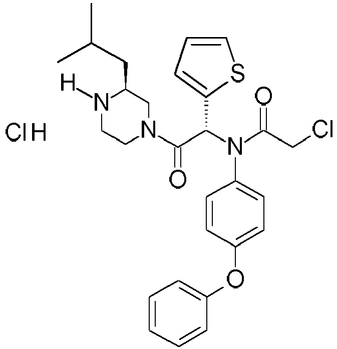 |
| I-51a | I-51b |
| 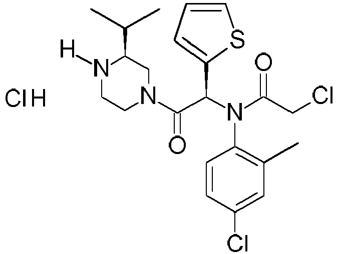 | 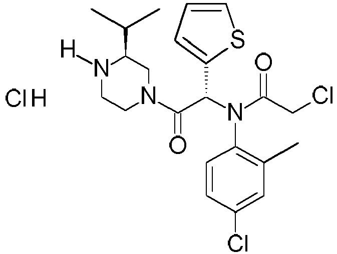 |
| I-52a | I-52b |
| 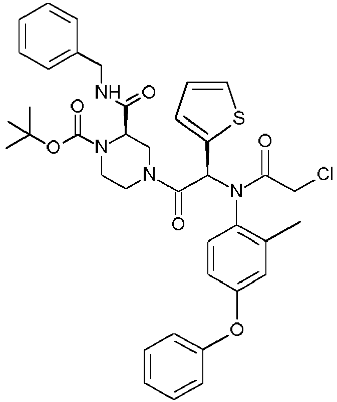 | 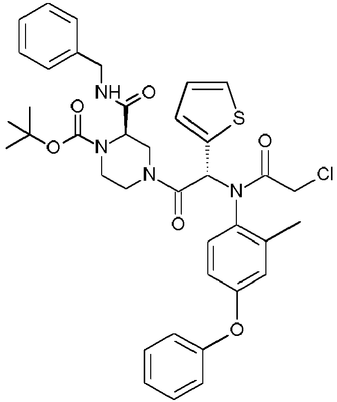 |
| I-53a | I-53b |
| 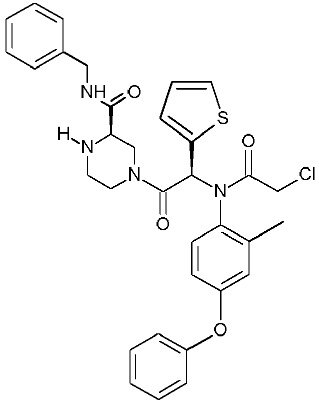 | 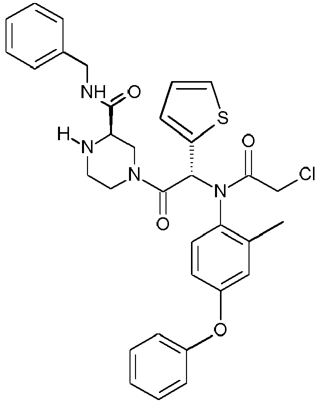 |
| I-54a | I-54b |
| 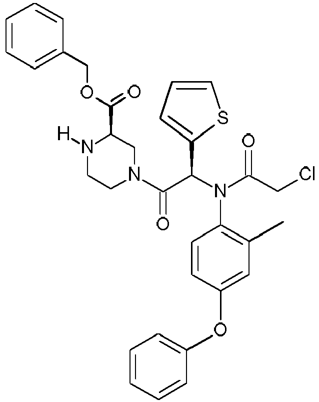 | 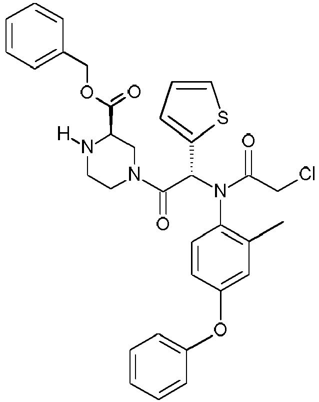 |
| I-55a | I-55b |
| 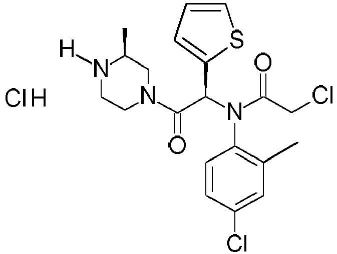 | 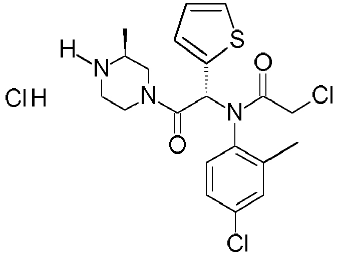 |
| I-56a | I-56b |
| 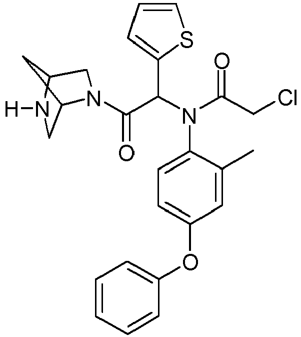 | 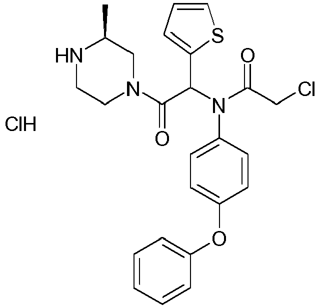 |
| I-57 | I-58 |
| 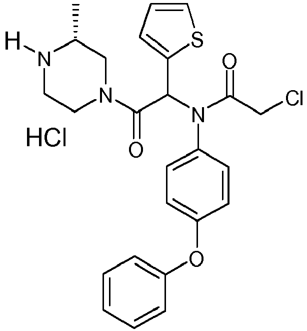 | 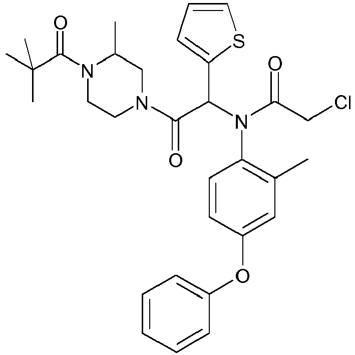 |
| I-59 | I-60 |
| 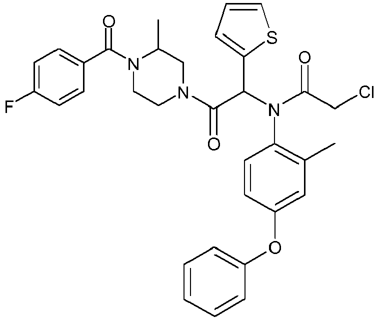 | 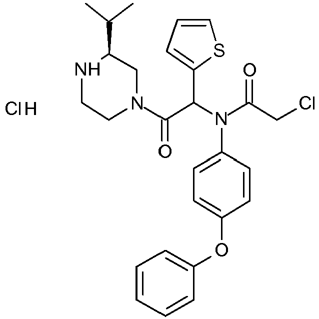 |
| I-61 | I-62 |
| 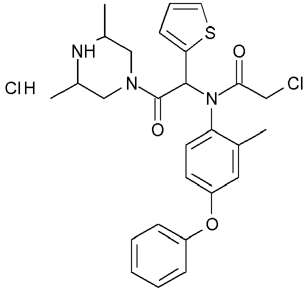 | |
| I-63 | |

8. A compound of general formula (I) according to any one of claims 1 to 7 for use as a drug.

9. A compound of general formula (I) according to any one of claims 1 to 7 for use in the treatment or prevention of cancer and in particular chemotherapy-resistant cancer.

10. A pharmaceutical composition comprising at least one compound of general formula (I) according to any one of claims 1 to 7, in association with one or more pharmaceutically acceptable excipients.

11. The pharmaceutical composition according to claim 10, **characterized in that** it comprises at least one other active ingredient such as an anticancer agent.

12. The pharmaceutical composition according to claim 11, **characterized in that** the active ingredient(s) are chosen from among cisplatin and its derivatives such as carboplatin and oxalyplatin; taxanes such as taxol, taxotere, paclitaxel and docetaxel; vinca alkaloids such as vinblastine, vincristine and vinorelbine; purine analogues such as mercaptopurine, thioguanine, pentostatin and 2-chlorodeoxyadenosine; topoisomerase I inhibitors such as camptothecin compounds e.g. irinotecan and topotecan; topoisomerase II inhibitors such as epipodophyllotoxin, podophyllotoxin and the derivatives thereof such as etoposide and teniposide; anti-tumour nucleoside derivatives such as 5-fluorouracil, leucovorin, gemcitabine or capecitabine; alkylating agents such as nitrogen mustards e.g. cyclophosphamide, mechlorethamine, chlorambucil and melphalan, nitroso-ureas such as carmustin, lomustin and streptozocin, alkylsulfonates such as busulfan, ethylenimines and methylmelamines such as thiotepa and hexamethylmelamine, and tetrazines such as dacarbazine; derivatives of anti-tumour anthracyclines such as daunorubicin, adriamycin, doxil, idarubicin and mitoxantrone; molecules targeting the IGF-I receptor such as picropodophyllin; tetracarcin derivatives such as tetrocarcin A; corticosteroids such as prednisone; antibodies such as trastuzumab (anti-HER2 antibody), rituximab (anti-CD20 antibody), gemtuzamab, cetuximab, pertuzumab and bevacizumab; antagonists or selective modulators of oestrogen receptors such as tamoxifen, fulvestrant, toremifene, droloxifene, faslodex and raloxifene, aromatase inhibitors such as exemestane, anastrozole, letrozole and vorozole; differentiating agents such as retinoids e.g. retinoic acid and vitamin D and agents blocking the metabolism of retinoic acid such as accutane; DNA methyl-transferase inhibitors such as azacytidine and decitabine; antifolates such as permetrexed disodium; antibiotics such as antinomycin D, bleomycin, mitomycin C, actinomycin D, carminomycin, daunomycin and plicamycin; antimetabolites such as chlofarabine, aminopterin, cytosine arabinoside, floxuridine and methotrexate; apoptosis-inducing agents and anti-angiogenic agents of Bcl-2 inhibitors such as YC 137, BH 312, ABT 737, gossypol, HA 14-1, TW 37 and decanoic acid; agents binding to tubulin such as combrestatin, colchicine derivatives and nocodazole; kinase inhibitors such as flavoperidol, imatinib mesylate, erlotinib and gefitinib; farnesyl transferase inhibitors such as tipifarnib; histone-deacetylase inhibitors such as sodium butyrate, suberoylanilide hydroxamic acid, depsipeptide, NVP- LAQ824, R306465, JNJ-26481585 and trichostatin A; inhibitors of the ubiquitin-proteasome system such as MLN .41, bortezomib and yondelis; and telomerase inhibitors such as telomestatin.

13. A pharmaceutical composition comprising:
(i) at least one formula (I) compound according to any one of claims 1 to 7; and
(ii) at least one other active ingredient such as an anticancer agent,
as combination products for simultaneous, separate or time-staggered use thereof.

14. The pharmaceutical composition according to claim 13, **characterized in that** the active ingredient(s) are selected from among cisplatin and the derivatives thereof such as carboplatin and oxalyplatin; taxanes such as taxol, taxotere, paclitaxel and docetaxel; vinca alkaloids such as vinblastine, vincristine and vinorelbine; purine analogues such as mercaptopurine, thioguanine, pentostatin and 2-chlorodeoxyadenosine; topoisomerase I inhibitors such as camptothecin compounds e.g. irinotecan and topotecan; topoisomerase II inhibitors such as epipodophyllotoxin, podophyllotoxin and the derivatives thereof such as etoposide and teniposide; anti-tumour nucleoside derivatives such as 5-fluorouracil, leucovorin, gemcitabine or capecitabine; alkylating agents such as nitrogen mustards e.g. cyclophosphamide, mechlorethamine, chlorambucil and melphalan, nitroso-ureas such as carmustin, lomustin and streptozocin, alkylsulfonates such as busulfan, ethylenimines and methylmelamines such as thiotepa and hexamethylmelamine, and tetrazines such as dacarbazine; derivatives of anti-tumour anthracyclines such as daunorubicin, adriamycin, doxil, idarubicin and mitoxantrone; molecules targeting the IGF-I receptor such as picropodophyllin; tetracarcin derivatives such as tetrocarcin A; corticosteroids such as prednisone; antibodies such as trastuzumab (anti-HER2 antibody), rituximab (anti-CD20 antibody), gemtuzamab, cetuximab, pertuzumab and bevacizumab; antagonists or selective modulators of oestrogen receptors such as tamoxifen, fulvestrant, toremifene, droloxifene, faslodex and raloxifene; aromatase inhibitors such as exemestane, anastrozole, letrozole and vorozole; differentiating agents such as retinoids e.g. retinoic acid and vitamin D and agents blocking the metabolism of retinoic acid such as accutane; DNA methyl-transferase inhibitors such as azacytidine and decitabine; antifolates such as permetrexed disodium; antibiotics such as antinomycin D, bleomycin, mitomycin C, actinomycin D, carminomycin, daunomycin and plicamycin; antimetabolites such as chlofarabine, aminopterin, cytosine arabinoside, floxuridine and methotrexate; apoptosis-inducing agents and anti-angiogenic agents of Bcl-2 inhibitors such as YC 137, BH 312, ABT 737, gossypol, HA 14-1, TW 37 and decanoic acid; agents binding to tubulin such as combrestatin, colchicine derivatives and nocodazole; kinase inhibitors such as flavoperidol, imatinib mesylate, erlotinib and gefitinib; farnesyl transferase inhibitors such as tipifarnib; histone-deacetylase inhibitors such as sodium butyrate, suberoylanilide hydroxamic acid, depsipeptide, NVP- LAQ824, R306465, JNJ-26481585 and trichostatin A; inhibitors of the ubiquitin-proteasome system such as MLN .41, bortezomib and yondelis; and telomerase inhibitors such as telomestatin.

15. The pharmaceutical composition according to any one of claims 10 to 14 for use as a drug intended to treat or prevent cancer, in particular to treat a chemotherapy-resistant cancer.

16. A method for preparing a formula (I) compound according to any one of claims 1 to 7 comprising the following successive steps:
a) reacting an amine of following formula (II): 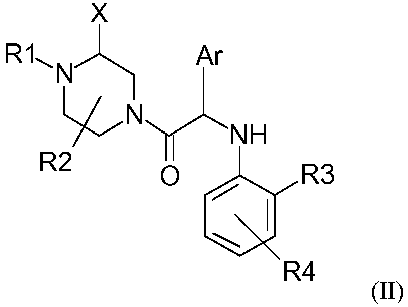
where X, R1, R2, R3, R4 and Ar are as defined in claim 1, R1 not representing a hydrogen atom,
with chloroacetyl chloride in the presence of a base to give a formula (I) compound where R1 ≠ H; and
b) optionally deprotecting the nitrogen atom carrying the R1 ≠ H group to give a formula (I) compound where R1 = H.

17. The method according to claim 16 comprising the following successive steps:
i) reacting a ketoester of following formula (V): 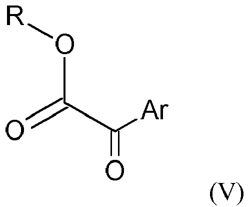
where Ar is as defined in claim 1 and R represents a (C₁-C₆)alkyl group, such as ethyl;
with an aniline of following formula (VI): 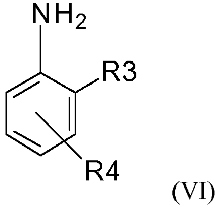 where R3 and R4 are as defined in claim 1, to give an imine of following formula (VII): 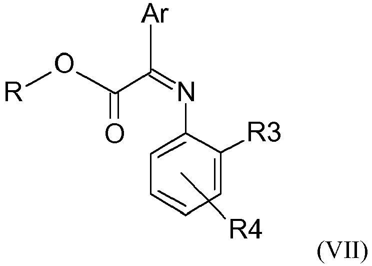 where R is as previously defined, and R3, R4 and Ar are as defined in claim 1;
ii) reducing the imine of formula (VII) obtained at the preceding step to give an amine of following formula (VIII): 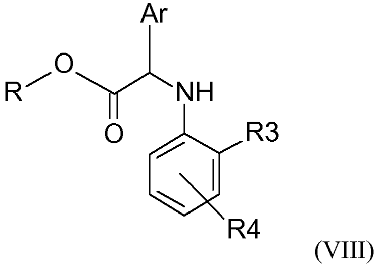 where R is as previously defined, and R3, R4 and Ar are as defined in claim 1;
iii) saponifying the ester function of the formula (VIII) compound obtained at the preceding step to give an acid of following formula (IV): 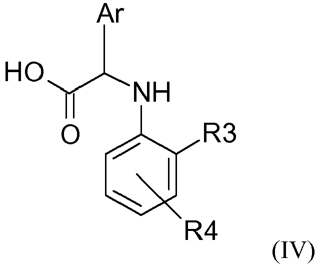 where R3, R4 and Ar are as defined in claim 1;
iv) reacting the acid of formula (IV) obtained at the preceding step with a piperazine of following formula (III): 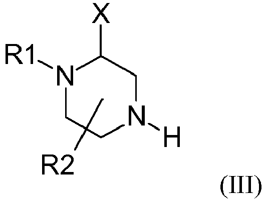
where X, R1 and R2 are as defined in claim 1, R1 not representing a hydrogen atom,
to give an amine of formula (II) according to claim 16;
v) reacting the amine of formula (II) obtained at the preceding step with chloroacetyl chloride in the presence of a base to give a formula (I) compound where R1 ≠ H; and
vi) optionally deprotecting the nitrogen atom carrying the R1 ≠ H group to give a formula (I) compound where R1 = H.
